# EUROPEAN PATENT SPECIFICATION

(11) **EP 3 131 560 B1**
(45) Date of publication and mention of the grant of the patent: **14.11.2018**
(21) Application number: 15718837.6
(22) Date of filing: 16.04.2015
(51) Int. Cl.: A61K 35/14, C12N 5/0783, A61K 45/00, G01N 33/68, A61K 39/00

(54) **METHOD FOR THE IDENTIFICATION OF CD4+ REGULATORY T-CELLS FOR USE IN THE TREATMENT OF INFLAMMATORY AND AUTOIMMUNE DISEASES**
VERFAHREN ZUR IDENTIFIZIERUNG VON CD4+ REGULATORISCHEN T-ZELLEN ZUR VERWENDUNG BEI DER BEHANDLUNG VON ENTZÜNDUNGS- UND AUTOIMMUNERKRANKUNGEN
PROCÉDÉ POUR L'IDENTIFICATION DE CELLULES T RÉGULATRICES CD4+ POUR UNE UTILISATION DANS LE TRAITEMENT DE MALADIES INFLAMMATOIRES ET AUTO-IMMUNES

(30) Priority: 16.04.2014 DK 201470225; 11.07.2014 DK 201470440
(43) Date of publication of application: 22.02.2017
(73) Proprietor: Genovie AB, 371 33 Karlskrona (SE)
(72) Inventor: JARVIS, Reagan Micheal, Sydney New South Wales 2087 (AU); THÖRN, Magnus, S-752 26 Uppsala (SE)
(74) Representative: Chas. Hude A/S
(86) International application number: PCT/EP2015/058322
(87) International publication number: WO 2015/158857

(56) References cited:
- BRUSKO TODD M ET AL: "Human regulatory T cells: role in autoimmune disease and therapeutic opportunities", IMMUNOLOGICAL REVIEWS, BLACKWELL PUBLISHING, MUNKSGAARD, vol. 223, 1 June 2008 (2008-06-01), pages 371-390, XP002596194, ISSN: 0105-2896
- Brian G. Engelhardt ET AL: "Homing in on Acute Graft vs. Host Disease: Tissue-Specific T Regulatory and Th17 Cells" In: "Vaccines for Pandemic Influenza", 1 January 2010 (2010-01-01), Springer Berlin Heidelberg, Berlin, Heidelberg, XP055159267, ISSN: 0070-217X ISBN: 978-3-54-092165-3 vol. 341, pages 121-146, DOI: 10.1007/82_2010_24, page 4, paragraphs 2,3 page 5, paragraph 2
- LEVINGS M K ET AL: "Human CD25+CD4+ T regulatory cells suppress naive and memory T cell proliferation and can be expanded in vitro without loss of function", THE JOURNAL OF EXPERIMENTAL MEDICINE, ROCKEFELLER UNIVERSITY PRESS, US, vol. 193, no. 11, 4 June 2001 (2001-06-04) , pages 1295-1301, XP008120051, ISSN: 0022-1007
- GEGINAT J ET AL: "The CD4-centered universe of human T cell subsets", SEMINARS IN IMMUNOLOGY, vol. 25, no. 4, 15 November 2013 (2013-11-15), pages 252-262, XP028793622, ISSN: 1044-5323, DOI: 10.1016/J.SMIM.2013.10.012
- JONULEIT H ET AL: "Identification and functional characterization of human CD4(+)CD25(+) T cells with regulatory properties isolated from peripheral blood", THE JOURNAL OF EXPERIMENTAL MEDICINE, ROCKEFELLER UNIVERSITY PRESS, US, vol. 193, no. 11, 4 June 2001 (2001-06-04) , pages 1285-1294, XP002310837, ISSN: 0022-1007, DOI: 10.1084/JEM.193.11.1285
- PIERRE DESREUMAUX ET AL: "Safety and Efficacy of Antigen-Specific Regulatory T-Cell Therapy for Patients With Refractory Crohn's Disease", GASTROENTEROLOGY, vol. 143, no. 5, 1 November 2012 (2012-11-01), pages 1207-1217.e2, XP055158934, ISSN: 0016-5085, DOI: 10.1053/j.gastro.2012.07.116
- SASKIA THOMAS ET AL: "Targeting leukocyte migration and adhesion in Crohn's disease and ulcerative colitis", INFLAMMOPHARMACOLOGY ; EXPERIMENTAL AND CLINICAL STUDIES - OFFICIAL PUBLICATION OF THE GASTROINTESTINAL SECTION OF THE INTERNATIONAL UNION OF PHARMACOLOGY (IUPHAR), BIRKHÄUSER-VERLAG, BA, vol. 20, no. 1, 20 December 2011 (2011-12-20), pages 1-18, XP035009879, ISSN: 1568-5608, DOI: 10.1007/S10787-011-0104-6

## Description

### Field of the invention

The present invention relates to cellular immunotherapy, in particular cellular immunotherapy with CD4⁺ T-regulatory cells (Treg) for the treatment of inflammatory and autoimmune diseases affecting mucosal and non-mucosal tissues in a targeted manner via identification and purification of involved Treg populations.

### Background of the invention

Inflammation is the manifestation of a complex immunological response toward harmful factors presented by pathogenic microorganisms, commensal microorganisms, foodstuffs and other foreign material, components of damaged self- and also healthy self-tissues. Inflammation can be classified as either acute or chronic. The classical signs of acute inflammation are pain, heat, redness, swelling and accumulating loss of tissue function. Prolonged inflammation, known as chronic inflammation, leads to a progressive shift in the character of the inflammation and is often accompanied by tissue destruction and impaired healing (e.g. fibrosis). In most instances, aberrant inflammation of tissues is associated with a misguided or over-exuberant adaptive immune response towards foreign and/or self-antigens. Reaction against self-antigens, or components of the host's own tissues, is termed autoimmunity. It is often unclear what triggers aberrant inflammatory states in most disorders. Aberrant reaction against foreign antigens and autoimmunity are certainly not mutually exclusive states, and dysfunctional activation against foreign antigens can trigger autoimmunity, or vice versa.

An example of typical inflammatory disorders, are inflammatory bowel diseases (IBD). Inflammatory bowel diseases IBD are a group of inflammatory conditions of the colon and small intestine. The major types of IBD are Crohn's disease (CD) and ulcerative colitis (UC). It is often considered that CD, affecting the small bowel, is largely driven by T-cell mediated adaptive immunity, and that the immunological reaction is driven by antigens of the luminal flora. On the other hand, it is often cited that the inflammatory state of the mainly colonic UC is at least partly driven by local humoral autoimmunity responses, with autoantibodies often detected in diseased UC tissues. However, this distinction is perhaps as much reflective of the differing immunological roles of the small and large bowel mucosa, as it is a fundamental difference in disease immunopathogenesis. Indeed, the small bowel is the first major encounter with bulk food and commensal/pathogenic microbiological antigens, and thus logically requires broader cell-mediated adaptive immunological mechanisms. On the other hand, the colon is used as a bioreactor for bulking of commensal bacteria, and thus logically might contain less exuberant cell-mediated adaptive immune mechanisms.

Most authors consider the immunopathogenesis of IBD as an over-exuberant reaction towards foreign antigens. It is this excessive activation that drives both establishment of localised chronic inflammation, and also drives a more systemic immune dysfunction in patients. Indeed, there are several well-recognised inflammatory, or specifically autoimmune, disorders associated with the chronic inflammation of IBD sufferers, including disorders of the joints, eyes, skin, liver and lungs.

T-cells are central to cell-mediated adaptive immunity. Two main subdivisions of T-cells may be defined, were T-effector cells (Teffs) can be generalised to represent pro-inflammatory activities, and Tregs to represent an anti-inflammatory check. Exuberant Teff activity is observable in both animal models and human disease alike, and has been attributed in recent years to a breakdown in Treg-mediated homoeostatic mechanisms. However, it remains difficult to attribute IBD immunopathogenesis to any specific functional or numerical defect in Tregs themselves. This is in no small part due to the fact that proposed *in vivo* mechanisms of Treg function in humans remain largely speculative. Regardless, numerous animal models and early clinical experiences have suggested that Treg cells could be harnessed for treatment of a range of inflammatory disorders, and particularly IBD.

T-cells impart control locally; individually influencing control of immune responses over relatively short distances. Consequently, the migration of T-cells between intestinal mucosa and other bodily compartments is a critical determinant of functional responses. Several large-scale clinical trials have focused on blocking T-cell migration to intestinal tissues through pharmaceutical blockade of either adhesion molecules or chemoattractants critical of T-cell migration to intestinal mucosa, with mixed success.

A majority of the knowledge around the T-cell pathology of IBD is inferred from mouse models. It is well established that transfer of naive conventional CD4⁺ T-cells into immune deficient mice results in a reaction against intestinal flora and establishment of intestinal inflammation, which can be rescued by co-transfer of Treg populations. It is also clear that Treg transfer into mice can resolve established intestinal inflammation. In the human setting, early indications of the link between intestinal tolerance and the human autoimmune syndrome were linked with FOXP3 mutations, the most common manifestation of which is chronic intestinal inflammation.

An accumulating body of data in patients with active and inactive IBD, and under various treatments, has yielded disparate results. Early studies suggested that the LP of both CD and ulcerative colitis (UC) patients contained functional Tregs. Some studies have reported increased levels of Tregs in inflamed LP of IBD patients.

Considering the importance of migration of cells between the periphery and mucosal tissues, it is critical to consider the peripheral Treg pool in relation to direct observations of the inflamed mucosa. Several early studies have reported decreased levels of peripheral CD4⁺ Treg cells in patients with active intestinal inflammation. However, the opposite has also been observed, with an increased frequency of peripheral CD4⁺ Tregs in IBD patients, though lower frequency is often observed in active when compared to inactive disease.

Studies investigating Treg response in IBD patients undergoing anti-tumour necrosis factor (anti-TNF) therapies have reported increased levels of peripheral Tregs, particularly among clinical responders. However, other studies have reported no change in peripheral Treg frequency, and even a decreased frequency. Similar studies in rheumatoid arthritis have shown that responders to anti-TNF and methotrexate therapies show increased numbers of peripheral Treg cells. Curiously, addition of anti-TNF drugs to activated T-cells from patients resulted in the generation of Treg cells *in vitro.*

In summary, while it may be generally anticipated that IBD is characterised by a breakdown of immunotolerance in the intestinal mucosa, there is a lack of consistent correlation with an impaired Treg function or diminished abundance in patient tissues. This may be a result of as yet crude analytical methods to identify Treg cells, discriminate Treg subsets, and to assay their functional properties. It is also likely a function of still incomplete understanding of Treg origin and function in intestinal immune homeostasis. Recent insights into T-cell immunity in the intestinal mucosa have come from more detailed studies of T-cell migration and induction in the periphery.

Accordingly, there is a need to identify Treg cells that are suitable for use in cellular immunotherapy for the treatment of inflammatory and autoimmune diseases and there is a need to develop protocols for how such Treg cells can be identified dependent on the particular disease and/or the diseased tissue.

Brusko et al. (Immunological Reviews, 2008) describes Treg based immunotherapy using cell sorting technologies. It teaches the sorting of Treg populations into subpopulations based on combinations of cell surface markers.

The general overview by Engelhardt and Crowe (Curr Top Microbiol Immunol, 2010) focuses on the underlying cause of acute graft versus host disease and it teaches that Tregs can be divided into distinct population subsets based on their homing characteristics.

Levings et al. (J. Exp. Med, 2001) shows that human CD25⁺ CD4⁺ Treg cells can expand *in vitro* and inhibit the proliferative response of both naive and memory T cells.

Geginat et al. (Seminars in Immunology, 2013) is a review that focus on T cell differentiation and describes that T cell subset can be purified *ex vivo* from relevant tissues to study T cell differentiation.

Desreumaux et al. (Gastroenterology, 2012) describes *in vitro* manufacture and administration of T cells raised against ovalbumin by a functional cloning method for the treatment of Crohn's disease.

### Description of the invention

The present invention addresses the above-mentioned needs. The present invention aims to identify Treg cells with unique characteristics suitable for the above-mentioned uses, and particularly selected for treatment of inflammatory and autoimmune diseases of defined tissues using the presented investigation of Crohn's disease as an investigational framework.

The present invention provides a method for identifying CD4⁺ Treg cells suitable for use as starting material in cellular immunotherapy, the method comprising
i) analysing samples from target tissue A to identify CD4⁺ Treg cells with migratory character between the diseased tissue, collecting lymphatics, peripheral blood, distinct tissue adjacent to the diseased target tissue A and/or distinct tissue that is not vicinal though has migratory Treg communication with target tissue A,
ii) optionally analysing samples from target tissue A to identify CD4⁺ Treg cells with functional character in tissue A,
iii) optionally analysing samples from lymphatic tissue B to identify CD4⁺ Treg cells with migratory character between disease draining lymphatics and non-disease draining lymphatics of diseased or non-diseased target tissue A,
iv) optionally analysing samples from lymphatic tissue B to identify CD4⁺ Treg cells with functional character in tissue B where the Treg cells are also emigrant from target tissue A,
v) analysing samples from peripheral blood, tissue C, to identify CD4⁺ Treg cells with migratory character and/or functional character where the Treg cells are also emigrant from target tissue A,
vi) analysing sample(s) from tissue compartments A and/or B and C, that are analytically or physically depleted of emigrants from thymus and/or immigrants from peripheral blood to a lymph node, to restrict analyses to CD4⁺ Treg cells of target tissue A origin and/or tropism,
to identify emigrant CD4⁺ Treg cell populations of target tissue A,
to identify emigrant CD4⁺ Treg cell populations with propensity to immigrate to target tissue A,
to identify a migratory and/or functional defect in the CD4⁺ Treg cell population identified as expressing migratory and/or functional elements specific for target tissue A in any of tissue A, B or C, and
whereby a combination of surface or intracellular markers on CD4⁺ Treg cells is identified, which combination identifies which surface or intracellular markers should be present and which surface markers should not be present in CD4⁺ Treg cell populations suitable for use as starting material in cellular immunotherapy.

Furthermore, the present invention provides a method for obtaining a CD4⁺ Treg cell population for use as starting material in cellular immunotherapy, the method comprising
i) subjecting peripheral blood from a patient suffering from an inflammatory or an autoimmune disease to single-cell analysis, whereby CD4⁺ Treg cells are separated from the blood, which CD4⁺ Treg cells have the signatures identified in the above-mentioned identification method and as defined herein, notably in the appended claims.

The methods of the invention are discussed in details herein.

Different forms of CD4⁺ Tregs may be considered. CD4⁺ Tregs that express the protein FOXP3 (FOXP3⁺CD4⁺ Tregs), and those that does not (FOXP3⁻CO4⁺ Tregs). In fact, FOXP3⁺CD4⁺ Tregs are now appreciated to be the major player in immunosuppressive function in most tissues of the body. Tr1 Tregs are identified functionally by their ability to secrete IL10. Another common type of FOXP3⁻CD4⁺ Treg is the Th3. This is similarly defined by its ability to secrete a protein called TGFβ. The conceptions of these FOXP3⁻CD4⁺ Tregs were based on early experimental mouse studies dealing with immunosuppressive function. In human immunology it is currently unclear how much of a role these cells play in overall immunosuppression, or what their precise role might be. In general, it is assumed that FOXP3⁻CD4⁺ cell immunosuppressive function is "contact-independent", while FOXP3⁺CD4⁺ immunosuppressive function is "contact-dependent". This means that FOXP3⁻CD4⁺ cells secrete factors that are free to diffuse in the general cellular milieu to affect general immunosuppression, while FOXP3⁻CD4⁺ cells need to physically cross-present inhibitory molecules on their plasma membrane to the cells they are targeting for suppression, thus requiring physical contact.

In the present context, the focus is on FOXP3⁺CD4⁺ cells, however, this presents an additional challenge. That is, unlike many marker proteins, which are presented on the cell plasma membrane, FOXP3 is actually expressed in the nucleus. Therefore, to detect FOXP3 it is necessary to destroy the cell. So despite being the defining marker of FOXP3⁺CD4⁺ Tregs, it is not possible to purify living cells on the basis of its expression.

FOXP3⁺CD4⁺ cells are subdivided into "natural Tregs" (nTregs) and "induced Tregs" (iTregs). nTregs arise naturally in the thymus, and are selected on the basis of being able to react with "self" antigens. This means that they are the general mediators of so called "self-tolerance". That is, they stop the immune system from attacking the body's own tissues. On the other hand, iTregs are those cells selected from naive T-cells in peripheral tissues for antigens from both self and extrinsic factors. Therefore, iTregs can be considered to mediate "adaptive tolerance", or tolerance towards mainly non-harmful things like antigens from our food, or commensal bacteria in our intestines. This concept of local clonal selection is something that can be referred to as antigen "education", and involves many co-stimuli.

This subdivision considers all Tregs that come from the selection of self-antigens in the thymus followed by emigration from the thymus to peripheral circulation as 'naturally' occurring nTregs. These nTregs are considered to be general drivers of self-tolerance, as they are raised against abundant self-antigens via high avidity interactions in the thymus. In contrast, iTregs are raised against antigens in the periphery from naive conventional T-cells. In the case of the intestinal mucosa, iTregs are likely to be raised primarily against foreign antigens such as those from food, and antigens arising from abundant commensal bacteria residing in the lumen. In this conception, iTregs represent the primary drivers of T-cell tolerance, for instance, towards foodstuffs and symbiotic bacteria. However, this subdivision between iTregs and nTregs in humans is largely conceptual, as they are practically very difficult to distinguish by surface markers, where their existence is ultimately inferred from interventional mouse experiments. To date, it has been difficult to address questions regarding nTreg and iTreg form and function in humans in any reliable and systematic manner due largely to practical limitations.

As mentioned above, the FOXP3 marker cannot be directly detected and used to the identification of live Tregs, since its detection is destructive. This issue has been solved and relies on one of two similar correlations of alternate plasma membrane markers. The simplest way to identify a FOXP3⁺ Treg is by positivity of CD4 and high expression of CD25, i.e. CD4⁺CD25^{hi}. A more rigorous identification can be conducted by further quantification of low CD127 expression: CD4⁺CD25^{hi}CD127^{lo}. As Teff cells by definition possess CD127^{hi} expression, CD4⁺CD25^{hi}CD127^{lo} effectively exclude Teff from identification and purification.

The current invention relates to leveraging on observed migratory patterns of CD4⁺ Treg cells in IBD patients, which allows identification of activated mucosal Treg subpopulations that may be purified as starting material for manufacture of cellular immunotherapeutic products. This IBD case study yields a methodological framework with which to conduct investigation of distinct mucosal and non-mucosal tissues in various disease states. Such an investigational framework is anticipated to yield unique identifications of tissue-specific Treg cells that may be used to treat various locally, regionally and systemically manifested inflammatory and autoimmune disorders. By association such targeted treatment could also affect reduced inflammation and autoimmunity that is systemic, or restricted to distinct tissues, though associated with the disease of the primary target tissue.

These findings suggest that CD4⁺ Tregs with a specific expression pattern are useful in the treatment of inflammatory diseases. The Treg cells should have specific signatures that
i)identify that the cells are CD4⁺ regulatory T-cells,
ii) identify that the regulatory T-cells are tissue type tropic, i.e. they can migrate to the diseased tissue type,
iii) optionally, identify that the Treg cells are diseased tissue tropic, i.e. they are so-called homing cells that can localize in the target diseased tissue region,
iv) identify that the regulatory T-cells are emigrant cells, i.e. they originate from the target tissue (antigen-experienced cells), and
v) optionally, identify that the regulatory T-cells are retained in the target tissue after administration to a subject,
wherein the T-cells have the signatures i), ii) and iv) and optionally iii) and/or v); or the T-cells have the signatures i), ii), iv) and v) and optionally iii), or the T-cells have the signatures i), ii), iv) and v), and optionally iii).

Moreover, the Treg cells should not have signatures that imply that they are thymus emigrant cells or cells immigrating to the lymph nodes from the peripheral blood. However, the present invention is primarily directed to a method for selecting CD4⁺ Treg cells that have the necessary signatures to ensure that they are emigrant/immigrant populations from/to the diseased tissue and that they can localise in the tissues and, moreover, that the cells do not have signatures that identify the cells as being immigrant cells to the lymph node or emigrant from the thymus. The CD4⁺ Tregs may contain further emigrant/immigrant markers (denoted "X") as well as functional markers denoted "Y".

The specific types of Tregs in accordance with the concept of the present invention are described in detail herein with regard to the CD case study. This description is indented to guide the investigational framework of distinct tissue types and/or disease states that represents the present invention. Indeed, it is contemplated that the here described Treg cells are suitable for use in the treatment of inflammatory diseases of the gastrointestinal tract in general and affecting different tissues, however particular proof of concept relates to inflammation of the small bowel.

The present invention is based on the findings that specific homing receptor expression patterns can be used to identify CD4⁺ regulatory T-cells in peripheral circulation as starting materials for therapeutic composition. The specific homing receptor expression pattern varies from tissue to tissue, but it is contemplated that the nature of the signatures (surface protein expression pattern) is the same, irrespective of the diseased tissue in question. Thus, the present invention is based on findings that e.g. Crohn's disease is not a disease defined by a deficiency of Tregs per se, but a deficiency in their ability to recirculate to the diseased tissue, in this case the small bowel. Significantly fewer recent mucosal emigrant and recirculating T-cells were observed in the peripheral blood and diseased tissues of patients (as defined by CD38⁺CD62L⁻CCR9⁺ or a4⁺B7⁺CCR9⁺ or CD103⁺CCR9⁺ expressing cells). These findings have led to identification of CD4⁺ Treg subtypes by surface marker signatures, that may be used in order to purify CD4⁺ Treg cells suitable for therapeutic use.

### Crohn's Disease Case Study of Investigational Framework

It was observed that Treg cells obtained from patients suffering from CD have markedly diminished CCR9 marking. T-cell CCR9 expression is induced within the small bowel lymphoid tissues in parallel with antigen engagement. Export of CCR9-expressing T-cells from the mucosal lymphoid tissues allows recirculation of these cells to regional mucosal tissue. This process is important for establishment of regional and subsequently systemic tolerance. It is anticipated that by targeting varying mucosal tropic and emigrant Treg populations, that the T-cell receptor clonotypes of these populations are restricted to those relevant to tissue-related and disease-related antigens.

The present invention has a proof of concept based on specific CD4⁺ Treg cells for use in the treatment of CD. The Treg cells should have specific signatures that i) identify that the cells are regulatory T-cells,
ii) identify that the regulatory T-cells are tissue type tropic, in this case mucosal tropic, i.e. they can migrate to the diseased area (i.e. small bowel mucosa),
iii) optionally, identify that the Treg cells are diseased tissue tropic, in case of CD in the small bowel the Treg cells are small bowel tropic, i.e. they are so-called homing cells that can localize in the small bowel mucosa
iv) identify that the regulatory T-cells are emigrant cells, i.e. they originate from the target tissue, i.e. in the case of CD in the small bowel the Tregs originate from the small bowel (antigen-experienced cells), and v) optionally, identify that the regulatory T-cells are retained in the diseased tissue (i.e. the small bowel) after administration to a subjectwherein the T-cells have the signatures i), ii) and iv) and optionally iii) and/or v); or the T-cells have the signatures i), ii), iv) and v) and optionally iii), or the T-cells have the signatures i), ii), iv) and v), and optionally iii).

In general CD4⁺ Tregs are defined as a type of T-cell that negatively regulates the immune responses in antigen-guided manner. It is further defined by expression of the transcription factor FOXP3 and comes in two versions, the induced Tregs, which develops from mature T-cells in periphery, and the natural Tregs, which develops from immature T-cells in the thymus.

The present inventors have found that specific homing receptor expression patterns can be used to identify Treg cells in peripheral circulation as starting materials for therapeutic applications, relating to both forms of CD4⁺ Tregs (iTregs and nTregs). CD4⁺ T-cells engage MHCII-antigen complexes. In this sense, CD4⁺ T-cells can be considered to engage antigens extrinsic to the cell presenting said antigens on the MHCII complexes. These cell-extrinsic antigens largely represent antigens from foodstuffs and environment, pathogenic and commensal bacteria and other microbiological pathogens or parasites. Importantly, cell-extrinsic antigens may also represent self-antigens.

These mucosal -tropic and -emigrant CD4⁺ T-cell populations may be dominated by Treg subsets that tolerise antigens from foodstuff and commensal bacteria, due to the fact that this is the largest site of direct interaction of the immune system with these factors. Such CD4⁺ Treg populations, and recirculation thereof, likely underpin homeostatic immune tolerance in the mucosa and systemic tolerance of commensal antigens.

As described in the examples herein, it was observed that Treg cells obtained from patients suffering from CD have markedly diminished CCR9 marking on Tregs. Treg CCR9 expression is induced within the small bowel lymphoid tissues in parallel with antigen engagement. Export of CCR9-expressing Tregs from the mucosal lymphoid tissues allows recirculation of these cells to regional mucosal tissue. This process is important for establishment of regional and subsequently systemic tolerance. It is anticipated that by targeting varying mucosal tropic and emigrant Treg populations, that the T-cell receptor clonotypes of these populations are restricted to those relevant to tissue-related and disease-related antigens.

These findings suggest that Tregs with a specific expression pattern are useful in the treatment of inflammatory or autoimmune diseases of the gastrointestinal tract. The Treg cells should have specific signatures that
i) identify that the cells are regulatory T-cells
ii) identify that the regulatory T-cells are tissue type tropic, i.e. they can migrate to the diseased tissue,
iii) optionally, identify that the Treg cells are diseased tissue tropic, i.e. they are so-called homing cells that can localize in the diseased region of the gastrointestinal tract, and
iv) identify that the regulatory T-cells are emigrant cells, i.e. they originate from the target tissue (educated cells), and
v) optionally, identify that the regulatory T-cells are retained in the target tissue after administration to a subject,
wherein the T-cells have the signatures i), ii) and iv) and optionally iii) and/or v); or the T-cells have the signatures i), ii), iv) and v) and optionally iii), or the T-cells have the signatures i), ii), iv) and v) and optionally iii).

In the present context the term "tissue type" means the specific type of tissue present in the diseased area. As an example the tissue type in relation to Crohn's disease in the small bowel is mucosa and the mucosa is healthy or diseased tissue from the gastrointestinal tract, i.e. the tissue type is not narrowly defined as being exactly from the diseased mucosa, but may be from another part of the gastrointestinal tract. In preferred aspect the tissue type is from the diseased tissue.

In the present context the term "target tissue" means the specific type of tissue present in the diseased area. As an example the target tissue in relation to Crohn's disease in the small bowel is mucosa from the small bowel.

In the present context the terms "tissue type tropic" and "diseased tissue tropic" denotes tropism in relation to the "tissue type" (i.e. tissue in general) and in relation to the "target tissue" (i.e. specific diseased tissue), respectively. The tropism may be to the diseased tissue as well as to the healthy tissue in the diseased area, tissue region or tissue type. It should be noted that immigration of cells from peripheral blood into the stromal/parenchyma of any tissue is mediated by factors intrinsic to the tissue itself, and by factors presented by the vasculature permeating said the tissue. As such, tissue tropism is an interaction of factors expressed by migratory cells with both tissue-centric and tissue vasculature-centric factors. This duality results in often-significant overlap in the functional elements of migratory cells with tropism towards related yet distinct tissue types and tissue subtypes.

The specific types of Tregs in accordance with the concept of such an invention are described in detail herein. It is contemplated that the Treg cells are suitable for use in the treatment of inflammatory diseases of the gastrointestinal tract and the particular proof of concept relates to inflammation of the small bowel in particular, but through mucosal tropism also for inflammatory diseases located in the whole mucosal gastrointestinal tract.

As mentioned above CD can affect the whole gastrointestinal tract, notably the distal part of the small bowel, the colon, the proximal part of the gastrointestinal tract or the anal canal and perianal area. It is envisaged that the Treg cells suitable for use in the treatment of CD mainly have the same signatures irrespective of which part of the gastrointestinal tract that is affected apart from the signature that identifies that the regulatory T-cells are gastrointestinal tropic. It is believed that the signature in this respect must be specific, i.e. proximal gastrointestinal tract tropic, large bowel tropic, small bowel tropic, anal canal tropic etc. dependent on the localisation of CD.

An invention as presented herein has a proof of concept based on specific Treg cells for use in the treatment of CD in the small bowel. The CD4⁺ Treg cells should have specific signatures as defined above. It is generally preferred that the Treg cells should have specific signatures that
i) identify that the cells are regulatory T-cells,
ii) identify that the regulatory T-cells are tissue type tropic, in this case mucosal tropic, i.e. they can migrate to the diseased area (mucosa),
iii) optionally, identify that the Treg cells are diseased tissue tropic, in case of CD in the small bowel the Treg cells are small bowel tropic, i.e. they are so-called homing cells that can localize in the small bowel,
iv) identify that the regulatory T-cells are emigrant cells, i.e. they originate from the target tissue, i.e. in the case of CD in the small bowel the Tregs originate from the small bowel (educated cells), and
v) optionally, identify that the regulatory T-cells are retained in the diseased tissue (the small bowel) after administration to a subject.

As seen from the above it is generally preferred that the signatures i), ii) and iv) are mandatory when the Tregs are used in the treatment of CD in the small bowel. However, it is contemplated that, different treatment strategies, or treatment of inflammatory diseases such as Crohn's disease affecting other parts of the gastrointestinal tract do not require the same signatures; thus, it is contemplated that the Tregs must have the ), ii) and iv) and optionally iii) and/or v); or the T-cells have the signatures i), ii), iv) and v) and optionally iii), or the T-cells have the signatures i), ii), iv) and v) and optionally iii).

In analogous manner when the CD is localized in the colon the Treg cells should have specific signatures that
i) identify that the cells are regulatory T-cells,
ii) identify that the regulatory T-cells are mucosal tropic, i.e. they can migrate to the diseased area (mucosa),
iii) optionally, identify that the Treg cells are colon tropic, i.e. they are so-called homing cells that can localize in the colon,
iv) identify that the regulatory T-cells are emigrant cells, i.e. they originate from the target tissue, i.e. from the colon, (educated cells), and
v) optionally, identify that the regulatory T-cells are capable of being retained in the target tissue, i.e. the colon, after administration to a subject.

Tregs for treatment of CD in other locations of the gastrointestinal tract have the same kind of signatures, but the signatures relate to the target tissue of the gastrointestinal tract.

In general Treg cells are defined as a type of CD4⁺ cell that negatively regulates the immune responses. It is further defined by expression of the transcription factor FOXP3 and comes in two versions, the induced Tregs, which develops from mature T-cells in periphery, and the natural Tregs, which develops from immature T-cells in the thymus.

The CD4⁺ Treg cells should have specific signatures that
i) identify that the cells are regulatory T-cells,
ii) identify that the regulatory T-cells are tissue type tropic, i.e. they can migrate to the diseased tissue (mucosa),
iii) optionally, identify that the Treg cells are diseased tissue tropic, i.e. they are so-called homing cells that can localize in the diseased part of the gastrointestinal tract,
iv) identify that the regulatory T-cells are emigrant cells, i.e. they originate from the target tissue (educated cells), and
v) optionally, identify that the regulatory T-cells are capable of being retained in the target tissue of the gastrointestinal tract after administration to a subject.

The present inventors have found that a preferred signature for identifying that the Treg cells are mucosal tropic is α4β7⁺, α4⁺β7⁺.

A preferred signature for identifying that the Treg cells can be retained in mucosal tissue is α4⁺αE⁺ β7^{high}.

The specific types of Tregs in accordance with the present invention are described in detail herein using CD localized in the small bowel as an example, but without limiting the invention thereto. It is contemplated that the Treg cells are suitable for use in the treatment of inflammatory diseases of the small bowel, especially in the treatment of CD.

If CD is located to the small bowel, the diseased as well as the target tissue is the small bowel.

Thus, the identification of a specific Treg cell population in peripheral blood, which is likely to represent mucosal emigrants with a strong propensity to recirculate to the small bowel, presents a further means to identify Treg cells based on homing receptor patterns for adoptive immunotherapy. Coupled to Treg markers and, optionally a marker set for cells marked for mucosal retention, the present inventors were able to identify four overlapping subsets of Tregs with therapeutic potential in CD located in the small bowel. Analogously, Treg cells with therapeutic potential in CD located in other parts of the gastrointestinal tract can be identified or Tregs with therapeutic potential in other inflammatory diseases of the gastrointestinal tract.
1. CD4⁺ Treg cells that have signatures for
   i) identifying that the T-cells are regulatory T-cells,
   ii) identifying that the Treg cells are mucosal tropic, i.e. they can migrate to mucosal tissue,
   iv) identifying that the Treg cells are emigrant cells, i.e. they originate from the bowel.
2. CD4⁺Treg cells that have signatures for
   i) identifying that the T-cells are regulatory Tcells,
   ii) identifying that the Treg cells are mucosal tropic, i.e. they can migrate to mucosal tissue,
   iii) identifying that the Treg cells are small bowel tropic, i.e. homing cells that can localize in the small bowel, and
   iv) identifying that the Treg cells are emigrant cells, i.e. they originate from the bowel.
3. CD4⁺Treg cells that have signatures for
   i) identifying that the T-cells are regulatory Tcells,
   ii) identifying that the Treg cells are mucosal tropic, i.e. they can migrate to mucosal tissue,
   iv) identifying that the Treg cells are emigrant cells, i.e. they originate from the bowel. and
   v) identifying that the Treg cells are marked for mucosal retention.
4. CD4⁺Treg cells that have signatures for
   i) identifying that the T-cells are regulatory Tcells,
   ii) identifying that the Treg cells are mucosal tropic, i.e. they can migrate into mucosal tissue,
   iii) identifying that the Treg cells are small bowel tropic, i.e. homing cells that can localize in the small bowel,
   iv) identifying that the Treg cells are emigrant cells, i.e. they originate from the bowel, and
   v) identifying that the Treg cells are marked for mucosal retention.

As mentioned herein before, the preferred CD4⁺ Treg cells are CD4⁺ Treg cells that have signatures for
i) identifying that the T-cells are regulatory Tcells,
ii) identifying that the Treg cells are mucosal tropic, i.e. they can migrate into mucosal tissue,
iii) optionally, identifying that the Treg cells are small bowel tropic, i.e. homing cells that can localize in the small bowel,
iv) identifying that the Treg cells are emigrant cells, i.e. they originate from the bowel, and
v) optionally identifying that the Treg cells are marked for mucosal retention.

Other alternatives may be derived from the description herein.

As will be explained in detail herein, the preferred signature for identifying that the T-cells are regulatory T-cells is, CD4⁺CD25^{hi}, CD4⁺CD25^{hi}CD127^{lo}, or CD4⁺Yₙ, where Y is a functional marker and n is an integer of 1 or more. Functional markers Y are described herein below.

The preferred signature for identifying that the Treg cells are mucosal tropic is α4β7⁺ or α4⁺β7⁺.

The preferred signature for identifying that the Treg cells are small bowel tropic, i.e. homing cells, is CCR9⁺ or in combination with one or more X signatures as defined herein.

The preferred signature for identifying that the Treg cells are educated cells (emigrants) is CD62L⁻ and/or CD38⁺ and/or α4⁺αE⁺β7^{high}, one or more X signatures as defined herein.

The preferred signature for identifying that the Treg cells are capable of being retained in mucosal tissue is α4⁺αE⁺β7^{high} or α4⁻αE⁺β7⁺, and/or one or more of any of X, and Y as defined herein.

Other signatures are CD45RA⁻/CD45RO⁺, or CCR7-.

Thus, in preferred aspect of the invention and relating to inflammatory disease of the small bowel, the Treg cells are selected from the following:
CD4⁺CD25^{hi}α4β7⁺CD62L⁻ CD38⁺
CD4⁺CD25^{hi}α4⁺β7⁺CD62L⁻ CD38⁺
CD4⁺CD25^{hi}CD127^{lo}α4β7⁺CD62L⁻ CD38⁺
CD4⁺CD25^{hi}CD127^{lo}α4⁺β7⁺CD62L⁻ CD38⁺

CD4⁺CD25^{hi}α4β7⁺CD62L⁻
CD4⁺CD25^{hi}α4⁺β7⁺CD62L⁻
CD4⁺CD25^{hi}CD127^{lo}α4β7⁺CD62L⁻
CD4⁺CD25^{hi}CD127^{lo}α4⁺β7⁺CD62L⁻

CD4⁺CD25^{hi}α4β7⁺CD62L⁻ CD38⁺CCR9⁺
CD4⁺CD25^{hi}α4⁺β7⁺CD62L⁻ CD38⁺ CCR9⁺
CD4⁺CD25^{hi}CD127^{lo}α4β7⁺CD62L⁻ CD38⁺ CCR9⁺
CD4⁺CD25^{hi}CD127^{lo}α4⁺β7⁺CD62L⁻ CD38⁺ CCR9⁺

CD4⁺CD25^{hi}α4β7⁺CD62L⁻ CCR9⁺
CD4⁺CD25^{hi}α4⁺β7⁺CD62L⁻ CCR9⁺
CD4⁺CD25^{hi}CD127^{lo}α4β7⁺CD62L⁻ CCR9⁺
CD4⁺CD25^{hi}CD127^{lo}α4⁺β7⁺CD62L⁻ CCR9⁺

CD4⁺CD25^{hi}α4β7^{high}αE⁺CD62L⁻ CD38⁺
CD4⁺CD25^{hi}α4⁺β7^{high}αE⁺CD62L⁻ CD38⁺
CD4⁺CD25^{hi}CD127^{lo}α4β7^{hi}αE⁺CD62L⁻ CD38⁺
CD4⁺CD25^{hi}CD127^{lo}α4⁺β7^{hi}αE⁺CD62L⁻ CD38⁺

CD4⁺CD25^{hi}α4β7^{high}αE⁺CD62L⁻
CD4⁺CD25^{hi}α4⁺β7^{high}αE⁺CD62L⁻
CD4⁺CD25^{hi}CD127^{lo}α4β7^{hi}αE⁺CD62L⁻
CD4⁺CD25^{hi}CD127^{lo}α4⁺β7^{hi}αE⁺CD62L⁻

CD4⁺CD25^{hi}α4β7^{high}αE⁺ CD38⁺
CD4⁺CD25^{hi}α4⁺β7^{high}αE⁺ CD38⁺
CD4⁺CD25^{hi}CD127^{lo}α4β7^{hi}αE⁺ CD38⁺
CD4⁺CD25^{hi}CD127^{lo}α4⁺β7^{hi}αE⁺ CD38⁺

CD4⁺CD25^{hi}α4β7^{high}αE⁺CD62L⁻ CD38⁺ CCR9⁺
CD4⁺CD25^{hi}α4⁺β7^{high}αE⁺CD62L⁻ CD38⁺ CCR9⁺
CD4⁺CD25^{hi}CD127^{lo}α4β7^{hi}αE⁺CD62L⁻ CD38⁺ CCR9⁺
CD4⁺CD25^{hi}CD127^{lo}α4⁺β7^{hi}αE⁺CD62L⁻ CD38⁺ CCR9⁺

CD4⁺CD25^{hi}α4β7^{high}αE⁺CD62L⁻ CCR9⁺
CD4⁺CD25^{hi}α4⁺β7^{high}αE⁺CD62L⁻CCR9⁺
CD4⁺CD25^{hi}CD127^{lo}α4β7^{hi}αE⁺CD62L⁻ CCR9⁺
CD4⁺CD25^{hi}CD127^{lo}α4⁺β7^{hi}αE⁺CD62L⁻ CCR9⁺

CD4⁺CD25^{hi}α4β7^{high}αE⁺ CD38⁺ CCR9⁺
CD4⁺CD25^{hi}α4⁺β7^{high}αE⁺ CD38⁺ CCR9⁺
CD4⁺CD25^{hi}CD127^{lo}α4β7^{hi}αE⁺ CD38⁺ CCR9⁺
CD4⁺CD25^{hi}CD127^{lo}α4⁺β7^{hi}αE⁺ CD38⁺ CCR9⁺

In all the specific Treg cell populations described herein (such as those mentioned above) it is within the scope of the present invention that whenever
a) CD4⁺ is mentioned it may be replaced with CD4⁺CD25^{hi} or CD4⁺CD25^{hi}CD127^{lo},
b) CD62L⁻ is mentioned this signature may be replaced or supplemented with CD38⁺ or with α4⁺αE⁺ β7^{high} or with CD38⁺α4⁺αE⁺ β7^{high}, and whenever
c) α4⁺β7^{high}αE⁺ is mentioned it may be replaced with α4⁺β7⁺αE⁺.

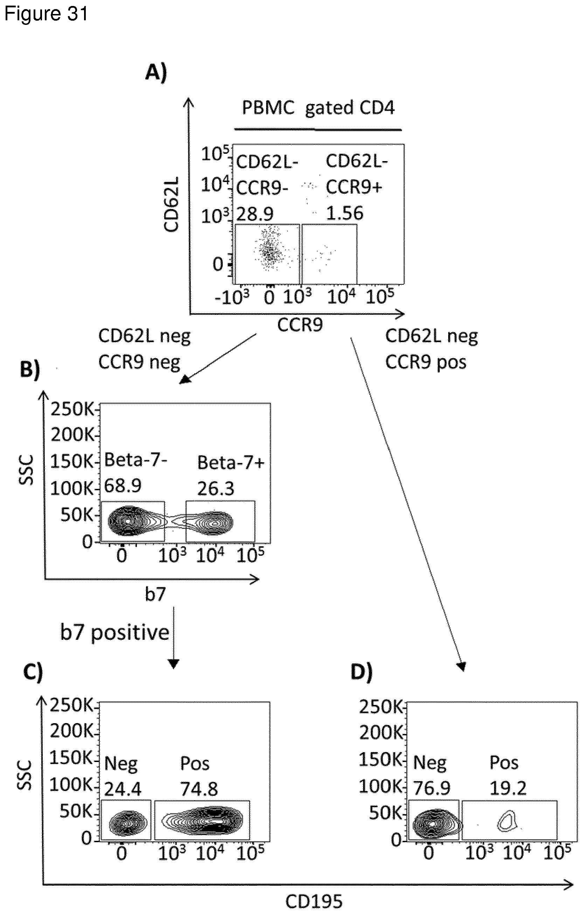

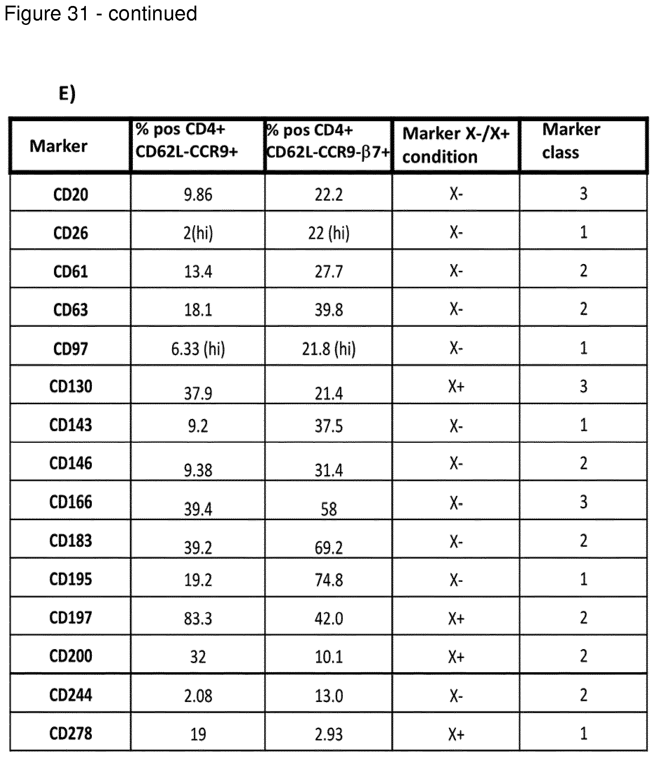

As described herein in details the above CD4⁺Treg cells may comprise one or more further signatures relating to the emigrant and/or immigrant nature of the CD4⁺ Treg cells. Such signatures are denoted "X". As explained herein examples of signatures X are given in **Figure. 31**. The CD4⁺Treg cells may also comprise signatures of functional nature, Y. However, as explained herein signatures relating to emigrant cells from thymus and immigrant cells from the peripheral blood to the lymph nodes should be excluded. In other preferred aspects such an invention and relating to CD in other parts of the gastrointestinal tract than the small bowel, the Treg cells are selected from the above- and below mentioned, wherein the Treg cells further may contain one or more of X and/or Y.
CD4⁺CD25^{hi}α4β7⁺CD62L⁻ CD38⁺X/Y
CD4⁺CD25^{hi}α4⁺β7⁺CD62L⁻ CD38⁺ X/Y
CD4⁺CD25^{hi}CD127^{lo}α4β7⁺CD62L⁻ CD38⁺ X/Y
CD4⁺CD25^{hi}CD127^{lo}α4⁺β7⁺CD62L⁻ CD38⁺ X/Y

CD4⁺CD25^{hi}α4β7⁺CD62L⁻ X/Y
CD4⁺CD25^{hi}α4⁺β7⁺CD62L⁻ X/Y
CD4⁺CD25^{hi}CD127^{lo}α4β7⁺CD62L⁻ X/Y
CD4⁺CD25^{hi}CD127^{lo}α4⁺β7⁺CD62L ⁻ X/Y

CD4⁺CD25^{hi}α4β7⁺ CD38⁺X/Y
CD4⁺CD25^{hi}α4⁺β7⁺ CD38⁺ X/Y
CD4⁺CD25^{hi}CD127^{lo}α4β7⁺ CD38⁺ X/Y
CD4⁺CD25^{hi}CD127^{lo}α4⁺β7⁺ CD38⁺ X/Y

CD4⁺CD25^{hi}α4β7⁺CD62L⁻ CD38⁺CCR9⁺ X/Y
CD4⁺CD25^{hi}α4⁺β7⁺CD62L⁻ CD38⁺ CCR9⁺ X/Y
CD4⁺CD25^{hi}CD127^{lo}α4β7⁺CD62L⁻ CD38⁺ CCR9⁺ X/Y
CD4⁺CD25^{hi}CD127^{lo}α4⁺β7⁺CD62L⁻ CD38⁺ CCR9⁺ X/Y

CD4⁺CD25^{hi}α4β7⁺CD62L⁻ CCR9⁺ X/Y
CD4⁺CD25^{hi}α4⁺β7⁺CD62L⁻ CCR9⁺ X/Y
CD4⁺CD25^{hi}CD127^{lo}α4β7⁺CD62L⁻ CCR9⁺ X/Y
CD4⁺CD25^{hi}CD127^{lo}α4⁺β7⁺CD62L⁻ CCR9⁺ X/Y

CD4⁺CD25^{hi}α4β7⁺ CD38⁺CCR9⁺ X/Y
CD4⁺CD25^{hi}α4⁺β7⁺ CD38⁺ CCR9⁺ X/Y
CCD4⁺CD25^{hi}CD127^{lo}α4β7⁺ CD38⁺ CCR9⁺ X/Y
CD4⁺CD25^{hi}CD127^{lo}α4⁺β7⁺ CD38⁺ CCR9⁺ X/Y

CD4⁺CD25^{hi}α4β7^{high}αE⁺CD62L⁻ CD38⁺ X/Y
CD4⁺CD25^{hi}α4+β7^{high}αE⁺CD62L⁻ CD38⁺ X/Y
CD4⁺CD25^{hi}CD127^{lo}α4β7^{hi}αE⁺CD62L⁻ CD38⁺ X/Y
CD4⁺CD25^{hi}CD127^{lo}α4⁺β7^{hi}αE⁺CD62L⁻ CD38⁺ X/Y

CD4⁺CD25^{hi}α4β7^{high}αE⁺CD62L⁻ X/Y
CD4⁺CD25^{hi}α4⁺β7^{high}αE⁺CD62L⁻ X/Y
CD4⁺CD25^{hi}CD127^{lo}α4β7^{hi}αE⁺CD62L⁻ X/Y
CD4⁺CD25^{hi}CD127^{lo}α4⁺β7^{hi}αE⁺CD62L⁻ X/Y

CD4⁺CD25^{hi}α4β7^{high}αE⁺ CD38⁺ X/Y
CD4⁺CD25^{hi}α4⁺β7^{high}αE⁺ CD38⁺ X/Y
CD4⁺CD25^{hi}CD127^{lo}α4β7^{hi}αE⁺ CD38⁺ X/Y
CD4⁺CD25^{hi}CD127^{lo}α4⁺β7^{hi}αE⁺ CD38⁺ X/Y

CD4⁺CD25^{hi}α4β7^{high}αE⁺CD62L⁻ CD38⁺ CCR9⁺ X/Y
CD4⁺CD25^{hi}α4⁺β7^{high}αE⁺CD62L⁻ CD38⁺ CCR9⁺ X/Y
CD4⁺CD25^{hi}CD127^{lo}α4β7^{hi}αE⁺CD62L⁻ CD38⁺ CCR9⁺ X/Y
CD4⁺CD25^{hi}CD127^{lo}α4⁺β7^{hi}αE⁺CD62L⁻ CD38⁺ CCR9⁺ X/Y

CD4⁺CD25^{hi}α4β7^{high}αE⁺CD62L⁻CCR9⁺ X/Y
CD4⁺CD25^{hi}α4⁺β7^{high}αE⁺CD62L⁻ CCR9⁺ X/Y
CD4⁺CD25^{hi}CD127^{lo}α4β7^{hi}αE⁺CD62L⁻ CCR9⁺ X/Y
CD4⁺CD25^{hi}CD127^{lo}α4⁺β7^{hi}αE⁺CD62L⁻ CCR9⁺ X/Y

CD4⁺CD25^{hi}α4β7^{high}αE⁺ CD38⁺ CCR9⁺ X/Y
CD4⁺CD25^{hi}α4⁺β7^{high}αE⁺ CD38⁺ CCR9⁺ X/Y
CD4⁺CD25^{hi}CD127^{lo}α4β7^{hi}αE⁺ CD38⁺ CCR9⁺ X/Y
CD4⁺CD25^{hi}CD127^{lo}α4⁺β7^{hi}αE⁺ CD38⁺ CCR9⁺ X/Y
X/Y means that at least one of X, and/or at least one of Y may be present and X, and Y may be X⁺ or X⁻, Y⁺ or Y⁻, and one or more X, and/or Y may be present. X is a signature indicating that the Tregs can localize, have emigrated from or are marked for preferential retention in the specific part of the small bowel that is diseased. X may be X⁺ or X may be X⁻. Y is a signature indicating that the T-cells can exercise immunosuppressive functions or do not promote pro-inflammatory activities Y may be Y⁺ or Y may be Y⁻.

However, as described in the experimental part herein an alternative signature is CD4⁺α4⁺β7^{high}αE⁺CCR9⁺ or CD4⁺α4⁺β7^{high}αE⁺CCR9⁺X/Y

Irrespective of the gastrointestinal location of the inflammation, any of the signatures may also comprise CD62L⁻ and/or CD38⁺.

As described in the experimental part herein it was found that β7^{hi} cells express higher levels of β7 owing to the fact that they require additional β7 to pair with αE, suggesting β7^{hi} cells express both the α4β7 and αEβ7 integrin pairs. The significance of this is that α4β7 is thought to be required for migration into mucosal tissues, while αEβ7 is required for retention. αEβ7 may also in some instances be considered to represent an identifier of recent mucosal emigration.

As mentioned above, the present invention relates to specific Tregs for treating inflammatory disorders of the bowel. To this end it is important to identify important subtypes of Treg cells, enabling their accurate purification from human tissues. This knowledge has been built on unique analyses of specimens from patients with CD, healthy individuals, and in some respects from patients with colorectal cancer.

With regard to marker X in the above claims, relating to markers that denote a signature indicating tissue localisation, emigration or immigration, further analyses revealed markers of particular interest.

Figure 31 shows an example of different adhesion molecule expression in the CD4⁺CD62L⁻CCR9⁺ population in comparison to the CD4⁺CD62L⁻CCR9⁻β7⁺ population that is targeted to mucosal tissues in general (**Figure 31** **A to D**). In this example, CD195 (CCR5) is almost absent in the CD4⁺CD62L⁻CCR9⁺ population. It is thus anticipated that CD195 may be used as a marker of preferred condition X-, with which to select for mucosal emigrant, immigrant and educated CD4+ Treg cells with small bowel tropism. The table presented in **Figure 31** **E** summarises other migratory-type markers associated with the CD4⁺CD62L⁻CCR9⁺ population. The markers positively correlated are of condition X+ and the markers negatively correlated are of condition X-. In the preferred aspect markers denoted X+ are used as a positive selection marker and markers denoted X- are used as a negative selection marker for the purification of mucosal emigrant, immigrant and educated CD4+ Treg cells with small bowel tropism. Each marker in this table is also assigned a class, where class 1 represents a strong association with the CD4⁺CD62L⁻CCR9⁺ population and high functional significance. Class 2 represents a strong association with the CD4⁺CD62L⁻CCR9⁺ population or high functional significance. Class 3 represents weak association and/or uncertain functional significance.

Any of these markers, X, can be included in a CD4⁺ Treg cell population according to the invention or used in a method of the invention to select the right signature pattern on the CD4⁺ Treg cells. As shown in Fig. 31 markers of class 1 include CD26, CD97, CD143, CD195 and CD278. Markers of class 2 include CD61, CD63, CD146, CD183, CD197, CD200, and CD244. Markers of class 3 include CD20, CD130, and CD166.

The aforementioned markers relate to tissue localisation, emigration, immigration and retention.

Analyses of cells with CD4⁺CD25^{hi}CD127^{lo}α4⁺β7⁺ character revealed strong enrichment of surface markers that denote regulatory function, and a restriction of markers that generally denote pro-inflammatory functions.

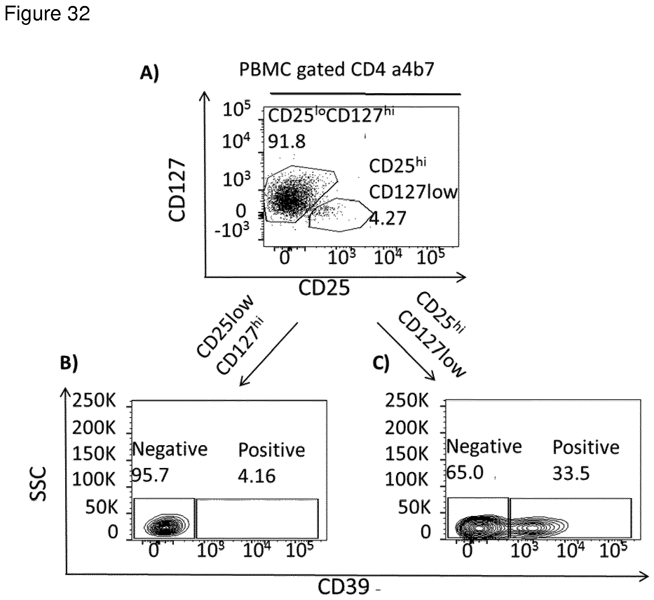

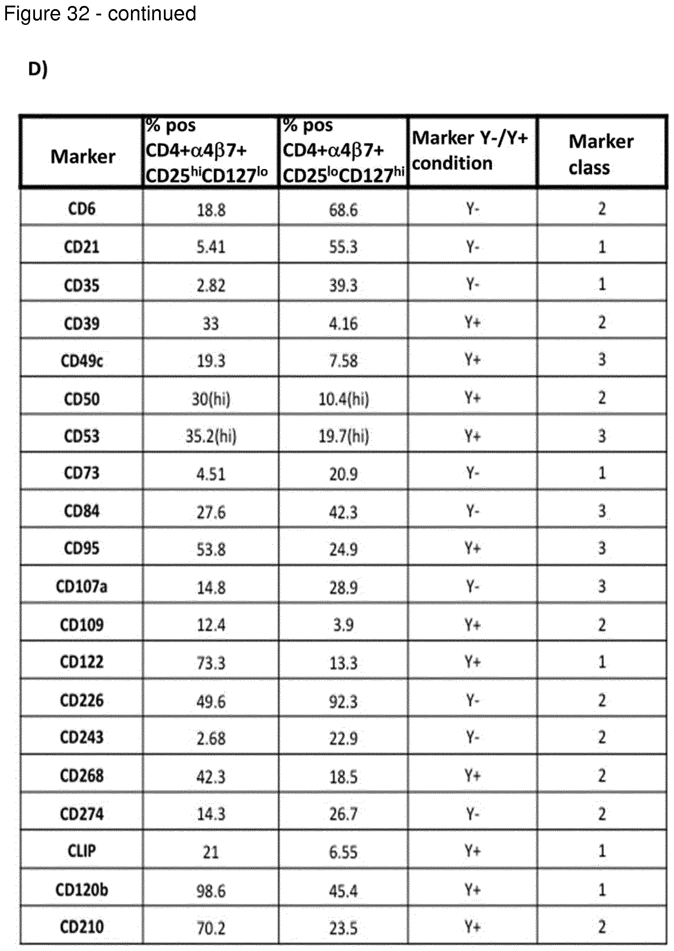

Figure 32 shows an example of a functional marker, CD39 (ENTPD1), which is a putative immunosuppressive element on the surface of T-cells, and which is enriched in the CD4⁺CD25^{hi}CD127^{lo}α4⁺β7⁺ population (**Figure 32** **A to C**).. It is thus anticipated that CD39 may be used as a marker of preferred condition Y+, with which to select for Treg cells within mucosal emigrant, immigrant and educated CD4+ T-cell populations. The table presented in Figure 32D summarises other functional-type markers associated with the CD4⁺CD25^{hi}CD127^{lo}α4⁺β7⁺ population. The markers positively correlated are of condition Y+, and largely represent entities with putative immunosuppressive activities, where in the preferred aspect they are used as a positive selection marker for the purification of Treg cells from mucosal emigrant, immigrant and educated CD4+ T-cell populations. The markers negatively correlated are of condition Y-, and largely represent entities with putative pro-inflammatory activities, where in the preferred aspect they are used as a negative selection marker for the purification of Treg cells from mucosal emigrant, immigrant and educated CD4+ T-cell populations. Each marker in this table is also assigned a class, where class 1 represents a strong association with the CD4⁺CD25^{hi}CD127^{lo}α4⁺β7⁺ population and high functional significance. Class 2 represents a strong association with the CD4⁺CD25^{hi}CD127^{lo}α4⁺β7⁺ population or high functional significance. Class 3 represents weak association and/or uncertain functional significance.

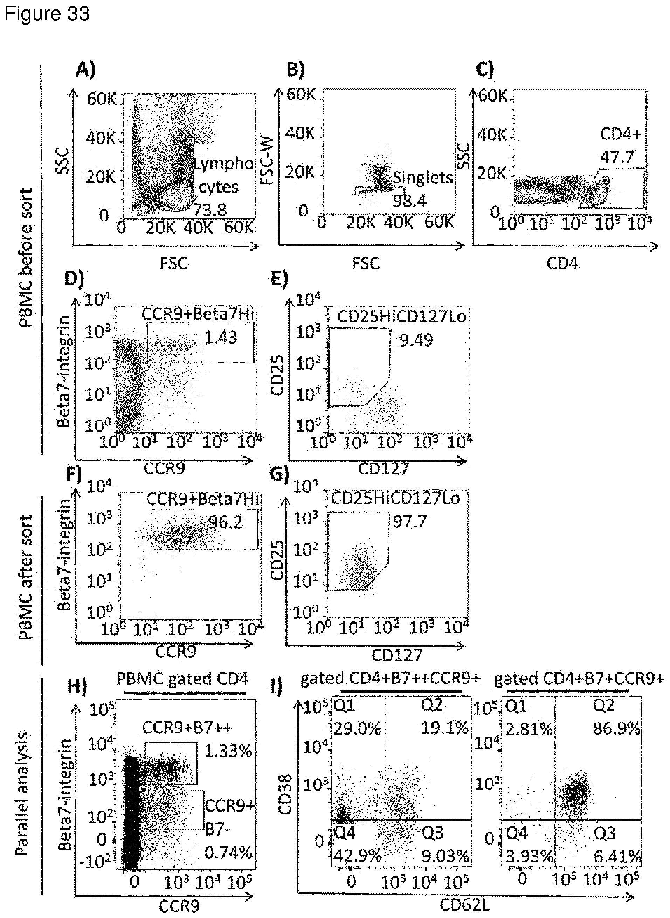

Any of these markers, Y, can be included in a CD4⁺ Treg cell population according to the invention or used in a method of the invention to select the right signature pattern on the CD4⁺ Treg cells. As shown in Fig. 33 markers of class 1 include CD21, CD35, CD73, CD122, CLIP, and CD120b. Markers of class 2 include CD6, CD39, CD50, CD109, CD226, CD243, CD268, CD274 and CD210. Markers of class 3 include CD49c, CD53, CD84, CD95, and CD107a.

The CD4⁺ Treg cell may thus have specific signatures that:
i) identify that the cells are regulatory T-cells, typically CD4⁺CD25^{hi}CD127^{lo}
ii) identify that the regulatory T-cells are tissue type tropic, i.e. they can migrate to the diseased area (i.e. small bowel mucosa), typically α4β7⁺ or α4⁺β7⁺
iii) optionally, identify that the Treg cells are diseased tissue tropic, i.e. they are so-called homing cells that can localize in the diseased tissue, typically CCR9⁺, and
iv) identify that the regulatory T-cells are emigrant cells, i.e. they originate from the target tissue, i.e. the diseased tissue (antigen-experienced cells), typically CD62L⁻ CD38⁺, and
v) optionally, identifying that the Treg cells are marked for mucosal retention typically α4⁺αE⁺ β7^{high} or α4αE⁺ β7^{high},
and optionally one of more X signatures selected from
a) CD26, CD97, CD143, CD195, CD278,
b) CD61, CD63, CD146, CD183, CD197, CD200, CD244,
c) CD20, CD130, CD166,
and optionally one or more Y signatures selected from
d) CD21, CD35, CD73, CD122, CLIP, CD120b,
e) CD6, CD39, CD50, CD109, CD226, CD243, CD268, CD274, CD210,
f) CD49c, CD53, CD84, CD95, CD107a.

Moreover, it is preferred that the CD4⁺ Treg cells are not recent thymic emigrants or are immigrant cells to the lymph nodes from the peripheral circulation. Therefore, the CD4⁺ Treg cells may have one or more of the following signatures:
h) CD62L⁺ i.e. to exclude cells that gain access to lymph nodes via HEV (high endothelial venules),
j) CCR9⁺CD45RA⁺, CCR9⁺CCR7⁺, CCR9⁺CD62L⁺, and/or CCR9⁺CD45RO⁻ to exclude cells that are recent thymic emigrants, e.g. cells that are CCR9⁺CD45RA⁺ or CCR9⁺CCR7⁺ or CCR9⁺CD62L⁺. Any combination of these markers for the denoted +/condition is considered relevant to the exclusion of recent thymic emigrant de novo T-cells, and for the parallel exclusion of resting central memory cells that have not been recently activated against antigen (i.e. should carry the a CD45RA⁺/CD45RO⁻ character),
k) CCR9⁺CCR7⁺CD62L⁺CD45RA⁺CD45RO⁻ to exclude all h) and j) above.

Herein is given a number of examples of possible combinations of signatures that are within the scope of the present invention. This text does not exclude any possible combination of signatures that can be derived from the specification and appended claims.

### Investigational Framework to Identify Diseased Tissue-Specific Treg Populations - Identification methods according to the invention

Inflammatory and autoimmune disorders are most commonly localised to a particular tissue. In the example of CD, inflammatory lesions are primarily localised in the small bowel. Whether the primary immunological dysfunction within the disease tissue is T-cell-centric or not, one should regard at least an accumulated defect in T-cell function to be involved in most inflammatory and autoimmune disorders, considering their central controlling function in adaptive immunity. Furthermore, it should be suspected that disease-related and disease-vicinal T-cell responses are driven by incident antigen availability at the diseased tissue location, whether these antigens represent tissue-restricted self-antigens, or foreign antigens available primarily at the diseased tissue site. This expectation is borne from the fact that, in the absence of a T-cell malignancy, there must be driving stimuli for T-cell activation and clonal expansion. T-cell receptor engagement is requisite for T-cell activation, thus the underlying stimuli is most likely antigenic in nature. In this conception, the functional subdivision of T-cells, into Teff or Treg and their respective subtypes, for instance, is critically important in the context of antigen-reactivity as defined by the clonotypes produced (via specific antigen engagement and clonal expansion) in the T-cell populations and subpopulations. Identifying Treg populations that are clonally restricted to the diseased tissue, and the disease state, can be considered as the primary criteria for identification of Treg populations suitable as starting materials for the manufacture of cellular immunotherapeutic compositions to treat inflammatory and autoimmune disorders. This concept guides the systematic study of inflammatory and autoimmune diseases in a tissue-restricted manner to identify subpopulations, which are restricted to tissue- and thus disease- relevant clonotypes.

It is contemplated that the methods described herein to identify/select CD4⁺ Treg cells that are suitable for use as starting materials in cellular immunotherapy can be applied to many types of inflammatory or autoimmune disorders. These include those affecting; the alimentary mucosal surfaces e.g. inflammatory bowel diseases, acute celiac disease, primary biliary cirrhosis and appendicitis; liver e.g. primary sclerosing cholangitis and autoimmune hepatitis; pulmonary mucosal surfaces or pleura e.g. chronic obstructive pulmonary disease and bronchiectasis; other mucosal surfaces including e.g. vaginitis, cervicitis, endometriosis, rhinitis and sinusitis and behçet disease; Lingual tissue e.g. glossitis; pancreas e.g. Type-1 diabetes and autoimmune pancreatitis; skin e.g. psoriasis, acute and chronic dermatitis including various eczemas and dermatitis herpetiformis, erythema nodosum and pyoderma gangrenosum; vascular tissues e.g. vasculitis, phlebitis and atherosclerosis; eyes e.g. keratoconjunctivitis sicca, uveitis, iritis, episcleritis; skeletal musculature e.g. polymyalgia rheumatic and tendonitis; synovial tissues e.g. bursitis; joint tissues and surfaces e.g. rheumatoid arthritis, ankylosing spondylitis and sacroiliitis; neural tissues e.g. autoimmune limbic encephalitis, chronic focal encephalitis and Hashimoto's encephalopathy; nervous tissues e.g. multiple sclerosis, Guillain-Barre syndrome and chronic inflammatory demyelinating polyneuropathy; breast tissues e.g. mastitis. These inflammatory conditions may relate wholly or in part to autoimmune reactions within individuals, and/or wholly or in part relate to reactions against foreign antigens. The invention relates to a method for identification of immunosuppressive CD4⁺ regulatory T-cells, partially or wholly restricted to disease-affected tissues, and to a method for expanding such cells in manufacture of therapeutic preparations.

As mentioned above, the present invention relates to a method for identifying CD4⁺ Treg cells suitable for use as starting material in cellular immunotherapy, the method comprising
i) analysing samples from target tissue A to identify CD4⁺ Treg cells with migratory character between the diseased tissue, collecting lymphatics, peripheral blood, distinct tissue adjacent to the diseased target tissue A and/or distinct tissue that is not vicinal though has migratory Treg communication with target tissue A,
ii) optionally analysing samples from target tissue A to identify CD4⁺ Treg cells with functional character in tissue A,
iii) optionally analysing samples from lymphatic tissue B to identify CD4⁺ Treg cells with migratory character between disease draining lymphatics and non-disease draining lymphatics of diseased or non-diseased target tissue A,
iv) optionally analysing samples from lymphatic tissue B to identify CD4⁺ Treg cells with functional character in tissue B where the Treg cells are also emigrant from target tissue A,
v) analysing samples from peripheral blood, tissue C, to identify CD4⁺ Treg cells with migratory character and/or functional character where the Treg cells are also emigrant from target tissue A,
vi) analysing sample(s) from tissue compartments A and/or B and C, that are analytically or physically depleted of emigrants from thymus and/or immigrants from peripheral blood to a lymph node, to restrict analyses to CD4⁺ Treg cells of target tissue A origin and/or tropism,
to identify emigrant CD4⁺ Treg cell populations of target tissue A,
to identify emigrant CD4⁺ Treg cell populations with propensity to immigrate to target tissue A,
to identify a migratory and/or functional defect in the CD4⁺ Treg cell population identified as expressing migratory and/or functional elements specific for target tissue A in any of tissue A, B or C, and
whereby a combination of surface or intracellular markers on CD4⁺ Treg cells is identified. In combination this identify which surface or intracellular markers should be present, and which markers should not be present in CD4⁺ Treg cell populations suitable for use as starting material in cellular immunotherapy.

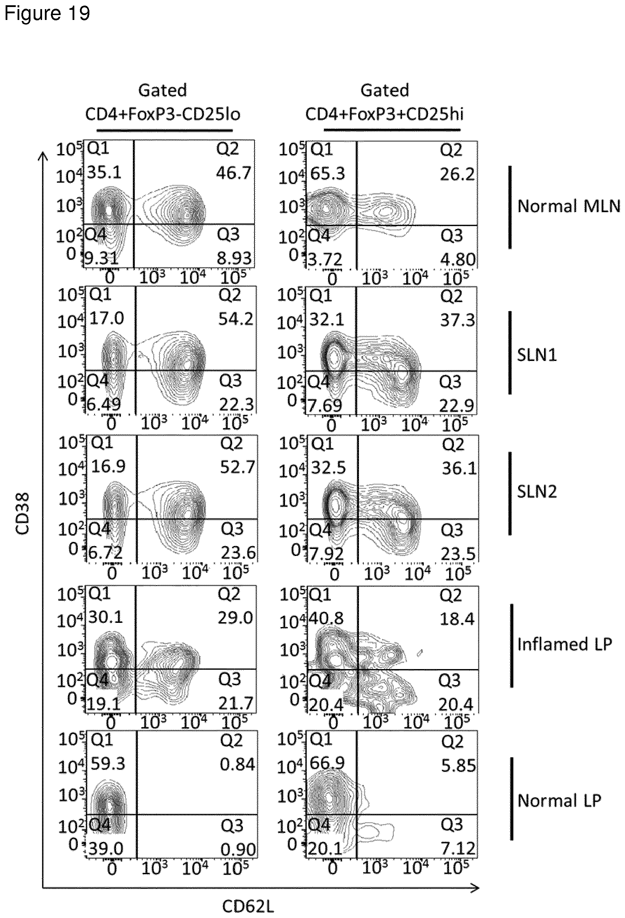

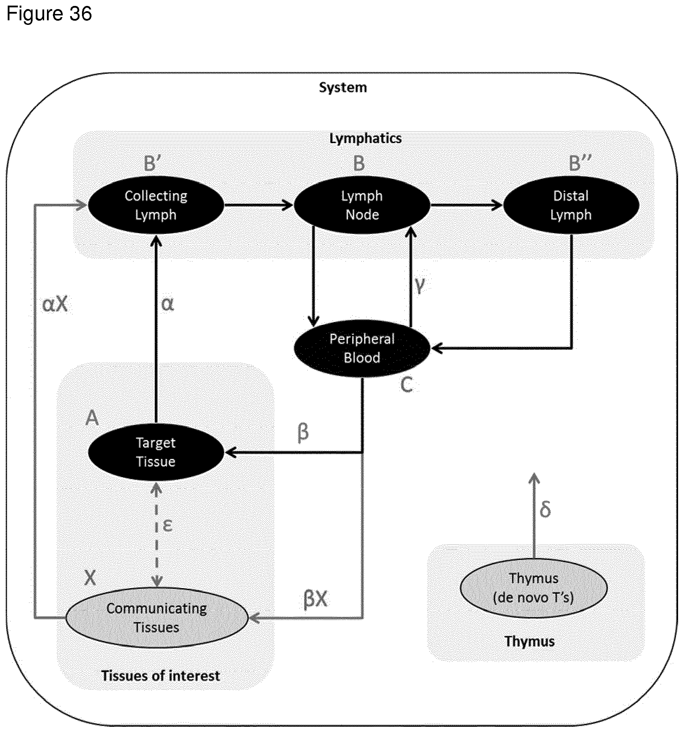

**Figure 36** shows the framework of investigation relating to a disease state of interest, where diseased target tissue is represented as tissue-A. In the CD case study presented above, target tissue-A represents diseased intestinal mucosa. **Figures 19 to 22** describe detailed analyses of tissue-A in the CD case study, representing the inflamed mucosa (lamina propria, LP). These analyses focus on relative cell counts of various Treg subpopulations of particular migratory character, between diseased tissue of the inflammatory lesion itself and adjacent diseased tissue. With regard to targeting a particular tissue other than the lamina propria of the small bowel in the presented example, the target tissue could represent both solid tissues, interstitial fluids of solid tissue, oedemic or inflammatory fluids of diseased tissue regions, or tissues represented in a fluid phase, which include:
i) Epithelial mucosal surfaces for investigation of intra-epithelial cell populations as collected by mucosal scrapings or lavage sampling, or fractionation of biopsy/resection specimens,
ii)Sub-epithleial surfaces (e.g. lamia propria) as collected by biopsy or resection,
iii) Stroma of solid tissues as collected by biopsy or resection,
iv) Parenchyma of solid tissues as collected by biopsy or resection,
v) Endothelial and endothelial-vicinal tissues as collected by resection,
vi) Dermal layers as collected by cutaneous punch sampling, biopsy by incision or samples of tissues collected for grafting,
vii) Interstitial fluids of solid tissues collected by passive fluid collection methods,
viii) Synovial fluids of joint capsules or bursae as collected by active sampling methods,
iix) Cerebrospinal fluids as collected by active sampling methods,
ix) Oedemic or lymphedema fluids of solid tissues of bodily cavities collected by passive or active sampling methods (e.g. ascites of peritoneal cavity, or oedemic and lymphedemic fluids accumulating in solid tissues),
x) Nervous tissues as collected by biopsy or recovery from resected tissues or limb amputation,
xi) Skeletal muscle tissues as collected by biopsy or recovery from resected tissues or limb amputation.

The selection of tissue depends *inter alia* on the disease in question and on the tissue that is diseased.

**Figure 36** shows the framework of investigation relating to a disease state of interest, where diseased target tissue is represented as tissue-A. In the CD case study presented above, target tissue-A represents diseased intestinal mucosa. Tissue-B represents the lymph node(s) directly draining the Target tissue via collecting lymphatic vessels. **Figures 19 to 22** describe detailed analyses of tissue-B in the CD case study, representing the inflamed mesenteric lymph nodes (MLN/SLN). These analyses focus on relative cell counts of various Treg subpopulations of particular migratory character, between diseased-draining MLN (SLN) and non-disease-draining MLN. With regard to targeting a particular tissue other than the inflamed lamina propria of the small bowel in the presented case study, the target nodes would be those identified as draining the target tissue-A by commonly applied dye and radioisotope lymph node mapping methods, or by logical deduction based on anatomical features in regions with more limited lymph node architecture than in the mesentery. In addition to the target-B lymph nodes, microsurgical access of both collecting lymphatic vessels (target-B') and distal lymphatic vessels (target-B") may be used to assess cell populations emigrating from diseased tissue of interest (target-A). Thus target-B type tissues will include:
i) Identified disease draining (sentinel) lymph nodes as sampled by resection and processing, or active sampling by puncture and fluid draw.
ii) Collecting lymph fluids of the diseased tissue as collected by microsurgical access of collecting lymph vessel and installation of a cannula for passive fluid collection.
iii) Distal lymph fluids communicating from disease draining lymph nodes as collected by surgical installation of a cannula for passive fluid collection of minor distal lymphatic vessels, or active sampling of major distal lymphatic vessels where appropriate.

As mentioned herein, the method may include samples from a subject suffering from an inflammatory or autoimmune disease and/or from a healthy subject. In connection with tissue-B", it should be noted that option iii) of the method mentioned above may involve comparison made either with samples from a single subject suffering from an inflammatory or autoimmune disease or with samples from a subject suffering from an inflammatory or autoimmune disease and a healthy volunteer.

In the preferred aspect, specimens collected from target-B i) above are used, since lymph nodes represent the organising centres of local immune response, especially for non-mucosal tissues.

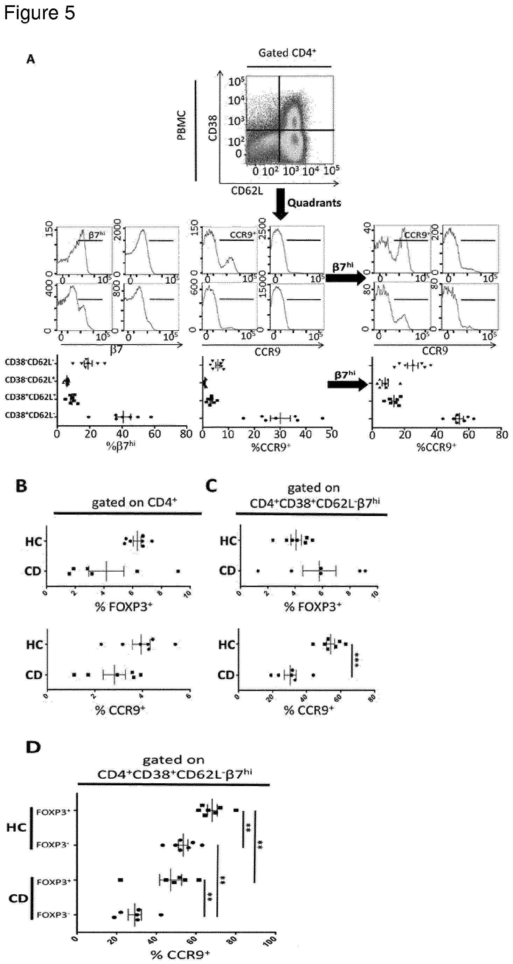

**Figure 36** shows the framework of investigation relating to a disease state of interest, where diseased target tissue is represented as tissue-A. In the CD case study presented above, target tissue-A represents diseased intestinal mucosa. Tissue-B represents the lymph node(s) directly draining the target tissue via collecting lymphatic vessels. **Figures 5 to 9** **and** **23 to 35** describe detailed analyses of migratory Treg subtypes in healthy individuals. This represents tissue-C, and will be common to analyses of any Target-A in any disease state.

It is anticipated that tissue-C is a parameter in the investigation of any target tissue, in addition to one or more of tissue -A, -B, -B' and -B".

**Figures 5 to** 9 summarises a comparative study of migratory Treg populations in peripheral circulation of patients with CD compared to healthy controls. Again this will be a common feature of any investigation of Target-A tissues, and one that is most readily achievable. A further aspect of the Target -A, -B and -C investigation that relates to the disease state of tissue target-A, is the comparison of diseased tissue with that of adjacent healthy tissue, and/or comparison with healthy tissue from non-diseased control subjects. The comparison and contrast of healthy and diseased tissue within diseased subjects, and between diseased and healthy subjects serves two purposes:
i) Identify the cell migratory and/or functional state in healthy tissue and natural communication axis of tissue-lymphatics-blood-tissue
ii) Compare and contrast the migratory and/or functional state in healthy tissue compared to tissue in diseased state.

Identification of quantitative or qualitative functional defect in migratory Treg populations, or defect in the migratory behaviour of Tregs cells themselves, related to the diseased tissue described in the CD cases study herein, is taken to confirm the diseased-tissue origin of the peripherally identified cells. However, such confirmation by functional defect is not considered necessary given sufficient correlative or associative evidence of diseased-tissue Treg origin. Such evidence could include, for example, direct identification of T-cell receptor clonotypes present in tissue target-A, and correlation to identical clonotypes in Treg subpopulations of suspected Target-A origin, as recovered from tissues B and C.

**Figure 36** presents, in Greek characters, T-cell migratory processes that are considered as key elements, with regard to primary criteria for identifying cells both locally and peripherally, within the investigational framework for identifying target-A-relevant Tregs. These parameters are drawn from the CD case study presented. The central aspects are considered as the emigration from target-A tissue, α, or the immigration to target-A tissue, β. A combination of α and β are considered emigrant populations with propensity to immigrate (recirculate) to target-A tissue of origin. Since the α and β, or α/β migratory processes are considered critical, it is of benefit to set criteria which distinguish these cells from the other major Treg migratory routes, γ and δ. Process γ represents cells entering lymph nodes (target-B) via high endothelial venules from peripheral blood. These are considered to contribute noise to the tissue-specific analyses of migratory processes α and/or β. Process δ represents emigration of de novo T-cells from the thymus into peripheral blood. These again contribute to analytical noise, and are irrelevant to identification of cells with α and β characteristics.

Based on these criteria the following analytical filters should generally be applied to analysis of Treg populations for identification of cells undergoing processes α and/or β:
i) Condition to exclude cells that gain access to lymph nodes via HEV, e.g. CD62L⁺ cells should be excluded (e.g. migratory process γ exclusion).
ii) Condition to exclude cells that are recent thymic emigrants, e.g. cells that are CCR9⁺CD45RA⁺ or CCR9⁺CCR7⁺ or CCD9⁺CD62L⁺ double positives should be excluded. Similarly, CCR9⁺CD45RO⁻ cells should be excluded. Any combination of these markers for the denoted +/- condition is considered relevant to the exclusion recent thymic emigrant de novo T-cells, and for the parallel exclusion of resting central memory cells that have not been recently activated against antigen (i.e. should carry the a CD45RA⁺/CD45RO⁻ character) (e.g. migratory process δ exclusion).

Thus the full analytical exclusion criteria are represented as CCR9⁺CCR7⁺CD62L⁺CD45RA⁺CD45RO⁻.
iii) Condition to include cells as Target-A emigrant and immigrant cells. This condition is defined as integrin-type or other adhesion molecules associated with Target-A tissue adhesion and transmigration through tissue-integral vasculature. For example, α4β7+ for cells with mucosal origin and tropism. This condition may also include chemoattractant receptors specific for the tissue region. For example, CCR9⁺ (CCL25 ligand) denotes activation of tight adhesion to vasculature of small bowel mucosal tissue. This condition may include markers that denote recent activation of emigrant population of interest, such as CD38⁺, CD69⁺ or CD44⁺ to identify cells as recent emigrants.

In establishment of criteria iii) above, the first step in the investigation may be to sample directly target-A tissue in question, and determine dominant expression of adhesion protein and chemoattractant receptors present on the observed local Tregs. Identified dominant migratory markers may then be applied to identification of emigrant cells in lymph and lymph nodes draining Target-A tissue, i.e. Target -B -B' and -B", and also emigrant/immigrant populations in target-C tissue (peripheral blood).

Target-A tissue-specific cells identified in the manner described above, within lymphatics or peripheral blood, are considered suitable starting material for the manufacture of cellular therapeutic compositions to treat inflammatory and autoimmune disorders of given Target-A tissue. In addition, cells derived from the diseased tissue itself, or adjacent healthy tissues of same type, are suitable as starting material. Regardless of the tissue sources, the purification of target cells on the basis of identified markers is relevant as to restrict starting material to cells most suitable for treatment of disease state. In the preferred aspect, starting material is purified from peripheral blood due to the relative ease of specimen collection.

A method of the invention may also be described as involving the following steps:
i) analysis of migratory factor expression by Tregs within target-A tissues,
ii) optional analysis of functional factor expression by Tregs cells within Target-A tissues,
iii) identification of cells of migratory (and optionally functional) element expression in target-C (peripheral blood),
iv) identification of cell of migratory (and optionally functional) element expression in target -B and/or -B' and/or -B" tissues (lymphatics),
v) identification of a migratory or functional defect in Treg population identified as expressing migratory (and optionally functional) elements specific for Target-A tissue in any of the above compartments (A, B or C), and
vi) identification of a migratory or functional defect any Leukocyte population being reasonably responsible for, or a result of, a migratory or functional defect in Treg population identified as expressing migratory (and optionally functional) elements specific for Target-A tissue in any of the above compartments (A, B or C).

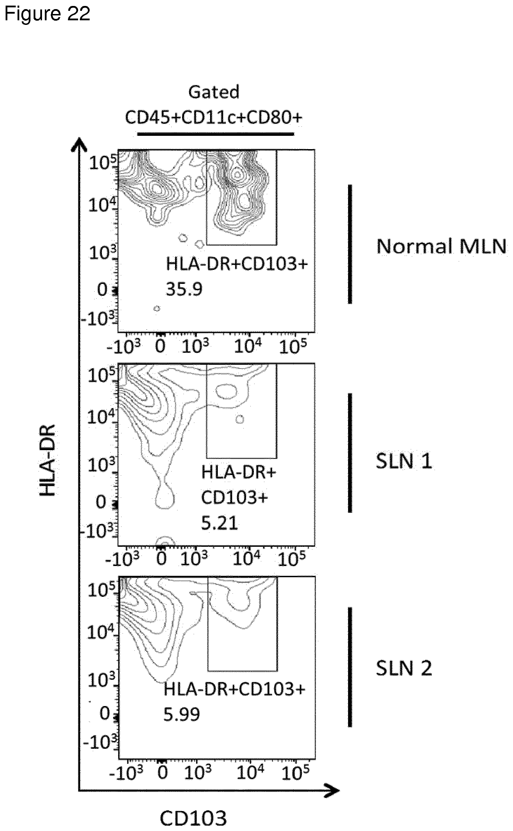

Within the above-described investigational framework, observations of disease-associated defects in cell migration and/or function are taken to confirm the diseased tissue (target-A) origin of the cell population in question. In the CD case study presented, the observable defect (CCR9) is one of migratory receptor imprinting on the Treg population of interest. However, any observable defect need not be related to migratory function, but could be distinct cellular and/or molecular dysfunction within the observed migratory population suspected to be of Target-A tissue origin. However, a lack of observable defect within the observed Treg population need not exclude an identified migratory population as being relevant starting material for manufacture of cellular therapeutic products. Indeed, disease-associated immunological dysfunction can arise from distinct dysfunction in non-Treg cells within the immunological response network. In the CD case study presented, this defect is proposed to represent a numerical defect in CD103⁺ dendritic cells (e.g. **Figure 22**). Considering these observations, it is contemplated that observable defects between healthy and diseased tissue of the patient, or between tissues of patients and healthy controls, can represent:
i) Migratory dysfunction in the Treg population of interest, originating from the Target-A tissue of interest
ii) Non-migratory dysfunction in the Treg population of interest, originating from the Target-A tissue of interest
iii) Migratory dysfunction in antigen-presenting cell populations reasonably associated (in location and/or function) with the Treg population of interest. This can include antigen-presenting dendritic cells, macrophage and non-professional APCs (including T-cells, fibroblasts, epithelial, endothelial cells etc) within Target-A tissue, or dendritic cells and B-cell in the associated Target-B (lymphatic) tissues, reasonably associated with guiding target-Treg activation.
iv) non-migratory dysfunction in antigen-presenting cell populations reasonably associated (in location and/or function) with the Treg population of interest. This can include antigen-presenting dendritic cells, B-cells, macrophage and non-professional APCs (including T-cells, fibroblasts, epithelial, endothelial cells etc) within Target-A tissue, or dendritic cells, B-cell and non-professional APCs (including T-cells, fibroblasts, epithelial, endothelial cells etc) in the associated Target-B (lymphatic) tissues, reasonably associated with guiding target-Treg activation.
v) Functional defects, or hyper-/hypo- activation, in immunological effector populations, particularly conventional T-cells, B-cells and plasma cells, that may be considered as a readout of a primary target-Treg numerical or functional insufficiency.

In the above-described investigational framework, all assayable parameters are cell-centric. That is to say that tissue compartment-specific assays are conducted on a single-cell basis, or on the basis of highly restricted purified cell populations. This is one of the defining features of the investigational framework, and distinguishes the approach from routine biomarker discovery workflows that rely on measurement of analyte abundance in the bulk extracts of target tissues and fluids. In classical workflows analysing non-restricted bulk extract of solid tissues and fluids, it is unclear whether a difference in analyte abundance is associated to a mechanistic defect that must logically arise from specific cellular and molecular defects, or rather simply corollary of a differing representation of cells present in the diseased tissue specimen when compared to a reference. In the later instance, the influx of cells to diseased tissue can be secondary to specific cellular and molecular dysfunctions, and this may simply be corollary of disease state, as opposed to rationally causative. The focus on single-cell, or highly restricted cell types and subtypes, allows rational elucidation of disease involvement of identified difference in disease state and reference. A majority of analyses in the presented CD case study utilise flow cytometric methods that interrogate each cell independently. Methods for single-cell or highly restricted cell population analyses may thus include:
i) Flow cytometric methods detecting surface antigen expression
ii) Flow cytometric methods detecting intracellular antigen expression
iii) Flow cytometric methods detecting transcript or genomic parameters by in situ probe hybridisation techniques
iv) Flow cytometric analyses of enzyme function or metabolite abundance
v) Flow cytometric purification of single cells for submission to downstream analytical workflows
vi) Flow cytometric purification of highly restricted cell populations and subpopulations for submission to downstream analytical workflows
vii) Substrate immunoaffinity enrichment of highly restricted cell populations and subpopulations for submission to downstream analytical workflows
viii) Substrate immunoaffinity enrichment of highly restricted cell populations and subpopulations, coupled with flow cytometric purification of single cells and/or highly restricted cell populations, for submission to downstream analytical workflows

In the aspects i), ii) and iii) above, with regard to direct flow cytometric analyses, assayed parameters on analyte cell populations identified by analytical inclusion/exclusion markers can include:
i) Abundance of cell surface expressed somatically invariant protein antigens (e.g. CD4, HLA haplotype),
ii) Abundance of surface expression of somatically rearranged protein antigens (e.g. T-cell receptor by tetramer staining, T-cell receptor assessment by profiling Vβ segment presentation, B-cell receptor by labelled antigen staining),
iii) Enzyme activity or metabolite abundance by fluorometric/colorimetric linked conversion assays (e.g. aldehyde dehydrogenase activity assay by cleavable substrate fluorescence detection),
iv) Abundance of intracellular expressed protein antigens, including activated versus inactivated signal protein abundance (e.g. FOXP3 abundance or unphosphorylated/phosphorylated mitogen activated protein kinase abundance),
v) Transcript abundance or genomic rearrangement detection by in situ probe hybridisation.

In a separate aspect of above-described downstream analyses, the method comprises further steps of purification and/or enrichment of cells. The above-mentioned single-cell assays v, vi, vii and viii are of relevance in this connection. Such analyses would include:
i) Protein abundance or post-translational modification by immune-blotting or other immune-detection methods or spectroscopic methods,
ii) Coding transcript abundance or presence by quantitative or qualitative PCR methods,
iii) Coding transcript abundance or presence sequencing or resequencing methods,
iv) Non-coding transcript abundance or presence (e.g. miRNAs and T-cell receptor excision circles) by PCR methods,
v) Analyses of somatic genomic rearrangements (e.g. T-cell receptors) and anomalous genomic rearrangement (e.g. aberrant malignant or pre-malignant genomic rearrangement) by PCR methods,
vi) Analyses of somatic genomic rearrangements (e.g. T-cell receptors) and anomalous genomic rearrangement (e.g. aberrant malignant or pre-malignant genomic rearrangement) by direct sequencing or resequencing methods,
vii) Analysis of protein (e.g. cytokine or immunoglobulin) or metabolite (e.g. lactic acid or retinoic acid) secretion,and/or
viii) Analysis of cellular function by mixed cell reactions (e.g. immunosuppression, specific cytotoxicity or antigen cross-presentation).

### Method for obtaining a CD4⁺ Treg cell population for use as starting material in cellular immunotherapy

Having identified a CD4⁺ Treg cell population with signatures suitable for use in cellular immunotherapy, a starting material can be obtained e.g. from the subject suffering from the inflammatory or autoimmune disease.

Accordingly and as mentioned herein before, the present invention also relates to a method for obtaining a CD4⁺ Treg cell population for use as starting material in cellular immunotherapy, the method comprising
i) subjecting peripheral blood from a patient suffering from an inflammatory or an autoimmune disease to single-cell analysis, whereby CD4⁺ Treg cells having the signatures identified in an identification method as described herein.

The method typically apply analytical filters to
i) exclude cells that gain access to lymph nodes via HEV, and
ii) exclude cells that are recent thymic emigrants.

The cells that gain access to lymph nodes via HEV may be CD62L+ cells; and recent thymic emigrants may be CCR9+CD45RA+, CCR9+CCR7+, CCD9+CD62L+, or CCR9+CD45RO- cells.

Thus, in the CD4⁺ Treg cells to be excluded are CCR9+CCR7+CD62L+CD45RA+CD45RO- cells.

The method may moreover include conditions for identifying CD4⁺ Treg cells that are emigrant and immigrant cells such as integrin-type or other adhesion molecules associated with Target-A tissue adhesion and transmigration through tissue-integral vasculature.

More specific a method of the invention is a method, wherein the CD4⁺ Treg cells obtained have specific signatures that
i) identify that the cells are CD4⁺ regulatory T-cells,
ii) identify that the regulatory T-cells are tissue type tropic, i.e. they can migrate to the diseased area,
iii) optionally, identify that the Treg cells are diseased tissue tropic, i.e. they are so-called homing cells that can localize in the diseased tissue,
iv) identify that the regulatory T-cells are emigrant cells, i.e. they originate from the target tissue, i.e. the diseased tissue (antigen-experienced cells), and
v) optionally, identify that the regulatory T-cells are retained in the target tissue after administration to a subject, and
optionally one or more X-signatures and/ or Y-signatures.

It is contemplated that migratory markers may have some overlap with related mucsoal surfaces of the alimentary canal, and there may be some overlap with distinct tissues. However, it is anticipated that additional and distinct migratory markers of type X are required to treat different regions of alimentary mucosa, and of distinct tissues.

One may also expect tissue-type and differing regions of the same tissue type, to possess a different functional marker representation, reflecting tissue specific activities. Thus, the Y condition in some instances should be anticipated as a condition that is selective of cells that are involved specifically in target tissue A in a similar context as the X condition.

Accordingly, the following specific markers identified as being of relevance in connection with CD may also be of relevance in connection with other types of inflammatory or autoimmune diseases such as those mentioned herein; however, and most likely, the markers for other types of tissue may be different, but can be identified by employing the identification method described herein.

The signature i) is typically CD4⁺CD25^{hi}CD127^{lo}.

The signature ii) for a gastrointestinal mucosa in the small bowel typically is α4β7⁺ or α4⁺β7⁺.

The signature for iii) for localization in the small bowel typically CCR9⁺,

The signature for antigen-experienced cells from the small bowel, iv) typically is CD62L⁻CD38⁺,

As demonstrated in the examples herein and in particular with reference to CD8⁺ Treg cells for use in the treatment of CD in the small bowel, an X-signature is selected from
a) CD26, CD97, CD143, CD195, CD278,
b) CD61, CD63, CD146, CD183, CD197, CD200, CD244,
c) CD20, CD130, CD166,

A Y-signature is typically selected from
d) CD21, CD35, CD73, CD122, CLIP, CD120b,
e) CD6, CD39, CD50, CD109, CD226, CD243, CD268, CD274, CD210,
f) CD49c, CD53, CD84, CD95, CD107a.

The CD4⁺ Treg cells may also contain the signatures CD38+, CD69+ and/or CD44+ to denote recent activation.

In a separate aspect, the invention relates to a composition for cellular immunotherapy, the composition comprising an isolated CD4⁺ Treg cell population identified and/or obtainable as described herein.

Treg cells may be dispersed in a suitable medium before administration to the patient. A suitable medium may be an aqueous medium e.g. containing substances that ensures viability of the cells. It may also contain osmotically active substances, pH regulating substances or other physiologically acceptable substances. To this end, the present invention also relates to a pharmaceutical composition comprising the Treg cells specified herein together with an aqueous medium. The pH and osmotic pressure of the composition are adjusted to physiologically acceptable values, i.e. pH in a range of from 3 to 8 including 7.4, and the osmotic pressure in a range of from 250 - 350 mOsm/l including 285-300 mOsm/l. A specific example of a suitable medium is a 0.9% w/w sodium chloride solution comprising up to 3% w/w human serum albumin such as up to 2% w/w serum albumin or up to 1% w/w serum albumin. Another suitable medium is an aqueous medium comprising albumin such as 2% w/w albumin. They may also be suspended in saline-based solutions of physiological pH, and with appropriate biological and non-biological additive to promote cell survival and stability.

The Treg cells may also be admixed with a blood sample preferably from the patient's own blood or at least from blood compatible with the patient's own blood.

The Treg cells are normally administered parenterally to the patient such as intraveneous, intraarterial, intrathecal or intraperitoneal administration.

The number of cells to be administered depends on the disease and the severity of the disease to be treated, as well as the weight and age of the patient. It is contemplated that the number of cells is in a range of from 1x10⁵ to about 10 x 10⁹.

The Treg cells are administered by the parenteral route, preferably via injection into the circulatory system.

### Examples on specific inflammatory or autoimmune diseases

Below are given examples of inflammatory or autoimmune diseases affecting specific tissues. It is not generally speculated what the underlying source of antigen specificity with regard to adaptive T-cell response within an inflamed tissue. Indeed, it should be noted that inflammatory and autoimmune disorders are seldom characterised with regard to T-cell antigen specificity. Therefore, it is difficult to assert whether a particular inflammatory condition is driven solely by foreign-antigens, self-antigens, or a combination of foreign- and self- antigens. Indeed, many rare 'orphan' diseases that have inflammatory and autoimmune aspects, affecting mucosal surfaces, skin, visceral organs and other bodily tissues, are poorly characterised with regard to immunological involvement. It is thus anticipated that, while such rare diseases affecting specific tissues may be treated with tissue targeted Treg therapies, the low (and geographically diffuse) availability of study subjects means that there is little chance of obtaining sufficient systematic data to identify suitable Treg cell populations by direct investigation of disease state. In such an event, severe infections or allergic reactions localised to a tissue of interest (tissue A), and/or distinct inflammatory states affecting that tissue, represents a suitable substitute with which to identify cells with migratory characteristics that are specific for the affected tissue-A. This is based on the premise that factors that affect cell homing to diseased tissues remain defined as tissue-centric, not disease-centric per se. For example, it is well known that small bowel inflammation results in up-regulation of CCL25 chemoattractant, boosting CCR9-dependnet immigration of lymphocytes to that inflamed tissue. In this instance, the disease state provokes an increase in specific chemoattraction that is tissue-centric. It is considered that infectious and allergic inflammation that is localised to tissue type, and/or anatomical location of that tissue, may be used to identify Tregs suitable for treatment of autoimmune/idiopathic inflammatory conditions afflicting that tissue type and/or anatomical location - particularly in applications of rare orphan diseases.

The methods described in the paragraphs above can be used to identify suitable CD4⁺ Treg cell populations for use in cellular immunotherapy for treatment of the diseases. The method steps are not repeated below, but the general methods described above and in the claims are applicable. Thus, the method for identifying a CD4⁺ Treg cell population for use in therapy as well as the method for obtaining a CD4⁺ Treg cell population for use as a starting composition in therapy described herein apply mutatis mutandis for all inflammatory/autoimmune diseases, notably for those mentioned herein.

In the method for obtaining CD4⁺ Treg cells for use in accordance with the invention, the method should include analytical filters to:
i) exclude cells that gain access to lymph nodes via HEV, e.g. CD62L+ cells should be excluded,
ii) exclude cells that are recent thymic emigrants, e.g. cells that are CCR9+CD45RA+ or CCR9+CCR7+ or CCD9+CD62L+ double positives should be excluded. Similarly, CCR9+CD45RO- cells should be excluded. Any combination of these markers for the denoted +/- condition is considered relevant to the exclusion recent thymic emigrant de novo T-cells, and for the parallel exclusion of resting central memory cells that have not been recently activated against antigen (i.e. should carry the a CD45RA+/CD45RO-character). Thus the full analytical exclusion criteria are represented as CCR9⁺CCR7⁺CD62L⁺CD45RA⁺CD45RO⁻,
iii) include cells as emigrant and immigrant cells. This condition is defined as integrin-type or other adhesion molecules associated with tissue adhesion and transmigration through tissue-integral vasculature.

### Tregs for Inflammatory Disorders of Major Alimentary Mucosa

The present invention provides a means to identify specific CD4⁺ Treg cells for use in the treatment of inflammatory disorders of the alimentary mucosa other than CD, for example, for ulcerative colitis. In this example, where UC affects primarily the colorectal mucosa rather than the mucosa of the small intestine as in CD, the Treg cells should have specific signatures that:
i) identify that the cells are regulatory T-cells,
ii) identify that the regulatory T-cells are tissue type tropic, in this case colorectal mucosal tropic, i.e. they can migrate to the diseased area (colorectal mucosa),
iii) optionally, identify that the Treg cells are diseased tissue tropic, in this case colon and/or rectum tropic, i.e. they are so-called homing cells that can localize in the diseased tissue eg colon or rectum,
iv) identify that the regulatory T-cells are emigrant cells, i.e. they originate from the target tissue area (educated cells), and v) optionally, identify that the regulatory T-cells are retained in the target tissue after administration to a subject.

It is anticipated that different regions of the colorectal mucosa will have distinct migratory cues. In that, sampling of different regions of the mucosa and lymphatics draining these differing regions may yield region-specific migratory T-cells. These main regions may be divided into the following:
i) Ascending colon and segments thereof.
ii) Transverse colon and segments thereof.
iii) Descending colon and segments thereof.
iv) Sigmoid colon and segments thereof.
v) Rectum
vi) Anal canal

These mucosal tissues may be sampled by biopsy or surgical resection under routine clinical management of patients with UC or other inflammatory colitis condition.

Lymph nodes sampled to drain these colorectal regions are located in the mesentery close to the colon and named paracolic lymph nodes or in the mesentery at random places and also associated with the major colonic vessels (arteries and veins). These nodes are termed mesenteric lymph nodes and they finally drain to the thoracic duct which empties into the left subclavian vein close to the right atrium. Lymph nodes draining rectum are located in the rectal mesentery and along the superior rectal vessels. The lower part of the anal canal is drained through skin lymphatics and the first draining lymph node(s) is in the right or left groin. Draining lymph nodes should be identified by injection of healthy tissue or disease lesions with suitable tracer compound, and tracer-based identification of primary draining nodes. Cells can be harvested from the lymph nodes through surgical biopsy or needle biopsy.

### Tregs for Inflammatory Disorders of Pulmonary Mucosa

The present invention provides a means to identify specific CD4⁺ Treg cells for use in the treatment of inflammatory disorders of the pulmonary mucosa other than CD, for example, chronic obstructive pulmonary disease (COPD). In this example, COPD affects mainly pulmonary mucosa located in the bronchial tree, the Treg cells should have specific signatures that:
i) identify that the cells are regulatory T-cells,
ii) identify that the regulatory T-cells are tissue type tropic, in this case bronchial mucosal tropic, i.e. they can migrate to the diseased tissue (bronchial mucosa),
iii) optionally, identify that the Treg cells are diseased tissue tropic, in this case bronchial tropic, i.e. they are so-called homing cells that can localize in the diseased tissue eg bronchial mucosal tree,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling mucosal tissues would occur via bronchoscopic biopsy, bronchoalveolar lavage, or sampling of diseased and normal resected tissues in the event of surgical procedures, such as pulmonary resections due to lung lesions or emphysema.

Lymph nodes sampled to drain this pulmonary region will include paratracheal, inferior tracheobronchial and bronchopulmonary nodes. Draining lymph nodes should be identified by injection of healthy tissue or disease lesions with suitable tracer compound, and tracer-based identification of primary draining nodes,

### Tregs for Inflammatory Disorders of Vaginal and Uterine Mucosa

The present invention provides a means to identify specific CD4⁺ Treg cells for use in the treatment of inflammatory disorders of the vaginal and uterine tissues, particularly mucosal surfaces, for example, vaginitis. In this example, vaginitis affects vaginal mucosa, the Treg cells should have specific signatures that:
i) identify that the cells are regulatory T-cells,
ii) identify that the regulatory T-cells are tissue type tropic, in this case vaginal mucosal tropic, i.e. they can migrate to the diseased tissue (mucosa),
iii) optionally, identify that the Treg cells are diseased tissue tropic, in this case lung tropic, i.e. they are so-called homing cells that can localize in the diseased tissue eg vagina,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling mucosal tissues would occur via mucosal scrapings or biopsy.

Lymph nodes sampled to drain this vaginal region will include the external iliac nodes (upper third of the vagina), the common and internal iliac nodes (middle third), and the superficial inguinal and perirectal nodes (lower third). Draining lymph nodes should be identified by injection of healthy tissue or disease lesions with suitable tracer compound, and tracer-based identification of primary draining nodes

In a related aspect, the present invention provides a means to identify specific CD4⁺ Treg cells for use in the treatment of inflammatory disorders of the uterine and cervical tissues, particularly mucosal surfaces, for example, cervicitis and endometriosis. In these examples, where disease affects cervical and uterine tissues, the Treg cells should have specific signatures that:
i) identify that the cells are regulatory T-cells,
ii) identify that the regulatory T-cells are tissue type tropic, in this case cervical or uterine mucosal tropic, i.e. they can migrate to the diseased tissue (mucosa),
iii) optionally, identify that the Treg cells are diseased tissue tropic, in this case cervical or uterine tropic, i.e. they are so-called homing cells that can localize in the diseased tissue eg vagina,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling mucosal tissues would occur via mucosal scrapings or biopsy. In the case of uterine tissues, collection of menstrual material would be of benefit. Collection of placental tissues may also be of value in these investigations, and investigation of foetal tolerance or immune-rejection in related aspects in this tissue region (e.g. Rh disease and pre-eclampsia).

Lymph nodes sampled to drain these cervical and uterine tissues will include aortic and lateral groups, superficial inguinal, external iliac, obturator, paracervical, internal iliac and sacral nodes. Draining lymph nodes should be identified by injection of healthy tissue or disease lesions with suitable tracer compound, and tracer-based identification of primary draining nodes.

### Tregs for Inflammatory Disorders of Oral and Pharyngeal Mucosa

The present invention provides a means to identify specific CD4⁺ Treg cells for use in the treatment of inflammatory disorders of the oral and pharyngeal tissues, particularly mucosal surfaces, for example; various stomatitis conditions affecting mucosa of the oral cavity, laryngitis affecting laryngopharynx and pharyngitis affecting oropharynx tissues. These disorders of the oral and pharyngeal tissues are grouped due to their interrelated nature within and highly immunoactive proximal mucosa. In this example, inflammatory diseases that affect the proximal mucosa (inferior to nasal and sinus cabaties), the Treg cells should have specific signatures that:
i) identify that the cells are regulatory T-cells,
ii) identify that the regulatory T-cells are tissue type tropic, in this case proximal mucosal tropic, i.e. they can migrate to the diseased tissue (mucosa),
iii) optionally, identify that the Treg cells are diseased tissue tropic, in this case proximal mucosa tropic, i.e. they are so-called homing cells that can localize in the diseased tissue eg oral and pharyngeal mucosa,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling mucosal tissues would occur via mucosal scrapings or biopsy.

Lymph nodes sampled to drain these regions will include submental, submandibular, preauricular, parotoid, subdigastric, buccal and retropharyngeal nodes. Draining lymph nodes should be identified by injection of healthy tissue or disease lesions with suitable tracer compound, and tracer-based identification of primary draining nodes.

Notable in the frame of proximal mucosa is the addition of major mucosal associated lymphoid tissues (MALT), represented by the tonsils. In this instance, biopsy or preparation of resected tissues of Waldeyers tonsillar ring (adenoid, tubal, palatine and lingual tonsils) should be included in the investigational framework cell migratory axis. These can be considered as the centre of primary immune reaction, and recirculation to the disease-affected mucosa, or as secondary activating sites of cells migrating (via peripheral blood), to these regions. This tonsillar tissue can be considered as communicating tissue-X within the model presented in Figure 36. Communication of cells between disease-affected tissue-A and communicating tissue-X, this could occur through αX and βX migratory processes. It may also reasonably occur via local migratory processes, ε. αX/βX and ε communication processes are reasonably anticipated to be manifestations of different migratory protein expression on Tregs on interest. Tonsillar tissue may reasonably be included in the investigation on the simple basis of enlargement, as to indicate an inflammatory status within the disease-affected region, or my molecular and cellular pathological indicators.

### Tregs for Inflammatory Disorders of Lingual Tissue

The present invention provides a means to identify specific CD4⁺ Treg cells for use in the treatment of inflammatory disorders of the lingual tissues, for example, various forms of glossitis. This is considered as district from oral and pharyngeal tissues of the proximal mucosa due to the unique tissue form, and distinct lymphatic drainage. In this example of glossitis, the Treg cells should have specific signatures that:
i) identify that the cells are regulatory T-cells,
ii) identify that the regulatory T-cells are tissue type tropic, in this case lingual tropic, i.e. they can migrate to the diseased tissue (tongue),
iii) optionally, identify that the Treg cells are diseased tissue tropic, in this case lingual tropic, i.e. they are so-called homing cells that can localize in the diseased tissue eg tongue
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of lingual tissues would occur via biopsy.

Lymph nodes sampled to drain this region include submental, submandibular, inferior deep cervical, superior deep cervical. Draining lymph nodes should be identified by injection of healthy tissue or disease lesions with suitable tracer compound, and tracer-based identification of primary draining nodes.

Notable in the frame of lingual tissue is the addition of major mucosal associated lymphoid tissues (MALT), represented by the tonsils. In this instance, biopsy or preparation of resected tissues of Waldeyers tonsillar ring (adenoid, tubal, palatine and lingual tonsils) should be included in the investigational framework cell migratory axis centered on lingual tissue. These can be considered as the centre of primary immune reaction, and recirculation to the disease-affected mucosa, or as secondary activating sites of cells migrating (via peripheral blood), to these regions. This tonsillar tissue can be considered as communicating tissue-X within the model presented in Figure 36. Communication of cells between disease-affected tissue-A and communicating tissue-X, this could occur through αX and βX migratory processes. It may also reasonably occur via local migratory processes, ε. αX/βX and ε communication processes are reasonably anticipated to be manifestations of different migratory protein expression on Tregs on interest. Tonsillar tissue may reasonably be included in the investigation on the simple basis of enlargement, as to indicate an inflammatory status within the disease-affected region, or molecular and cellular pathological indicators. In is anticipated that the lingual tonsils are the most relevant to glossitis and other inflammatory disorders of lingual tissues.

### Tregs for Inflammatory Disorders of Nasal and Sinus Mucosa

The present invention provides a means to identify specific CD4⁺ Treg cells for use in the treatment of inflammatory disorders of the nasal and sinus mucosa, for example, various forms rhinitis and sinusitis. This is considered as district from oral and pharyngeal tissues of the proximal mucosa due to lymphatic drainage and associated MALT. In these examples of rhinitis and sinusitis, the Treg cells should have specific signatures that:
i) identify that the cells are regulatory T-cells,
ii) identify that the regulatory T-cells are tissue type tropic, in this case nasal or sinus mucosal tropic, i.e. they can migrate to the diseased tissue (nasal or sinus mucosa),
iii) optionally, identify that the Treg cells are diseased tissue tropic, in this case lingual tropic, i.e. they are so-called homing cells that can localize in the diseased tissue eg nasal and sinus mucosa,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of nasal or sinus tissues would occur via mucosal scrapings or biopsy.

Lymph nodes sampled to drain this region include retropharyngeal, paratoid, buccal submental and submandibular nodes. Draining lymph nodes should be identified by injection of healthy tissue or disease lesions with suitable tracer compound, and tracer-based identification of primary draining nodes.

Notable in the frame of nasal and sinus tissue is the addition of major mucosal associated lymphoid tissues (MALT), represented by the tonsils. In this instance, biopsy or preparation of resected tissues of Waldeyers tonsillar ring (adenoid, tubal, palatine and lingual tonsils) should be included in the investigational framework cell migratory axis centred on nasal and sinus tissues. These can be considered as the centre of primary immune reaction, and recirculation to the disease-affected mucosa, or as secondary activating sites of cells migrating (via peripheral blood), to these regions. This tonsillar tissue can be considered as communicating tissue-X within the model presented in Figure 36. Communication of cells between disease-affected tissue-A and communicating tissue-X, this could occur through αX and βX migratory processes. It may also reasonably occur via local migratory processes, ε. αX/βX and ε communication processes are reasonably anticipated to be manifestations of different migratory protein expression on Tregs on interest. Tonsillar tissue may reasonably be included in the investigation on the simple basis of enlargement, as to indicate an inflammatory status within the disease-affected region, or my molecular and cellular pathological indicators. In is anticipated that the adenoid tonsils are the most relevant to rhinitis and sinusitis, and other inflammatory disorders of nasal and sinus tissues.

### Tregs for Inflammatory Disorders of Minor Mucosal Surfaces

The present invention provides a means to identify specific CD4⁺ Treg cells for use in the treatment of inflammatory disorders of minor mucosal tissues. In this example, the Treg cells should have specific signatures that:
i) identify that the cells are regulatory T-cells,
ii) identify that the regulatory T-cells are tissue type tropic, in this case tropic for minor mucosa under consideration, i.e. they can migrate to the diseased tissue (minor mucosal surface),
iii) optionally, identify that the Treg cells are diseased tissue tropic, in this case tropic for minor mucosa under consideration, i.e. they are so-called homing cells that can localize in the diseased tissue eg minor mucosa under consideration, and optionally specific signatures that
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of minor mucosal tissues would occur via mucosal scrapings or biopsy. Lymph nodes sampled to drain the mucosal regions under investigation should be identified by injection of mucosal surface with suitable tracer compound, and identification of primary draining nodes.

Notable in the frame of minor mucosal tissues like tissue is the addition of major mucosal associated lymphoid tissues (MALT), represented by the tonsils. In this instance, biopsy or preparation of resected tissues of Waldeyers tonsillar ring (adenoid, tubal, palatine and lingual tonsils) should be included in the investigational framework cell migratory axis centered on lingual tissue. These can be considered as the centre of primary immune reaction, and recirculation to the disease-affected mucosa, or as secondary activating sites of cells migrating (via peripheral blood), to these regions. This tonsillar tissue can be considered as communicating tissue-X within the model presented in **Figure 36**. Communication of cells between disease-affected tissue-A and communicating tissue-X, this could occur through αX and βX migratory processes. It may also reasonably occur via local migratory processes, ε. αX/βX and ε communication processes are reasonably anticipated to be manifestations of different migratory protein expression on Tregs on interest. Tonsillar tissue may reasonably be included in the investigation on the simple basis of enlargement, as to indicate an inflammatory status within the disease-affected region, or my molecular and cellular pathological indicators.

### Tregs for Inflammatory Disorders of the Liver

The present invention provides a means to identify specific CD4⁺ Treg cells for use in the treatment of inflammatory disorders of the liver tissues including the biliary tree, for example, primary sclerosing cholangitis (PSC), primary biliary cirrhosis (PBC) and autoimmune hepatitis (AH). In the examples of PSC and PBC, the Treg cells should have specific signatures that:
i) identify that the cells are regulatory T-cells,
ii) identify that the regulatory T-cells are tissue type tropic, in this case liver and/or biliary tree tropic, i.e. they can migrate to the diseased tissue (liver and biliary tree),
iii) optionally, identify that the Treg cells are diseased tissue tropic, in this case liver and biliary tree tropic, i.e. they are so-called homing cells that can localize in the diseased tissue eg liver and biliary tree,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of biliary tissues would occur by endoscopic brushings (swabs) or biopsy from bile ducts, or processing of resected tissues from surgical procedures related to bile bladder, bile ducts or liver.

Notable in the frame of liver and biliary tree tissue is the apparent close communication between these tissues, where cells of the biliary mucosa may infiltrate vicinal liver tissue. That is, representing local migratory communication, ε, in the model present in **Figure 36**. One could also reasonably expect communication of cells between disease-affected tissue-A and communicating tissue-X, to occur via αX and βX migratory processes in the case of disorders of the biliary tree and liver; whether the primary disease is considered to be related to the liver or biliary tree.

First draining lymph nodes can be identified both from different parts of the biliary tree and various parts of the liver. The identification is done through injection of tracer (dye and/or isotope) in either the diseased tissue or in normal tissues (for comparative purposes). The draining node(s) from the biliary tree is located along the hepatoduodenal ligament. The draining node(s) from the right lobe is usually located at the midpart or distal part of the hepatoduodenal ligament. The draining node(s) from the left liver lobe is often located proximally in the same ligament.

### Tregs for Inflammatory Disorders of Pancreas

The present invention provides a means to identify specific CD4⁺ Treg cells for use in the treatment of inflammatory disorders of pancreatic tissues, for example, Type-1 diabetes and autoimmune pancreatitis. In these examples of Type-1 diabetes and autoimmune pancreatitis, the Treg cells should have specific signatures that:
i) identify that the cells are regulatory T-cells,
ii) identify that the regulatory T-cells are tissue type tropic, in this case pancreas tropic, i.e. they can migrate to the diseased tissue (pancreas),
iii) optionally, identify that the Treg cells are diseased tissue tropic, in this case pancreas tropic, i.e. they are so-called homing cells that can localize in the diseased tissue eg pancreas
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of biliary tissues would occur via biopsy, or processing of resected tissues during clinical management of patients. Pancreatic tissues may also be collected during pancreatic transplant surgeries.

Lymph nodes sampled to drain these tissues include in most cases lymph nodes located close to the pancreas (parapancreatic nodes) or nodes located along the proximal part of the hepatoduodenal ligament. Lymph nodes sampled to pancreatic tissues should be identified by injection of disease lesions or healthy tissues with suitable tracer compound, and identification of primary draining nodes.

### Tregs for Inflammatory Disorders of the Skin

The present invention provides a means to identify specific CD4⁺ Treg cells for use in the treatment of inflammatory disorders of the skin, for example psoriasis, skin for of SLE, acute and chronic dermatitis including various eczemas and dermatitis herpetiformis, erythema nodosum and pyoderma gangrenosum, In this example of inflammatory skin disorders, the Treg cells should have specific signatures that:
i) identify that the cells are regulatory T-cells,
ii) identify that the regulatory T-cells are tissue type tropic, in this case skin tropic, i.e. they can migrate to the diseased tissue (skin),
iii) optionally, identify that the Treg cells are diseased tissue tropic, in this case skin tropic, i.e. they are so-called homing cells that can localize in the diseased tissue eg skin,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of skin tissues would occur via cutaneous punch sampling, biopsy by incision or samples of tissues collected for grafting. It anticipated that different regions of the skin would have distinct migratory patterns. This should be taken into account when sampling localised or regional inflammation of the skin, and matched regions of healthy tissue should be considered as appropriate reference.

Sampling of skin-draining lymph nodes is common in management of melanoma patients, for example. This requires, as in all cases discussed herein, an accurate map of the pattern of lymphatic drainage from the primary site of disease. Lymphoscintigraphic is routinely used, however, it is clear that patterns of lymphatic drainage from the skin are not clinically predictable. It is common to observe unexpected lymphatic drainage from the skin of the back to nodes in the triangular intermuscular space, and in the paraaortic, paravertebral, and retroperitoneal areas. Drainage can also be observed from the base of the neck up to nodes in the occipital or upper cervical areas or from the scalp down to nodes at the neck base, bypassing many node groups, for instance. Upper limb drainage can be to nodes superior to the axilla. Lymphatic drainage may involve nodes in multiple groupings, and drainage across the midline of the body is common. Considering the extensive clinical experience in management of melanoma by identification and biopsy of skin-draining lymph nodes, the sampling of disease-associated lymph nodes in inflammatory disorders of the skin is considered achievable. However, considering the skin is the second most immunoactive surface after the mucosa, existing knowledge may be applied for the application of skin-specific analytical filters for direct identification of cells in peripheral blood specimens.

In addition, the ready access and relatively non-invasive nature of skin biopsy makes direct skin (tissue-A) sampling to be first preferred to collection of lymphatic (tissues - B, -B', -B") in most inflammatory disease conditions of the skin.

### Tregs for Inflammatory Disorders of the Central Nervous System

The present invention provides a means to identify specific CD4⁺ Treg cells for use in the treatment of inflammatory disorders of the central nervous system, for example, multiple sclerosis (MS), amyotropic lateral sclerosis (ALS) or sterile inflammation arising from radiotherapy protocols. In these examples of CNS inflammation, the Treg cells should have specific signatures that:
i) identify that the cells are regulatory T-cells,
ii) identify that the regulatory T-cells are tissue type tropic, in this case CNS tropic, i.e. they can migrate to the diseased tissue (CNS, white and/or gray tissue),
iii) optionally, identify that the Treg cells are diseased tissue tropic, in this case CNS tropic, i.e. they are so-called homing cells that can localize in the diseased tissue eg CNS,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of CNS tissues would occur exclusively via sampling of CNS fluids e.g. cerebrospinal and interstitial fluid collected from brain or spinal cord. It is contemplated that different ventricular and sinuses of the brain or the spinal cord contain distinct forms of Treg migratory cells, and thus may be informatively sampled separately in certain instances.

The caveat in sampling CNS T-cell emigration is in that the CNS possesses no conventional lymphatic drainage. Moreover, mechanisms of T-cell immigration into CNS across the blood-brain barrier remain unclear. It is contemplated that the primary interrogation of homing receptor patterns of CNS-integral Tregs collected via cerebrospinal fluids will enable identification of CNS-specific Tregs in peripheral blood compartment. That is, the target tissue-A is represented as the cerebrospinal fluid phase. Lymphatic drainage (tissue -B, -B', -B") is not considered directly relevant to identification of CNS-specific cells.

Lymphatic drainage of the CNS is sometimes referred to as 'glymphatic' drainage. It is thought that subarachnoid CSF enters the brain rapidly, along the paravascular spaces surrounding penetrating arteries exchanging with the surrounding interstitial fluid. Interstitial fluid is cleared from the brain parenchyma via the paravascular spaces surrounding large draining veins. Paravascular spaces are CSF-filled channels formed between the brain blood vessels and the leptomeningeal sheathes surrounding cerebral surface vessels, and proximal penetrating vessels. It is considered that para-arterial ininflux and para-venous efflux represents the general course of T-cell immigration and emigration to and from the brain parenchyma, respectively. Integrin αLβ2 and α4β1 have been implicated in adhesion and transmigration of leukocytes into the CNS via post capillary venules. It is unclear whether migratory imprinting of these and other specific CNS-tropic markers occurs within CNS tissues, as occurs in extra-CNS tissues. Direct sampling of cerebrospinal and interstitial fluids of the brain is anticipated to address these issues, and allow subsequent identification of peripheral CNS-tropic Tregs in the periphery.

### Tregs for Inflammatory Disorders of Peripheral Innervation

The present invention provides a means to identify specific CD4⁺ Treg cells for use in the treatment of inflammatory disorders of the peripheral nervous system (PNS), for example, Guillain-Barre syndrome and chronic inflammatory demyelinating polyneuropathy. In these examples of PNS inflammation, the Treg cells should have specific signatures that:
i) identify that the cells are regulatory T-cells,
ii) identify that the regulatory T-cells are tissue type tropic, in this case PNS tropic, i.e. they can migrate to the diseased tissue (PNS),
iii) optionally, identify that the Treg cells are diseased tissue tropic, in this case PNS tropic, i.e. they are so-called homing cells that can localize in the diseased tissue eg PNS,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of PNS tissues would occur via biopsy of primarily affected nerves and normal nerves with minor sensoric functions, for example inter-costobrachial nerves that runs through the axilla and usually are cut in normal surgical procedures such as axillary dissection. Dependent on the region of pathology and sampling, draining lymphatics and lymph nodes could reasonably be identified. For example, in pathologies and biopsy of distal regions of the sural nerve, direct access to the collection lymphatics of the lower leg may be mapped and sampled. Regional lymph nodes draining the region should be identified by injection of disease lesions or healthy tissues with suitable tracer compound, and identification of primary draining nodes.

It is anticipated that the expression of migratory markers for local or regional peripheral nervous pathologies will be associated with the tissue region in general, rather than necessarily displaying tropism restricted to the nervous tissue itself.

### Tregs for Inflammatory Disorders of Joint Capsule, Joint Surfaces and Bursae

The present invention provides a means to identify specific CD4⁺ Treg cells for use in the treatment of inflammatory disorders of joint-associated tissues (JAT) including the joint capsule, cartilaginous and bone surfaces and bursae, for example, rheumatoid arthritis, osteoarthritis and bursitis. In these examples JAT inflammation, the Treg cells should have specific signatures that:
i) identify that the cells are regulatory T-cells,
ii) identify that the regulatory T-cells are tissue type tropic, in this case JAT tropic, i.e. they can migrate to the diseased tissue (JAT),
iii) optionally, identify that the Treg cells are diseased tissue tropic, in this case JAT tropic, i.e. they are so-called homing cells that can localize in the diseased tissue eg JAT,
iv) identify that the regulatory T-cells are emigrant cells, i.e. they originate from the target tissue area (educated cells), and
v) optionally, identify that the regulatory T-cells are retained in the target tissue after administration to a subject.

It is contemplated that sampling of JAT tissues would occur via biopsy or collection of tissues resected during surgical management of disease. In addition, it is anticipated that collection of synovial fluids from joint capsules and bursae may be sufficient to identify local Treg populations. Particularly collection of inflammatory tissues and joint capsule during orthopaedic prosthesis operations will be useful.

Joint diseases generally affect major limb joins including hands, wrists, ankles, knees, hips, elbows and shoulders. Thus lymph nodes draining diseased tissues are to be the major nodal groups of the legs and arms, and expected to terminate at inguinal and axillary nodes, respectively. First draining lymph nodes can also be mapped and biopsied as described above.

Notable in the frame of joint capsule and bursae, the tissue is in apparent close communication between these tissues. Indeed, bursitis is often associated with rheumatoid arthritis of the underlying joint capsule. That is, communication may represent local migratory communication , ε, in the model present in **Figure 36****.** One could also reasonably expect communication of cells between disease-affected tissue-A and communicating tissue-X, to occur via αX and βX migratory processes in the case of rheumatoid arthritis-associated bursitis and osteoarthritis-associated bursitis, for example.

It is anticipated that the symmetry of affected joints observed in many rheumatoid arthritis cases is reflective of distinct migratory axes for the differing joint fields e.g. Tregs migrate differently to finger joints than they do to elbow joints.

### Aspects of Tissues vicinal and/or communicating with Diseased Tissue

Most inflammatory disorders are tissue-specific. This can arise as a result of adaptive immune responses being driven against self-antigens relatively restricted to the affected tissue, or against foreign antigens of relatively high availability in the affected tissue. It is common for secondary inflammatory disorders, or specific autoimmunity, to be associated with a primary inflammatory disease. For example, primary sclerosing cholangitis and ankylosing spondylitis are often associated with primary UC; dermatitis herpetiformis is often associated with primary acute celiac disease; bursitis often associated with underlying rheumatoid arthritis. In another aspect, autoimmune syndromes, such as demyelination of peripheral nerves (e.g. chronic inflammatory demyelinating polyneuropathy) are often associated with prior viral or microbiological infection. In the instances where such associated diseases run in parallel, it is simple to allude to a generalized immunological dysfunction that permits establishment of distinct diseases at multiple locations, and that association is driven by the common underlying immunological defect. It is contemplated that dissemination of dysfunctional immune cells, including T-cells, from the primary disease location/region, permits establishment of distinct diseases within distinct tissue types at unrelated tissue locations in the body.

In the above conception, it is the simplest to assume that T-cell associated pathologies at distinct tissue locations are driven by common antigenic support of the pathogenic T-cell response. Indeed, this may be the case for primary sclerosing cholangitis associated with a primary IBD pathology, for example. Wherein, both affected tissues are mucosal, of a continuous region, and known to share similar and overlapping T-cell migratory cues. It is also reasonable to assume that antigens would be, to a degree, commonly available to these mucosal tissues of continuous nature. In contrast, considering associated inflammatory disorders of distinct tissue types (e.g. celiac disease of the small bowel mucosa and dermatitis herpetiformis) it is difficult to expect shared patterns of T-cell migration and antigen availability. In both instances, however, one can predict that the dissemination of inflammatory disease state is linked to the migratory behaviours of T-cells (and indeed other leukocytes).

Dissemination of inflammatory disease state to related and distinct tissues alike, is likely associated with the dissemination of immune reaction or tolerisation against antigens. Indeed, export of both Teff and Treg populations recognizing cognate antigen from the foci of antigen encounter underpins the effectiveness of adaptive immunity. Conventional T-cell populations are well known to for central memory of antigen experience, while systemic tolerance of orally available antigens is a well-studied feature of tolerogenic T-cell populations. Indeed, activation of naive T-cell populations against cognate antigen in the periphery is likely to be a relatively rare event, requiring T-cell-antigen encounter and a subsequent series of gated activation events to occur; culminating in clonal expansion of the antigen-specific population. Thus regional, and systemic, dissemination of focal engagement and activation against T-cell antigens, via cell migration, is required for efficient function of the immune system. It is this native dissemination of antigen-specific reaction that is likely to underpin the dissemination of inflammatory states to tissues that are extrinsic, and potentially distinct in nature, to that of the primary affected tissue.

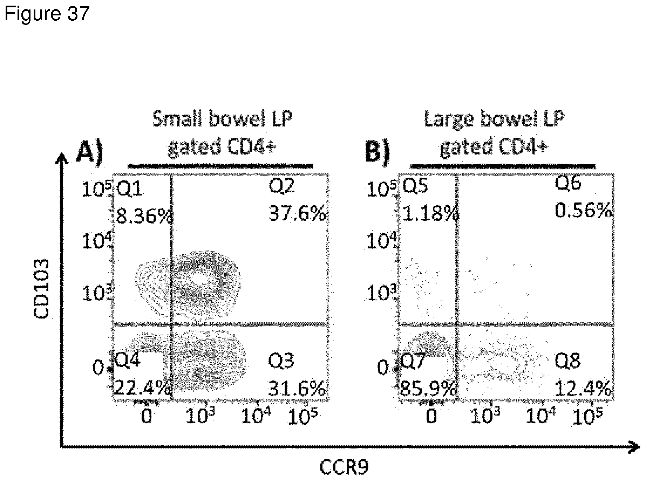

In the model presented in **figure 36** α/αX export processes, and β/βX import processes, are those that are likely to primarily represent dissemination of inflammatory disease states. It is unclear whether active migratory patterning of T-cells drives this inter-tissue communication, or arises from biological inefficiency (noise) of the migratory mechanism. What speaks for in favor of an active process is observations such as α4⁺β7⁺CCR9⁺ T-cells being observable in the LP and MLN of the colon (**Figure 37**)**.** This likely reflects that colonic-emigrant T-cells are actively patterned to recirculate to the small bowel. This would represent a mechanism of colonic-antigen experience being disseminated to small intestine, for example.

Regardless of the precise mechanisms, the dissemination of inflammatory states to distinct tissues is likely to represent an accumulative 'antigen-specific immunological dysplasia'. This is defined as a malformation of an antigen-specific and diffuse immunological tissue. For example, the over-exuberant reaction and/or under-tolerisation against a particular gluten antigen in acute celiac disease first represents a focal immunological dysplasia, in that, the incorrect reaction has been established against a given antigen at primary site(s) of encounter. Due to the constant nature of lymphocyte migratory patterns, such a focal dysplasia, results in a disseminated antigen-specific (e.g. gluten-specific in acute celiac disease) immunological tissue that is dysplastic in the short term. That is to say, the diffuse (body-wide) dysplastic antigen-specific immunological tissue is represented, in isolation, as all T-cell clones baring the particular antigen-restricted T-cell receptor clonotype sequence responsible for aberrant immunological reaction. The subsequent establishment of a pro-inflammatory state through over-exuberant antigen reaction, or under-tolerisation of antigen, can establish microenvironments that permit accumulation of antigen-reactivity within the focal, regional and/or systemic immunological dysplasia. Therefore, the antigen-specific dysplasia is capable of acquiring multiple antigen specificities to become an accumulative 'poly-antigen-specific immunological dysplasia'.

The concept of establishment of 'antigen-specific' and 'poly-antigen-specific' 'immunological dysplasia' resolves with the common feature of inflammatory disorders associated with a distinct primary disease, but do not run in parallel, and may be separated by many years in time. The formation of central memory cells of aberrant antigen reaction/tolerisation can result in a latent antigen-specific immunological dysplasia that may be triggered later in life by antigen re-encounter, or indirect provocation by unrelated infection, even if the disease at the primary location has been resolved.

The above-described concepts are closely related to Treg migratory patterns, the defined investigational framework for identification of tissue-specific Tregs, and to the treatment of secondary disease states with tissue-specific Tregs of the primary tissue. Indeed, the identification of tissue-specific Tregs permits their purification as starting material for manufacture of a cell therapeutic composition, which itself has potential to affect establishment of immune tolerance in communicating tissues given that:
i) Tissue-restricted Tregs are reactive against abundant self or commensal/environmental antigens available at distinct tissue site(s)
ii) Tissue-restricted Tregs are reactive against abundant self or commensal/environmental antigens at the primary tissue site to a sufficient degree as to establish a strongly tolerogenic environment that permits poly-antigenic tolerance.

Thus, tissue-specific Tregs have the potential to treat secondary and systemic inflammatory manifestations extrinsic in location, distinct in tissue type, and of potentially distinct antigen specificity, to that of the primary diseased tissue. Such a method, for example, could be used to treat extra-intestinal manifestations of IBD.

### Terminology of Immunology Cell Marker Identification

Through the use of flow cytometry it is possible not only to detect the presence or absence of a protein on a cell surface, but also accurately quantify how much of a protein is on the cell surface. Some plasma membrane markers are either expressed or not on a particular cell, while others have expression that can be quite graded across various cell types. For example, CD4 is either expressed or not, so cells are annotated simply as CD4- or CD4⁺, respectively. On the other hand, a graded expression of the CD25 protein is common, so CD25 expression is sometimes noted as CD25^{lo}, CD25^{int}, CD25^{hi}, for low, intermediate or high expression, respectively. It should be noted that measurement of fluorescence intensity in flow cytometric applications is generally visualised in a log scale. In addition, multi-fluorochrome analyses generally require computational compensation of data to correct for spectral overlap of the different fluoro-chromes. Therefore, depending on the content and style of analysis, marker resolution can be differently represented, even when the same antibody/fluorochrome reagent is used for analysis of the marker in question. In practical terms, this means that the resolution of X^{hi} from X^{int} populations is not always achievable, especially in more complex multivariable analyses. In such instances, it is common to refer to the X^{+/-} annotations, where the X⁺ condition is inclusive of known X^{int} and X^{hi} populations. Thus X+ may be used in the analytical or physical definition of X^{hi}, for example, so long as X^{int}/X^{hi} differentiator is not representative of a critical and otherwise unqualified descriptor of population identity.

### T-cell Migration

Integrin proteins are proteins that generally form dimeric complexes on the cell surface of two different integrin forms. These dimeric forms represent an adhesive unit that adheres to specific receptors presented on the walls of blood vessels and other structures. This means, cells that express a specific integrin pair can bind to a specific receptor, which itself can be expressed on the blood vessels in a specific tissue. In effect, the expression of specific integrin pairs on a cell can essentially barcode a cell to stick to the blood vessel walls of a specific tissue. The integrin pair responsible for sticking cells to the blood vessels of mucosal tissue is the a4β7-integrin dimer, for example.

However, to transmigrate across the cell wall into a target tissue, the cell needs to also receive a second signal, effectively serving as a further refinement of exactly what part of the selected tissue the cell should access by matching activators produced by specific tissue compartments to cognate receptors expressed by specific cells. In the case of the small bowel mucosa a small protein called CCL25, which is a "chemokine", is produced. This can trigger cells to transmigrate into the small bowel by binding to the CCR9 receptor on migrating cell surfaces. CCL25 binding to the CCR9 receptor induces the active state of the α4β7-integrin dimer, allowing tight binding and endothelial transmiration. In this example, a cell must possess both α4β7-integrin and CCR9 on their cell surface to move into the small bowel mucosa.

There exist distinct types of adhesion molecules and chemoattractants involved in directed cell migration, than the integrin and chemokine examples above.

### Therapeutic use - immunotherapy

Immunotherapy is broadly used to describe any clinical treatment that aims to modulate immune function. With respect to cellular immunotherapy, the two major fields of cellular immunotherapy focus on cell-based vaccine (mainly DC) immunotherapy and T-cell immunotherapy. In traditional vaccination, antigen preparations are injected directly to the subject to raise immune responses against antigens specific for disease pathogen. DC immunotherapy is thought to be more effective as antigens are pre-loaded onto DC cells, and they can more effectively enhance antigen cross-presentation to T-cells and B-cells *in vivo.* T-cell immunotherapies can be divided into immunostimulatory and immunosuppressive classes. Adoptive transfer of CD4 T-effector cells, or cytotoxic CD8 T-cells in the case of cancer, is seen as immunostimulatory in provoking immune responses against tumours. Treg immunotherapies by contrast aim to provide immunosuppressive capacity in treatment of inflammatory and autoimmune conditions.

The markers identified in the case study described herein may be used to define Treg populations that may be harvested from patient blood, purified *ex vivo*, expanded, repatterned, if necessary, and then infused back to the patient. The method of autologous Treg adoptive immunotherapy is thus defined at the level of cell identification by the presented markers, as a means of purification by flow cytometric (or affinity) approaches.

The Tregs as identified and obtained as described herein can be used in the treatment of inflammatory or autoimmune diseases (see above).

Aspects relating to treatment of Crohn's disease affecting the small bowel are described herein. However, as explained herein before CD may affect the whole gastrointestinal tract and, accordingly, the aspects of the invention may be broadened to treatment of CD affecting other parts of the gastrointestinal tract. Furthermore, inflammatory diseases (also outside the gastrointestinal tract) may be treated with Tregulatory cells using the same approach. As mentioned above the signatures are expected to be of similar nature. Elements of marker signatures relating to small bowel tropism and emigration, which in case e.g. of CD of the colon or perianal area should be changed to colon tropism and anal canal tropism etc, when targeting disease in these areas. That is to say, when treating other inflammatory diseases, the identity of Treg cells may be similar whereas the homing functions will be related to the tissue type and location of inflammation. However, it may be anticipated that functional makers of Tregs of a specific tissue type or anatomical location may be particular to this tissue type or location, in as much that functional makers may serve to further define Treg origin is that of the tissue of interest.

The invention also relates to a method for treating a patient suffering from an inflammatory disease, the method comprises
a) obtaining a CD4⁺ Treg cell population as described herein from a tissue sample obtained from a patient suffering from an inflammatory or autoimmune disease,
b) expanding the Treg cells *in vitro,*
c) optionally re-patterning the expanded Treg cells to obtain Tregs that have the desired for
   ii) identifying that the Treg cells are tissue type tropic, i.e. they can migrate to target tissue,
   iii) optionally, identifying that the Treg cells are diseased tissue tropic, i.e. homing cells that can localize in the diseased tissue,
   iv) identifying that the Treg cells are emigrant cells, i.e. they originate from the target tissue, and v) optionally, identifying that the Treg cells are retained in the target tissue,
d) administering the Treg cells obtained from b) or c) to the patient.

In a specific aspect, the invention relates to a method for treating a patient suffering from Crohn's disease, the method comprises
a) obtaining a CD4⁺ Treg cell population as described herein from a tissue sample obtained from a patient suffering from Crohn's disease,
b) expanding the Treg cells *in vitro,*
c) optionally re-patterning the expanded Treg cells to obtain Tregs that have the desired signatures for
   ii) identifying that the Treg cells are tissue type tropic, in this case mucosal tropic, i.e. they can migrate to mucosal tissue,
   iii) optionally, identifying that the Treg cells are diseased tissue tropic, i.e. homing cells that can localize in the diseased tissue of the gastrointestinal tract,
   iv) identifying that the Treg cells are emigrant cells, i.e. they originate from the target tissue of the gastrointestinal tract, and
   v) optionally, identifying that the Treg cells are retained in the target tissue of the gastrointestinal tract,
d) administering the Treg cells obtained from b) or c) to the patient.

The expanded and optionally repatterned Treg cells from step b) or c) should have features as defined herein.

The Tregs are suitably obtained from a tissue sample from a patient. The sample may be from a lymph node such as a mesenteric lymph node draining inflamed bowel, or it may be from bowel mucosa, from lamina propria or it may be from a blood sample. Most conveniently, the sample is a peripheral blood sample.

The step referred to as step a) refers to first the recovery of mononuclear cells from patient tissue specimens, and labelling said pool of mononuclear cells with antibodies specific for appropriate markers. The cells can be retrieved from mucosa through microdissection of lamina propria and preparation of the tissue e.g. using enzyme collagenase and other substances. The cells may also be prepared from lymph nodes starting with microdissective trimming of the tissue followed by careful mechanical degradation before using collagenase and substances mentioned above. The cells may also be prepared from peripheral blood.

Once labelled, cells are purified by immunoaffinity and/or flow cytometric sorting techniques to yield highly enriched or purified Treg populations of desired characteristics. In vitro expansion of isolated Treg populations as referred to in step b) is achieved by way of recombinant T-cell stimulation in the form of anti-CD3/anti-CD28 activating antibodies in combination with IL2, or alternatively the outgrowth of Treg populations on transgenic feeder cell populations, or irradiated autologous/allogeneic APCs with IL2 supplementation. Repatterning of the correct homing receptor expression post-expansion as referred to in c) entails the recombinant reactivation of expanded T-cell populations with anti-CD3/anti-CD28 activating antibodies and subsequent introduction of stimuli in precise combination Stimuli include all-trans retinoic acid, Interleukin-10 and transforming growth factor-beta.

In the case where the Treg cells lack the signature α4⁺β7⁺ or α4⁺β7⁺CCR9⁺, the signature may be introduced or re-introduced to the Treg cell by stimulation of cells with a combination of ATRA, TGFbeta and IL10. In the case that the repatterning relates to the signature α4⁺β7⁺X, the repatterning stimulation is the same as mentioned above, but includes additional rapamycin supplementation.

### Identification, purification and expansion of Tregs

The Tregs are identified, obtained and purified as described herein. Thus, the identification and purification typically involve the use of specific antibodies and techniques well known to a person skilled in the art.

One set of methods central to manipulating cells from the human body are those that identify a given cell type, and allow their purification as viable cells. In the following is described the key principals of cell identification and purification by direct and indirect means. There are many additional parameters by which cells can be identified, but are destructive in nature, so bare no use in purification and cloning of living cells.

### Direct Antibody Detection of Plasma Membrane Markers by Flow Cytometry

The most important method in cellular immunology is the specific detection of surface proteins by way of specific antibodies. Considering cells of different types inevitably express different proteins on their cells surface, identifying specific protein signatures on their surface is the simplest direct means of identifying a given cell type. For instance, CD4 and CD8 form the basis of identifying T-cells in most applications.

Antibodies can be created in controlled conditions against specific proteins, or peptide fragments of proteins. This is simply achieved by injecting a laboratory animal, usually a mouse or rabbit, with a quantity of protein or peptide antigen. In biochemical applications it is often sufficient to use a preparation of the animal's blood to recover large amounts of antibodies, and are termed polyclonal antibodies, since multiple different antibody clones populate these preparations. In contrast, monoclonal antibody production utilizes cloning and characterisation of B-cells from the antigen-challenged animals. The basis for cellular and molecular cloning of antibodies will be discussed further in later sections, but for now we can see that specific single antibodies can be generated for any protein.

It is simply not enough to bind specific antibodies to a cell surface; there must also be a means of detecting each individual antibody. This is most commonly achieved by labelling each antibody with a specific fluorescent dye. Fluorescence simply describes the spectral properties of a molecule that can be excited with light of a specific colour, and will then emit light of a different colour. By labelling each antibody with a different colour, many different antibodies can be used to bind specific proteins on a cell surface, and each be quantitatively detected by the amount of fluorescent signal of each colour emitted by the cell when appropriately excited.

Excitation of different fluorescent dyes in the platforms that we will discuss is achieved by means of different coloured lasers. That is to say lasers emitting light of differing wavelengths. The instrument that often is used to detect multi-parameter fluorescence of cells is called a "flow cytometer". These instruments take cells suspended in solution and flow them, one-by-one, past an array of lasers and photodetectors. We are thus able to measure extremely accurately and rapidly the expression of specific proteins on the surface of individual cells. This technique forms the basis of the vast majority of cellular immunology analysis in both experimental and clinical settings

### Direct Cell Purification by FACS

The use of flow cytometry to purify cells is called fluorescence-activated cell sorting (FACS). FACS instruments represent the same basic principle as analytical flow cytometers, though after the detection of cell fluorescence are able to physically sort cells. FACS instruments can sort cells in two basic manners. First, cells can be identified and sorted into up to four separate pools of cells. Second, single cells can be identified and deposited into single tubes. The single cell deposition is a powerful means of cell identity-based cloning, where individual cells represent clones that may be propagated, characterised and manipulated. A traditional method of single cell cloning is by 'limiting dilution'. This means you have a starting pool of cells, and you dilute these cells so there is on average less than one cell per given volume. The volume of cell suspension is then aliquoted such that you achieve single-cell distribution.

### Direct Cell Purification by MACS

Magnetic-activated cells sorting (MACS) technology is another method that can be used. The premise is basically that instead of a fluorescent label, specific antibodies are linked to magnetised microbeads. This in effect means that one is able to effectively magnetise specific cells based antibody binding. The largest drawback of this approach is the obvious limitation to the number of antibodies one can use, since a single antibody bound to a cell surface will magnetise the cell. It is most common to purify cells by a process of negative selection, that is, to magnetise all of the cells that you do not want to purify, and deplete these from your sample. The sophistication of the cell identities that can be purified is relatively low compared to FACS, and inevitably of much lower purity.

### Legends to figures

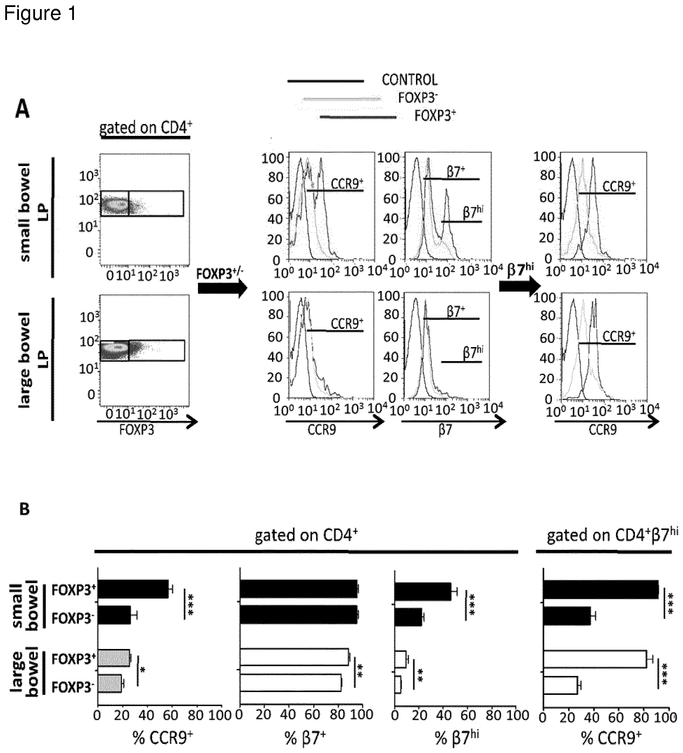

**Figure 1****. CD4⁺FOXP3⁺T_{regs} isolated from human intestinal LP carry higher levels of CCR9 than CD4⁺FOXP3⁻ T_{eff} counterparts.** (A) Single cell suspensions were prepared from LP dissected from histologically normal small and large bowel, resected from a representative CD patient with ileoceacal disease. Cells were stained for CD4, FOXP3, β7 and CCR9, then analysed by flow cytometry. Lymphocytes were gated for CD4⁺, expressed as CD4vsFOXP3 dotplots (left hand panels), and Tregs defined as CD4⁺FOXP3⁺ with CD4⁺FOXP3⁻ defined as effector T-cells. Overlaid histograms of CD4⁺FOXP3⁺ and CD4⁺FOXP3⁻ populations are presented for CCR9 and β7 signal intensities relative to singly unstained controls. (middle panels) and for CCR9 intensity in the CD4⁺β7^{hi}FOXP3⁺ in addition to CD4⁺β7^{hi}FOXP3⁻ populations (right hand panels). Histogram gates in (A) were used to quantify percentage of positive cells (n=3), presented in (B).

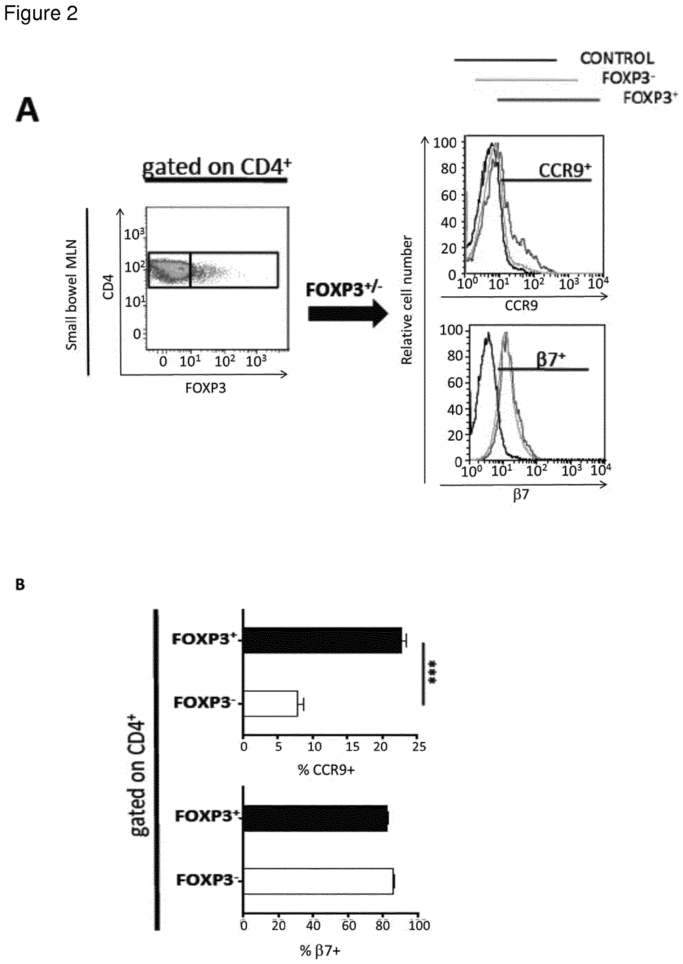

**Figure 2****. CD4⁺FOXP3⁺Tregs isolated from human MLN draining small bowel carry higher levels of CCR9 than CD4⁺FOXP3⁻ T_{eff} counterparts.** (A) Single cell suspensions were prepared from MLNs draining the small bowel, resected from a CD patient with ileoceacal disease. Cells were stained for CD4, FOXP3, β7 and CCR9, then analysed by flow cytometry. Lymphocytes were gated for CD4⁺, expressed as CD4vsFOXP3 dotplots (left hand panel), and Tregs defined as CD4⁺FOXP3⁺ with CD4⁺FOXP3⁻ defined as effector T-cells. Overlaid histograms of CD4⁺FOXP3⁺ and CD4⁺FOXP3⁻ populations are presented for CCR9 and β7 signal intensities relative to singly unstained controls (right hand panels). Histogram gates in (A) were used to quantify percentage of positive cells (n=3), presented in (B).

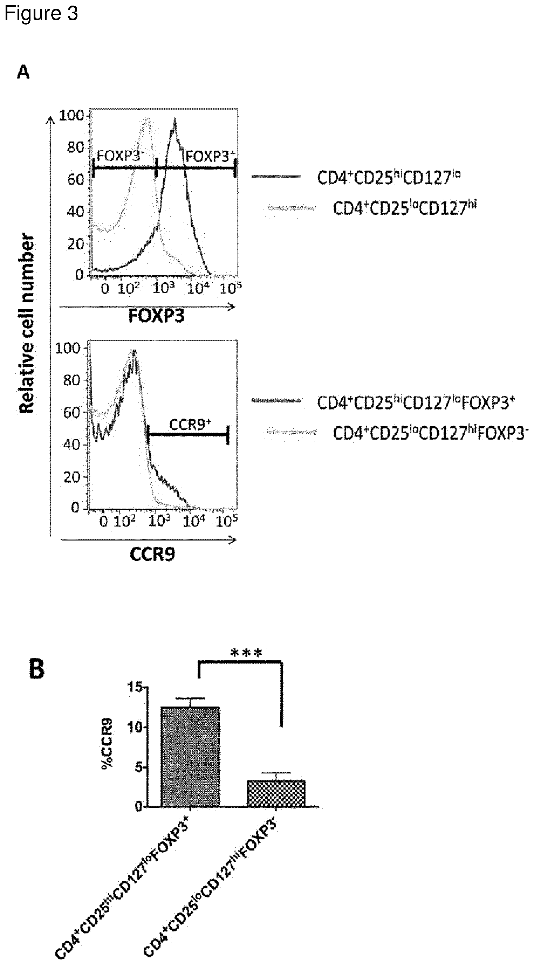

**Figure 3****. CD4⁺CD25^{hi}CD127^{lo}FOXP3⁺Tregs have higher CCR9 expression than CD4⁺CD25^{lo}CD127^{hi}FOXP3⁻non-Tregs in MLN draining the small bowel.** A) Single cell suspensions were prepared from small bowel draining MLNs resected from a CD patient and stained for CD4, CD25, CD127, FOXP3 and CCR9. CD4⁺CD25^{hi}CD127^{lo} Treg and CD4⁺CD25^{lo}CD127^{hi} effector T-cell populations were gated and overlaid as histograms of FOXP3 intensity (top panel). Histogram gates were used to select FOXP3⁺ and FOXP3⁻ populations and expressed as overlaid histograms of CCR9 intensity for CD4⁺CD25^{hi}CD127^{lo}FOXP3⁺Treg and CD4⁺CD25^{lo}CD127^{hi}FOXP3⁻ effector populations (bottom panel). CCR9 histogram gate was used to quantify results (n=3) in (B).

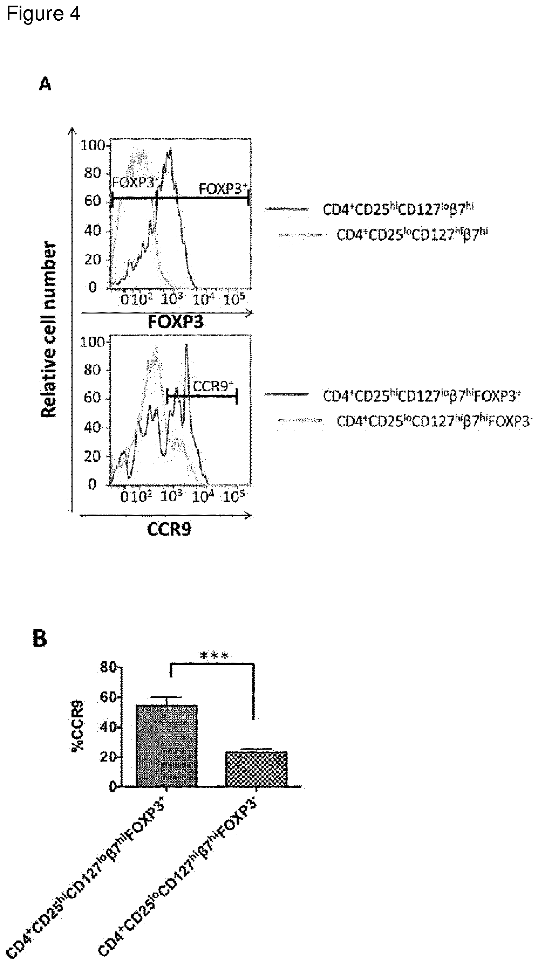

**Figure 4****. CD4⁺CD25^{hi}CD127^{lo}β7^{hi}FOXP3⁺Tregs have higher CCR9 expression than CD4⁺CD25^{lo}CD127^{h}β7^{hi}FOXP3⁻ non-Tregs in peripheral blood.** PBMCs were prepared from healthy controls and stained for CD4, CD25, CD127, FOXP3, β7 and CCR9. CD4⁺CD25^{hi}CD127^{lo}β7^{hi}Treg and CD4⁺CD25^{lo}CD127^{hi}β7^{hi} effector T-cell populations were gated and overlaid as overlaid histograms of FOXP3 intensity (top panel). Histogram gates were used to select FOXP3⁺ and FOXP3⁻ populations and expressed as overlaid histograms of CCR9 intensity for CD4⁺CD25^{hi}CD127^{lo}β7^{hi}FOXP3⁺Treg and CD4⁺CD25^{lo}CD127^{hi}β7^{hi}FOXP3⁻T_{eff} populations (bottom panel). CCR9 histogram gate was used to quantify results (n=3) in (B).
**Figure 5****. CD4⁺CD38⁺CD62L⁻ mucosal-educated FOXP3⁺ T_{regs} in peripheral circulation carry higher CCR9 marking than FOXP3⁻ T_{effs} and CD patients have diminished overall CCR9 marking on mucosal-educated T-cells.** (A) PBMCs prepared from healthy controls (HC) were stained for CD4, FOXP3, CD38, CD62L, β7 and CCR9 then analysed by flow cytometry. Lymphocytes were gated for CD4⁺, expressed as CD38vsCD62L dot plots and divided into quadrants (left hand panel). Histograms of each quadrant are displayed for signal intensity of β7, CCR9 and CCR9 after further gating on β7^{hi} (upper panels). Histogram gates were used to quantify signal intensities in each quadrant (n=7, lower panels). (B-D) PBMCs isolated from HC (n=7) and small bowel CD patients (n=6) were analysed as in (A). (B) Percentage of FOXP3⁺ (upper panel) and CCR9⁺ (lower panel) cells among total CD4 cells. (C) Percentage of FOXP3⁺ (upper panel) and CCR9⁺ (lower panel) cells among gated CD4⁺CD38⁺CD62L⁻β7^{hi} cells. (D) Percentage of CCR9⁺ cells among gated CD4⁺CD38⁺CD62L⁻β7^{hi} further gated on FOXP3⁺ and FOXP3⁻ cells.

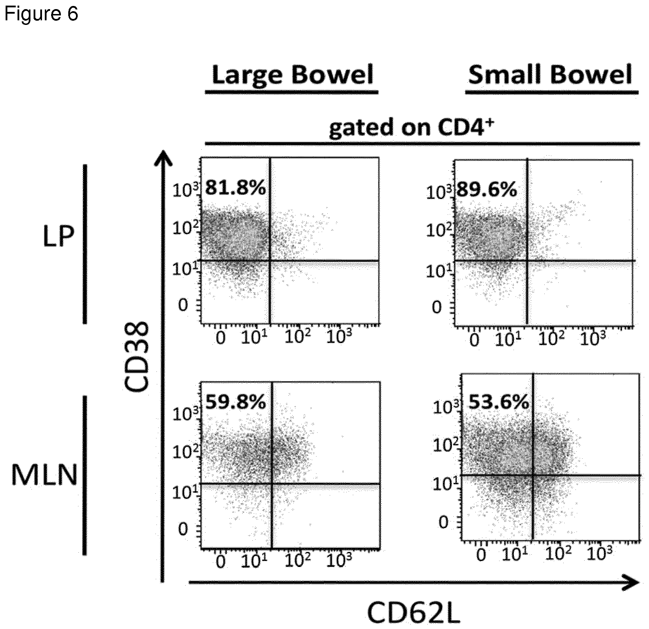

**Figure 6****. T-cells of CD38⁺CD62L⁻ phenotype predominate in the LP of the small and large bowel.** Single cell suspensions prepared from LP (top panels) and MLN (bottom panels) of large bowel (left panels) and small bowel (right panels) were stained for CD4, C38 and CD62L. CD4⁺ cells were expressed as CD38vsCD62L dotplots. Values in upper left quadrant represent percentage of CD38+CD62L- CD4 cells in this quadrant (n=1). Small bowel LP and MLN were from the ileum, large bowel LP and MLN were from the right colon resected from patient undergoing surgery for colorectal cancer.

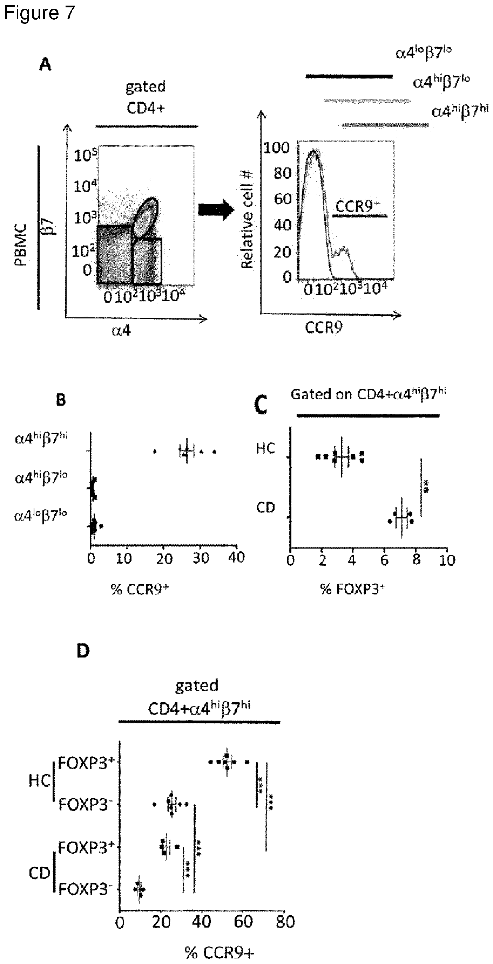

**Figure 7****. CD4⁺α4^{hi}β7^{hi} mucosal-tropic FOXP3⁺ Tregs in peripheral circulation carry higher CCR9 marking than FOXP3⁻ T_{effs} and CD patients have diminished CCR9 marking on mucosal-tropic T-cells** (A) PBMCs prepared from HC were stained for CD4, FOXP3, β7, α4 and CCR9 then analysed by flow cytometry. Lymphocytes were gated for CD4⁺, expressed as β7vsα4 dot plots and gated for α4^{hi}β7^{hi}, α4^{hi}β7^{lo}, α4^{lo}β7^{lo} (left panel), and displayed as overlaid histograms of CCR9 intensity (right panel). (B) Quantified CCR9 positivity among HC (n=7), for analysis as performed in (A). (C-D) PBMCs isolated from HC (n=7) and CD patients (n=4) were analysed as in (A). (C) Percentage of FOXP3⁺ cells among gated CD4⁺α4^{hi}β7^{hi} cells. (D) Percentage of CCR9⁺ cells among gated CD4⁺α4^{hi}β7^{hi} cells, further gated on FOXP3⁺ and FOXP3⁻ cells.

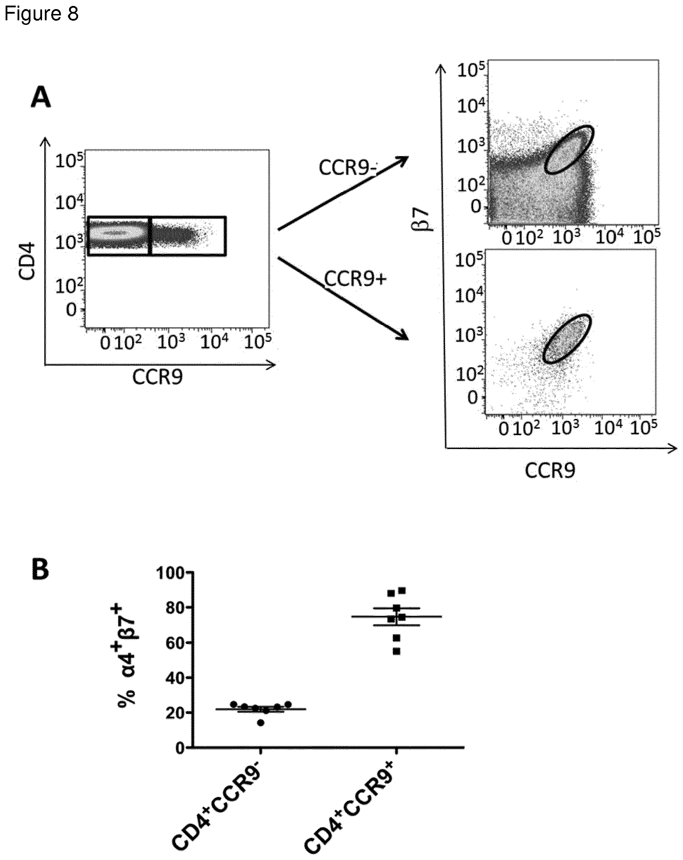

**Figure 8****. The majority of CD4⁺CCR9⁺ T-cells in peripheral circulation carry α4^{hi}β7^{hi} expression.** (A) PBMCs prepared from healthy controls and stained for CD4, α4, β7 and CCR9. CD4⁺ cells were expressed as CD4vsCCR9 dotplots and CD4⁺CCR9⁺ and CD4⁺CCR9⁻ populations subsequently expressed as β7vsα4 dotplots. The percentage of cells inside the α4^{hi}β7^{hi} gate is quantified (n=7) in (B).

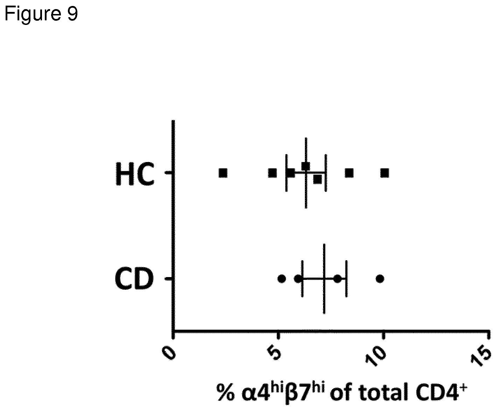

**Figure 9****. No difference in total numbers of CD4⁺α4^{hi}β7^{hi} T-cells in peripheral circulation of CD patients and healthy controls.** PBMCs prepared from healthy controls (HC) and CD patients were stained for CD4, FOXP3, β7, α4 and CCR9 then analysed by flow cytometry. Lymphocytes were gated for CD4⁺ and expressed as β7vsα4 dotplots. The percentage of CD4+ cells inside the α4^{hi}β7^{hi} is shown for HC (n=7) and CD patients (n=4).

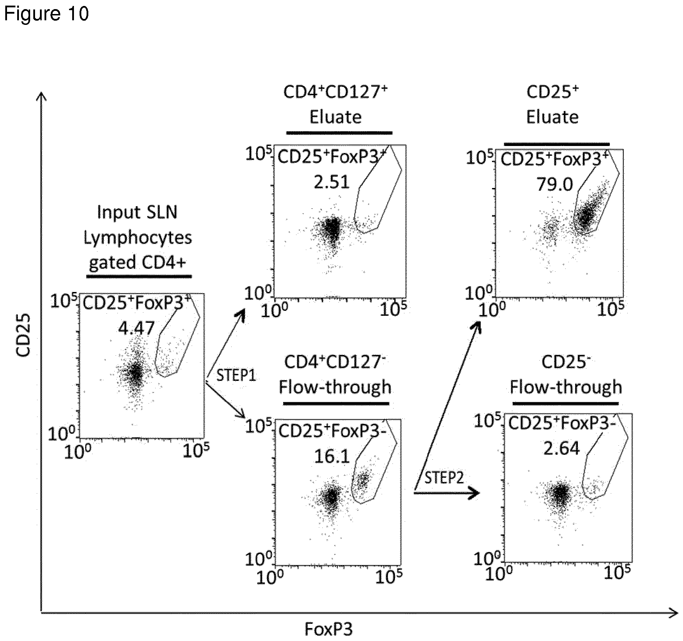

**Figure 10****. MACS 2-Step enrichment of MLN Tregs.** Total lymphocytes recovered from MLN of patient with CD were processed using Miltenyi Regulatory T Cell Isolation Kit II and an autoMACSpro instrument. Input cells are displayed in the far left plot, gated on CD4, and expressed as CD25vsFOXP3. CD25^{hi}FOXP3⁺ cells are enriched in the CD4⁺CD127⁻ flow-through of the first negative selection step, then compared to CD4⁺CD127⁺ positively selected eluate from the same step (middle panels). Further enrichment of CD25^{hi}FOXP3⁺ cells to approximately 80% is observed in the positive selection eluate of the second MACS step.

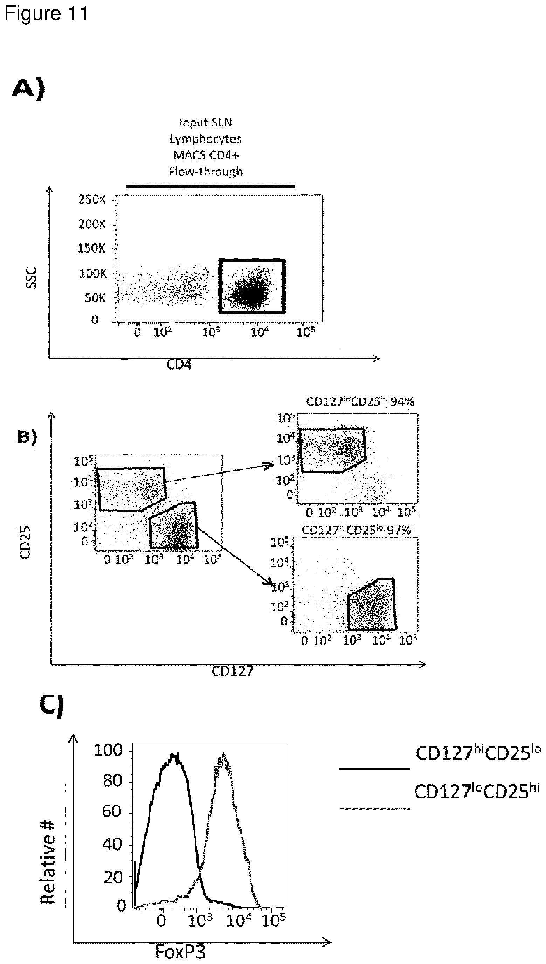

**Figure 11****. MACS enrichment and FACS purification of Tregs from MLN.** Total lymphocytes recovered from MLN of patient with CD were processed using Miltenyi CD4+ T Cell Isolation Kit II and an autoMACSpro instrument. Cells were immediately labeled with CD4, CD25 and CD127 antibodies and run on FACSaria II instrument. Un-gated events are displayed as SSCvsCD4 to assess enrichment (A). CD4-gated events in A) are displayed in the left panel of B). CD25^{hi}CD127^{lo} and CD25^{lo}CD127^{hi} gates of the resulting plot represent sorting-gates (left panel). A small amount of resulting sorted populations were immediately re-acquired on the FACSaria and displayed in the same plot format (right panels) to assess purity. A portion of the resulting cell population was fixed/permeabilised and stained for FOXP3. Cells were acquired on a FACScalibur instrument, and displayed as FOXP3 histograms of CD25^{lo}CD127^{hi} and CD25^{hi}CD127^{lo} populations (C).

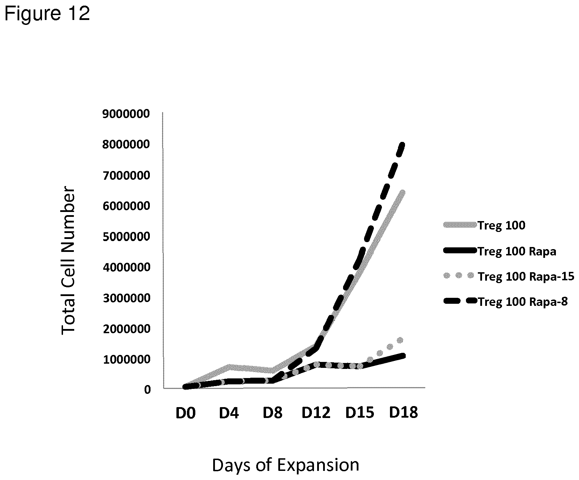

**Figure 12****. Viable cell numbers of MACS-enriched MLN Tregs over 18 days of ex vivo expansion.** Total lymphocytes recovered from MLN of patient with CD were processed using Miltenyi Regulatory T Cell Isolation Kit II and an autoMACSpro instrument. Resulting CD4⁺CD25^{lo}CD127^{hi} cells were rested overnight before being activated for 24hrs with antiCD3/CD28 magnetic microbeads. After 24 hours of activation IL2 (100 IU.mL) was added in the presence or absence of Rapamycin. Magnetic beads were removed after 24hrs further culture, and IL2 and rapamycin were refreshed every 24 to 48 hrs while maintaining cell density below 3x10⁶.mL by addition of fresh media and culture vessel exchanges. All cells were restimulated with antiCD3/CD28 magnetic microbeads for 24hrs starting on day 12. Treg100 group was cultured without rapamycin supplement for the entire 18 day experiment, where Treg100 Rapa groups was cultured with rapamycin for the entire 18 days.

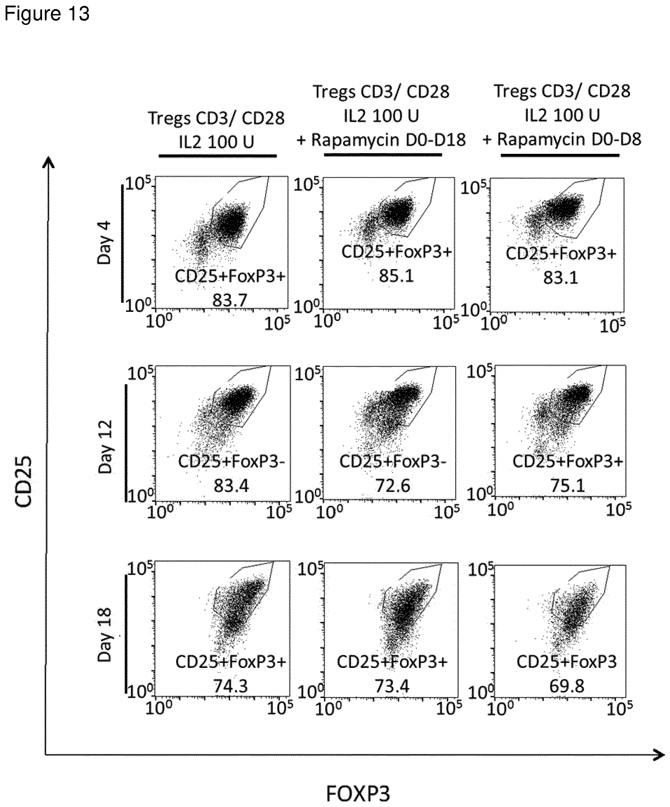

**Figure 13****. CD25-FOXP3 expression on MACS-enriched MLN Tregs on days 4, 12 and 18 of 18-day of ex vivo expansion.** MLN Treg expansion from Figure 12 analysed at days 4, 12 and 18 for expression of CD25 and FOXP3. Cells were recovered for counting viable population of each day, and a small aliquot stained for CD25 and FOXP3. Data was acquired on a FACScalibur instrument. Dead cells are excluded by Live/Dead fixable viability stain.

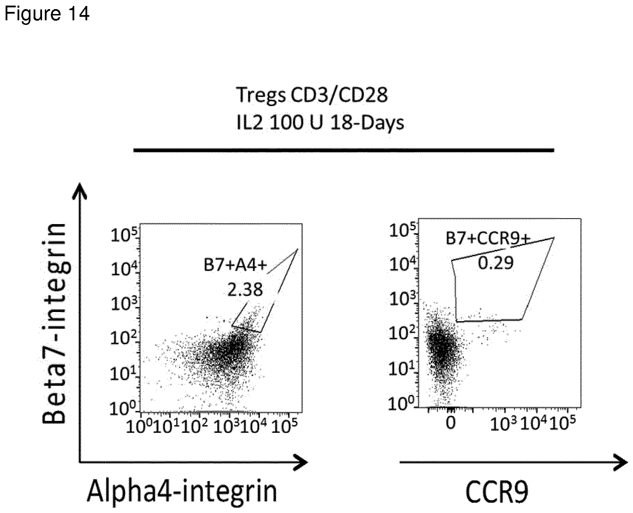

**Figure 14****. Homing receptor pattern on MACS-enriched MLN Tregs after 18-days of ex vivo expansion.** MLN Treg expansion from Figure 12 analysed at day 18 for expression of beta7- and alpha4- integrins and CCR9 expression. Cells were recovered for experimental manipulation, and a small aliquot stained for beta7- and alpha4- integrins and CCR9. Data was acquired on a FACScalibur instrument. Dead cells are excluded by Live/Dead fixable viability stain.

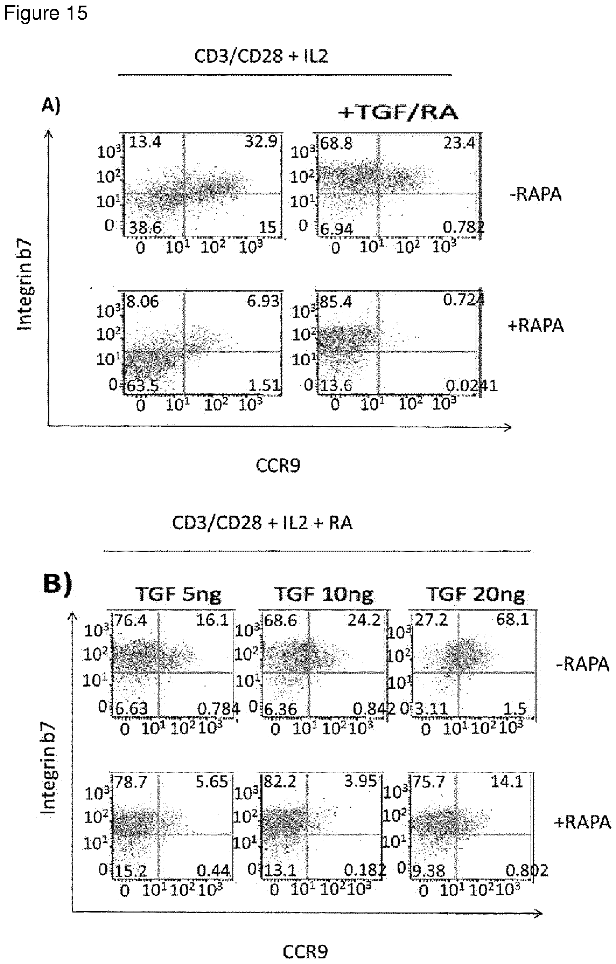

**Figure 15****. Homing recpetor expression and repatterning on MACS-enriched and FACS-purified MLN Tregs after 4-days of ex vivo expansion.** Total lymphocytes recovered from MLN of patient with CD were processed using Miltenyi CD4+ T Cell Isolation Kit II and an autoMACSpro instrument. Cells were immediately labeled with CD4, CD25 and CD127 antibodies and sorted on FACSaria II instrument for CD4⁺CD25^{hi}CD127^{lo} cells. Resulting purified population of Tregs was rested overnight before 24hr activation with antiCD3/CD28 magnetic microbeads. After 24 hours IL2 and all other indicated stimuli were added, and cultures left for a further 72hrs. all-trans retinoic acid (RA) was added an 1nM final concentration, IL2 at 100 IU.mL and microbeads at a ratio of 1:1 beads to cells. TGF in A) was added at 5ng.mL. Rapamycin was added at 100nM final concentration. Cells were recovered in 5-volumes of FACS-buffer, washed briefly, and stained for beta7-integrin and CCR9 before analysis on FACScalibur instrument.

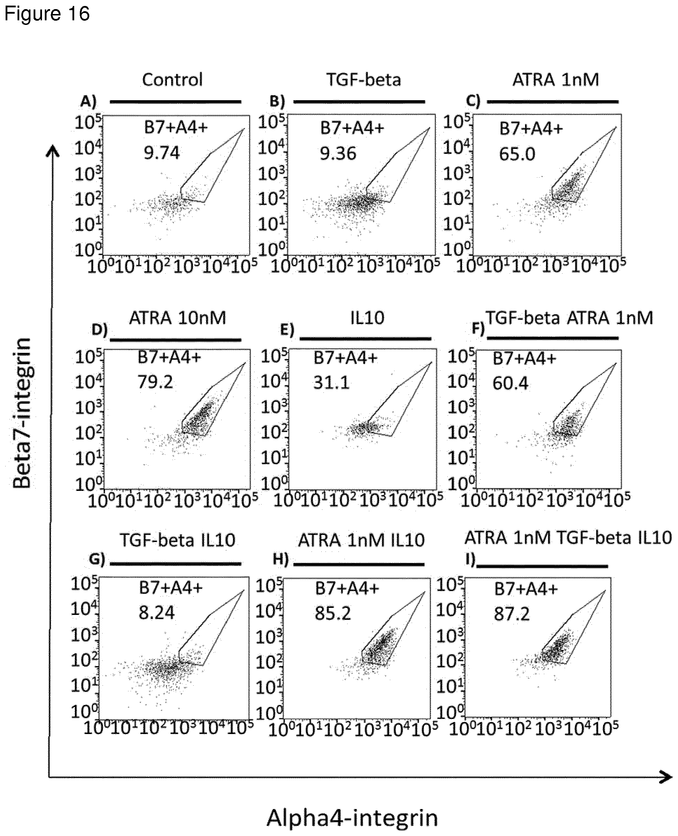

**Figure 16****. α4β7 receptor pattern on MACS-enriched MLN Tregs** after **18-days of ex vivo expansion and 6-days of receptor repatterning stimulation.** MLN Treg expansion from Figure 12 and 14 were recovered and aliquoted at 200k cells per well of a 96-well U-bottom plate in 150 uL medium. Cells were stimulated with 100 IU.mL IL2, and indicated stimuli. Control was unstimulated other than IL2. TGF-beta concentration was 12.5 ng.mL. IL10 concentration was 1 ng.mL. Cell medium and stimuli were refreshed at days 2 and 4 by pipetting 50 µL of media SN, and resuspending the entire well with 50 µL fresh supplemented media. On day-6 cells were recovered in 5-volumes of FACS-buffer, washed briefly and stained for beta7- and alpha4- integrins, and CCR9, before analysis on FACScalibur instrument. Dead cells are excluded by Live/Dead fixable viability stain.

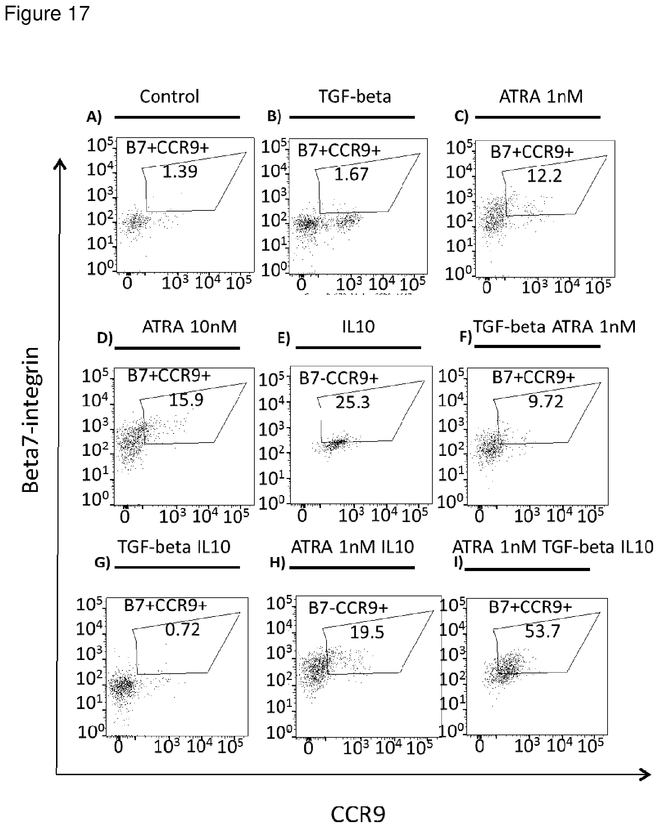

**Figure 17****. α4β7 receptor pattern on MACS-enriched MLN Tregs after 18-days of ex vivo expansion and 6-days of receptor repatterning stimulation.** Analysis from Figure 16 re-presented as beta7-integrin vs CCR9.

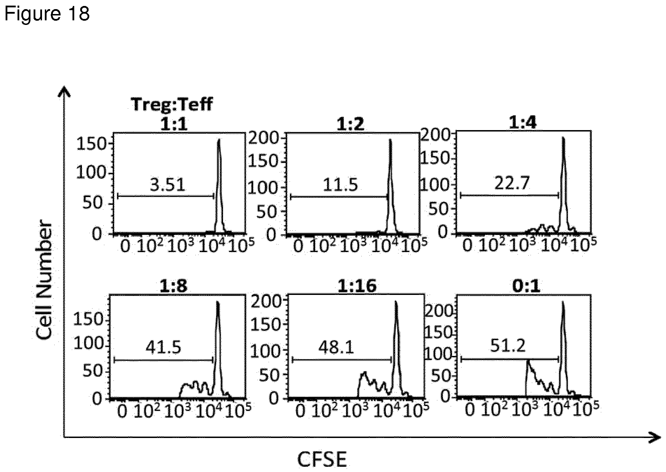

**Figure 18****. Day-18 expanded SLN Tregs suppress fresh-thawed CD4 Teff proliferation.** Cryopreserved total SLN cells were thawed on Day 15 of Treg expansion and cultured for 48 hours in the presence of IL2. On day 17 Teff cells were sorted as CD4+CD127hiCD25lo and stimulated overnight with CD3/CD28 dynal beads. On day 18 expanded Treg cells were harvested and mixed at indicated ratios with bead-depleted and CFSE labelled Teff cells. CFSE fluorescence was assessed by flow cytometry 4 days after initiation of assay with FOXP3 counterstaining, where histogram show FOXP3- cells.
**Figure 19****. CD4⁺CD25^{hi}FOXP3⁺ Tregs and CD4⁺CD25^{lo}FOXP3⁻ Teffs** have **altered CD38⁺CD62L⁻ distribution in inflamed tissues of CD patients.** Single cell suspensions prepared from indicated resected tissues of a representative CD patient with ileocaecal disease were immediately stained with the indicated antibodies. All plots display CD38 vs CD62L of CD4⁺CD25^{hi}FOXP3⁺ Tregs (right panels) and CD4⁺CD25^{lo-}FOXP3⁻ Teffs (left panels).

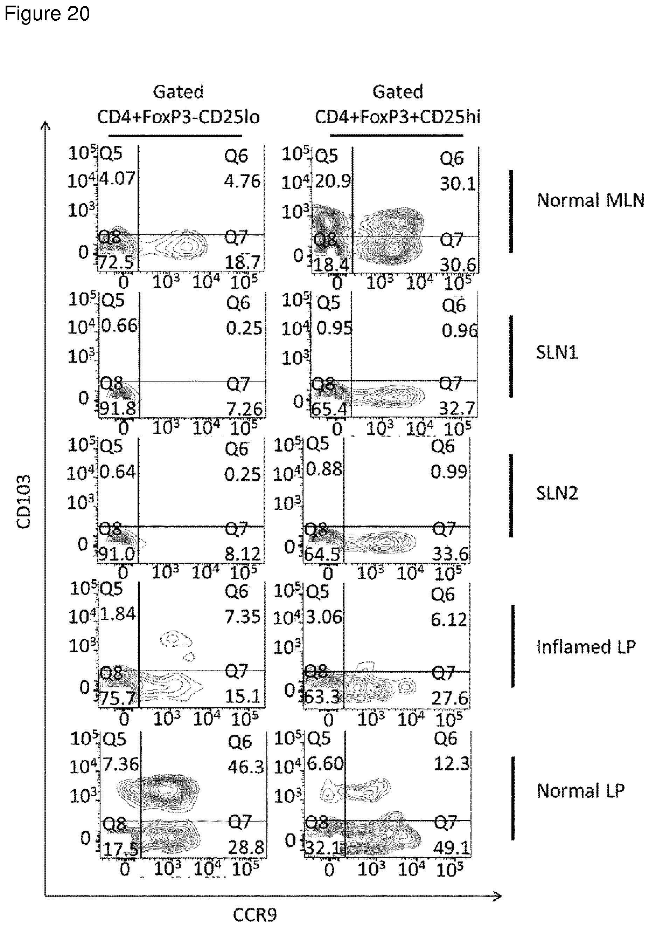

**Figure 20****. CD4⁺CD2S^{hi}FOXP3⁺ Tregs and CD4⁺CD25^{lo}FOXP3⁻ Teffs have altered CD103 and CCR9 expression in inflamed tissues of CD patients.** Single cell suspensions prepared from indicated resected tissues of a representative CD patient with ileocaecal disease were immediately stained with the indicated antibodies. All plots display CD103 vs CCR9 of CD4⁺CD25^{hi}FOXP3⁺ Tregs (right panels) and CD4⁺CD25^{lo-}FOXP3⁻ Teffs (left panels).

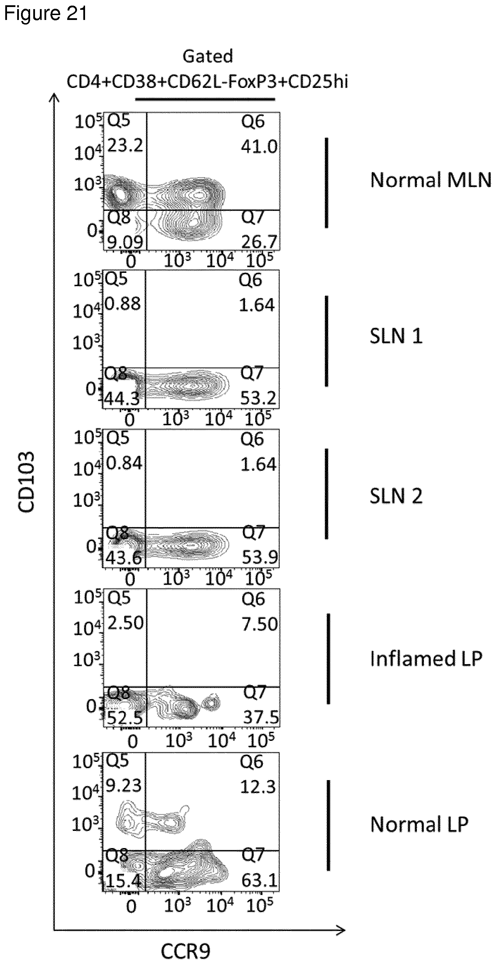

**Figure 21****. CD4⁺CD25^{hi}FOXP3⁺CD38⁺CD62L⁻ Tregs have altered CD103 and CCR9 expression in inflamed tissues of CD patients.** Single cell suspensions prepared from indicated resected tissues of a representative CD patient with ileocaecal disease were immediately stained with the indicated antibodies. All plots display CD103 vs CCR9 of CD4⁺CD25^{hi}FOXP3⁺CD38⁺CD62L⁻ Tregs.
**Figure 22****. Diminished representation of CD45⁺CD11c^{hi}CD80⁺HLA-DR^{hi}CD103⁺ DCs in inflamed MLN of CD patients.** Single cell suspensions prepared from indicated resected tissues of a representative CD patient with ileocaecal disease were immediately stained with the indicated antibodies. All plots display HLA-DR vs CD103 of CD45⁺CD11C^{hi}CD80⁺ cells.

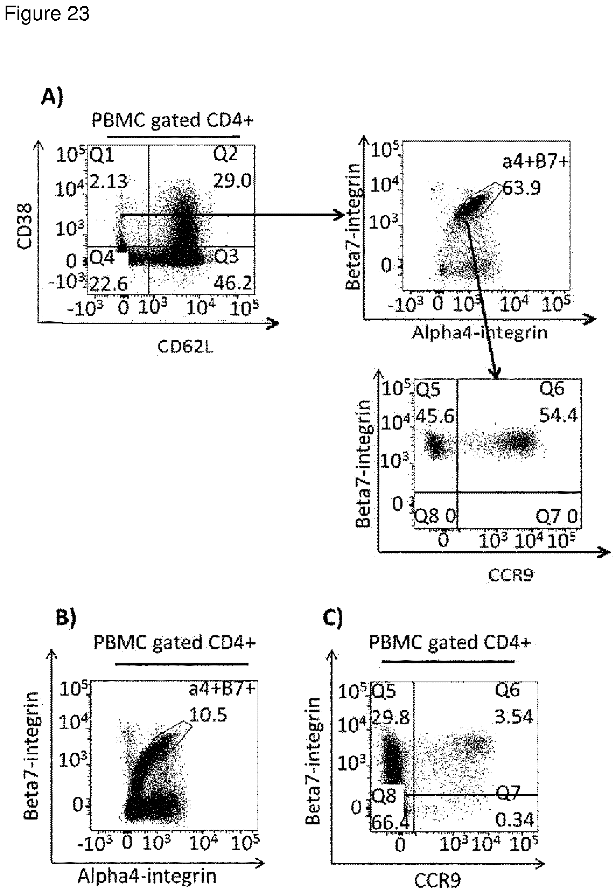

**Figure 23****. CCR9 enrichment in CD4⁺CD38⁺CD62L⁻ α4⁺β7⁺ T-cells in the peripheral blood.** PBMC recovered from healthy donor blood over ficoll were immediately labelled with indicated antibodies and analysed by flow cytometry. A) Displays gating strategy flow with arrows. B) and C) display total CD4+ PBMCs as reference.

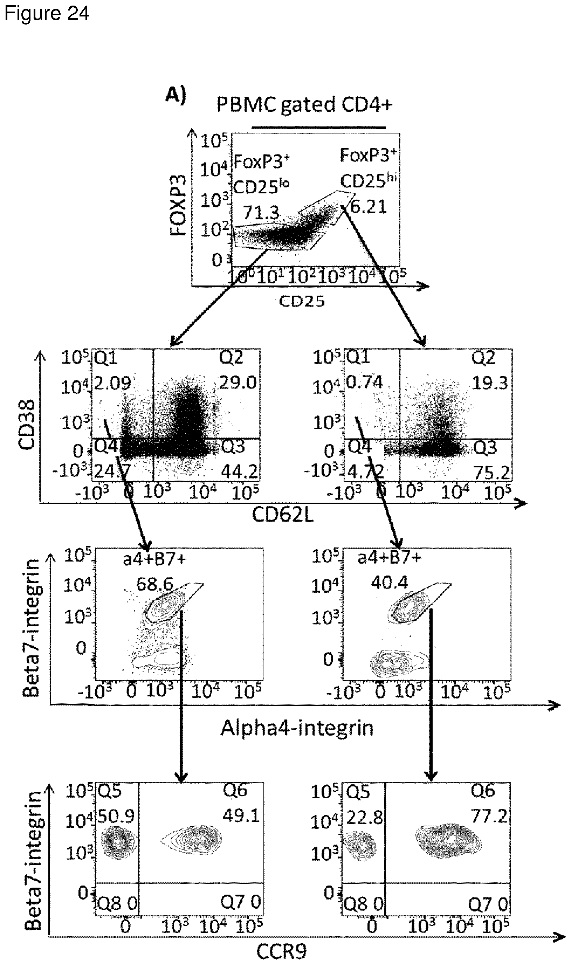

**Figure 24****. CCR9 enrichment in CD4⁺FOXP3⁺CD25^{hi}CD38⁺CD62L⁻ α4⁺β7⁺ T-cells in the peripheral blood.** PBMC recovered from healthy donor blood over ficoll were immediately labelled with indicated antibodies and analysed by flow cytometry. A) Displays gating strategy flow with arrows. Left panels display FOXP3⁻CD25^{lo} Teffs and right panels display FOXP3⁺CD25^{hi}Tregs.

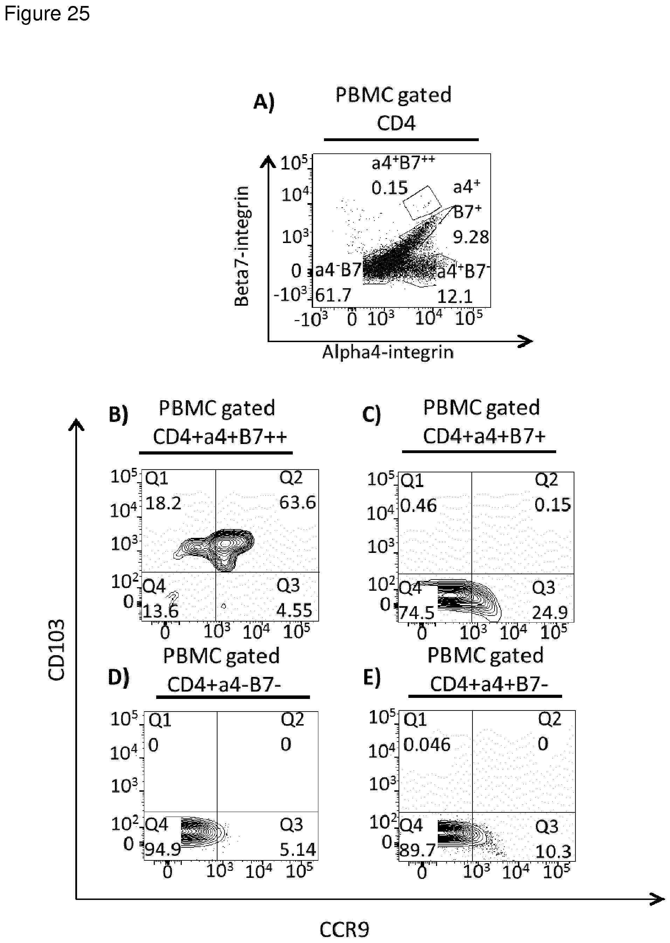

**Figure 25****. CD4⁺**α**4⁺β7^{high} T-cells in the peripheral blood are enriched for CD103 and CCR9 expression.** PBMC recovered from healthy donor blood over ficoll were immediately labelled with indicated antibodies and analysed by flow cytometry. A) Total CD4 lymphocytes expressed as beta7- vs alpha4- integrin dot plots. Each gate defining alpha4+beta7++, alpha4+beta7+, alpha4-beta7- and alpha4+beta7- are redisplayed as CD103 vs CCR9 contour plots in B), C), D) and E), respectively.

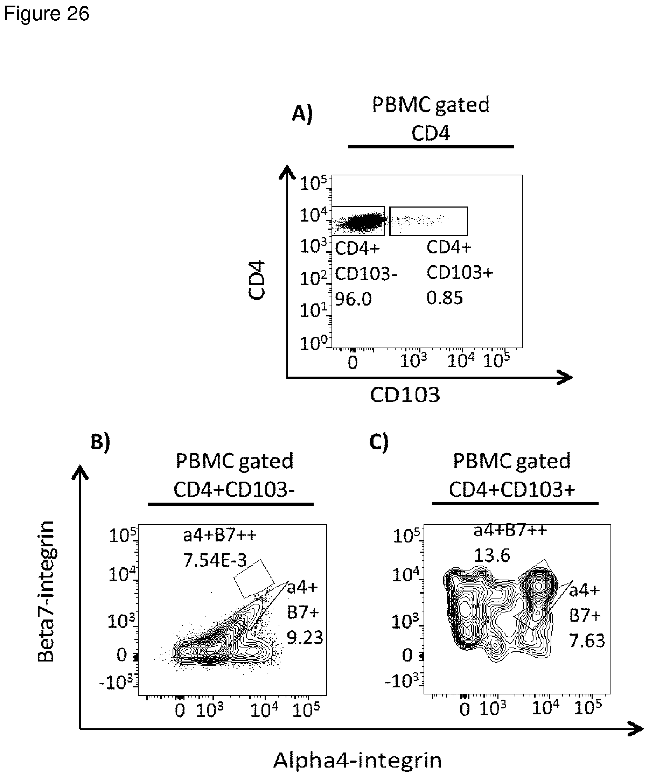

**Figure 26****. CD4⁺CD103⁺T-cells in peripheral circulation are highly enriched for** α**4⁺β7^{high} expressing T-cells.** PBMC recovered from healthy donor blood over ficoll were immediately labelled with indicated antibodies and analysed by flow cytometry. A) Total CD4 lymphocytes expressed as CD4 vs CD103 dot plots. Each gate defining CD4+CD103- and CD4+CD103+ is redisplayed as beta7- vs alpha4- integrin contour plots in B) and C), respectively.

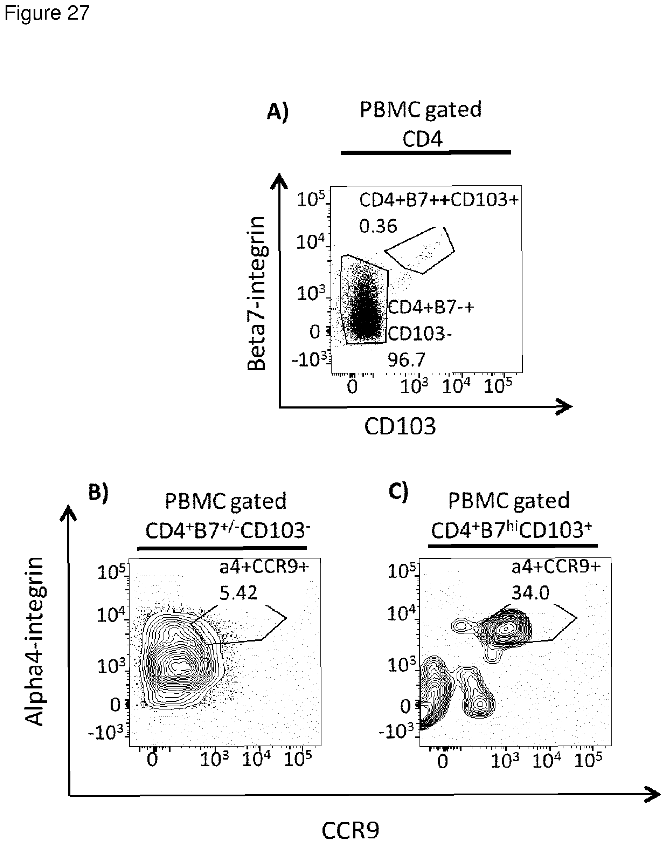

**Figure 27****. CD4⁺β7^{high}CD103⁺T-cells in peripheral circulation are enriched for** α**4⁺CCR9 expressing T-cells.** PBMC recovered from healthy donor blood over ficoll were immediately labelled with indicated antibodies and analysed by flow cytometry. A) Total CD4 lymphocytes expressed as beta7-integrin vs CD103 dot plots. Each gate defining beta7+-CD103- and beta7++CD103+ is redisplayed as alpha4- integrin vs CCR9 contour plots in B) and C), respectively.

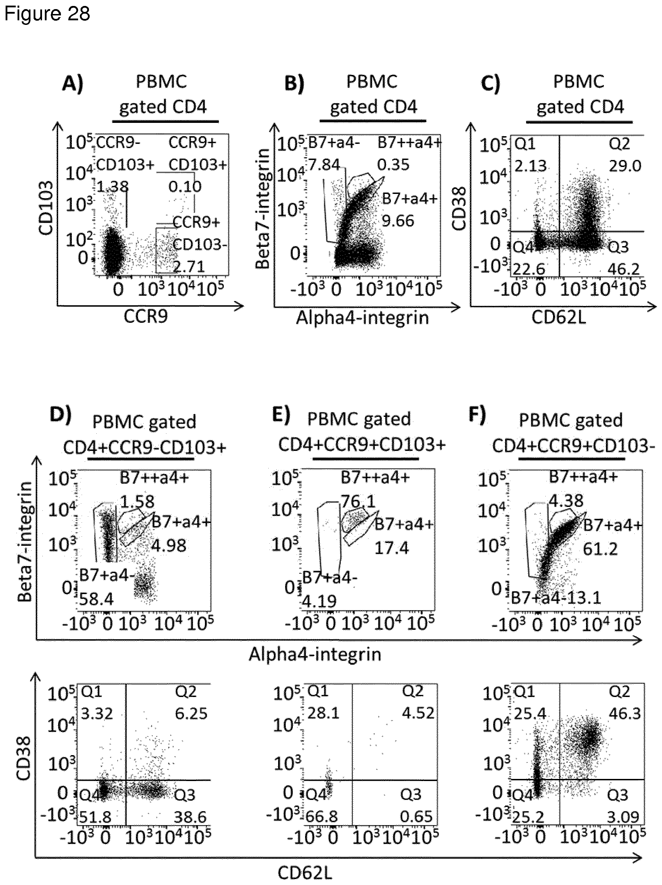

**Figure 28****. CD4⁺**α**4⁺β7^{high}CD103⁺CCR9⁺ T-cells in peripheral circulation contain an enriched proportion of CD38⁺CD62L⁻ mucosal emigrants..** PBMC recovered from healthy donor blood over ficoll were immediately labelled with indicated antibodies and analysed by flow cytometry. A) Total CD4 lymphocytes expressed as CD103 vs CCR9 dot plots. B) Total CD4 lymphocytes expressed as B7 vs a4 dot plots. C) Total CD4 lymphocytes expressed as CD38 vs CD62L dot plots. D) to F) gates of single and double positive CD103 and CCR9 cells in A) redisplayed as B7 vs a4 (top panels) and CD38 vs CD62L (bottom panels) dot plots.

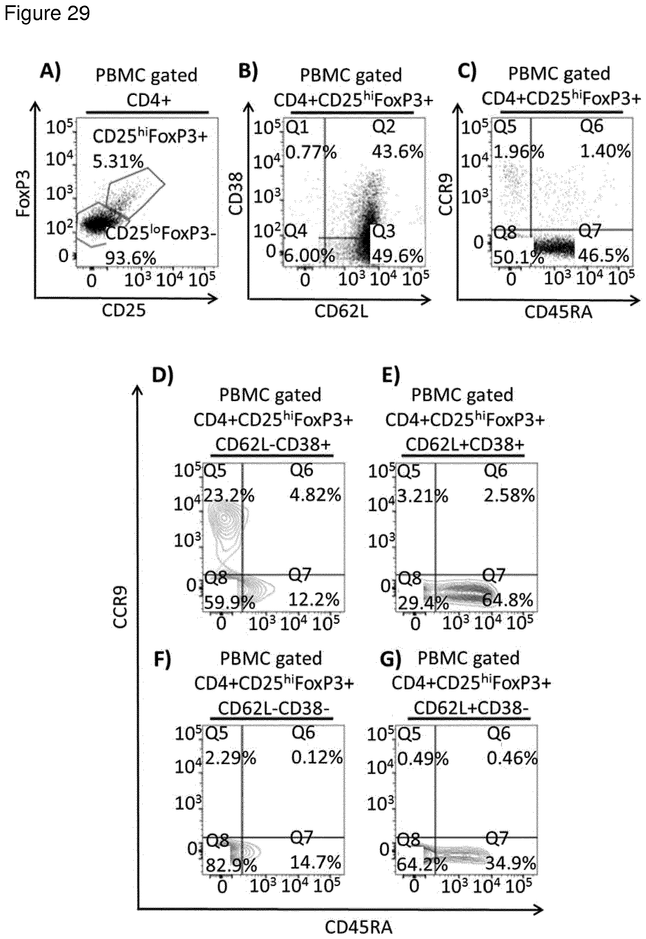

**Figure 29****. CD4⁺CD25^{hi}FoxP3⁺CD38⁺CD62L⁻CCR9⁺ T-cells do not express CD45RA.** PBMC recovered from healthy donor blood over ficoll were immediately labelled with indicated antibodies and analysed by flow cytometry. A) Total CD4+ lymphocytes expressed as FoxP3 vs CD25 dot plot. B) and C) display CD4⁺CD25^{hi}FoxP3⁺ cells as CD38 vs CD62L and CCR9 vs CD45RA dot plots, respectively. D) to G) display CCR9 vs CD45RA contour plots of gated populations from B) as indicated.

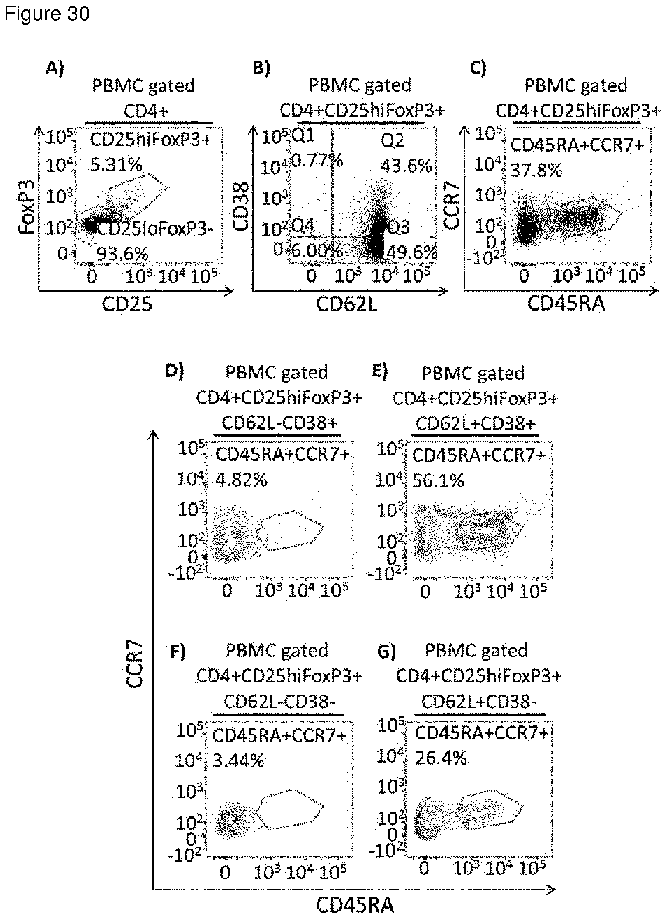

**Figure 30****. CD4⁺CD25^{hi}FoxP3⁺CD38⁺CD62L⁻ T-cells do not contain CD45RA/CCR7 double positives but CD4⁺CD25^{hi}FoxP3⁺CD62L⁺ T-cells are enriched for CD45RA/CCR7 double positive naive cells.** PBMC recovered from healthy donor blood over ficoll were immediately labelled with indicated antibodies and analysed by flow cytometry. A) Total CD4⁺ lymphocytes displayed as FoxP3 vs CD25 dot plot. B) and C) display CD4⁺CD25^{hi}FoxP3⁺ cells as CD38 vs CD62L and CCR7 vs CD45RA dot plots, respectively. D) to G) display CCR7 vs CD45RA contour plots of gated populations from B) as indicated.
**Figure 31****. T-cell migratory-type surface markers correlated with CD4⁺CD62L⁻CCR9⁺ mucosal T-cells.** PBMC recovered from healthy donor blood over ficoll were immediately labelled with indicated antibodies and analysed by flow cytometry in a high throughput screen. A) Total CD4⁺ lymphocytes displayed as CD62L vs CCR9 dot plot. B) displays CD4⁺CD62L⁻CCR9⁻ cells as SSC vs β7 contour plot. C) and D) display SSC vs CD195 contour plots of CD4⁺CD62L⁻CCR9⁻β7⁺ cells and CD4⁺CD62L⁻CCR9⁺ cells, respectively. E) Summary of ranked preferable migratory markers of X⁺/X⁻ condition for identification of small bowel tropic T-cells.
**Figure 32****. T-cell functional-type surface markers correlated with CD4⁺CD25^{hi}CD127^{lo}α4⁺β7⁺ mucosal T-cells.** PBMC recovered from healthy donor blood over ficoll were immediately labelled with indicated antibodies and analysed by flow cytometry in a high throughput screen. A) Total CD4⁺α4⁺β7⁺ lymphocytes displayed as CD127 vs CD25 dot plot. B) and C) show SSC vs CD39 contour plots of CD4⁺α4⁺β7⁺CD25^{lo}CD127^{hi} cells and CD4⁺α4⁺β7⁺CD25^{hi}CD127^{lo} cells, respectively. D) Summary of ranked preferable functional markers of Y⁺/Y⁻ condition for identification of regulatory mucosal T-cells. Markers noted with 'hi' in parenthesis indicate that the population with high expression of the indicated marker is of interest, indicating that both low and negative expression populations also exist.
**Figure 33****. Purification of a distinct subset of CD4⁺CD25^{hi}CD127^{lo}β7^{hi}CCR9⁺ Tregs from peripheral blood.** PBMC recovered from healthy donor blood over ficoll were immediately labelled with indicated antibodies and purified by fluorescent-activated cell sorting (FACS). Pseudo-color plots from A) to C) show lymphocytes gated from total PBMC in A) and subsequent sub-gates for single cells in B) and CD4+ cells in C). Plot D) redisplays total CD4+ lymphocytes as Beta7-integrin vs CCR9. The sub-population Beta7-integrin^{Hi} CCR9⁺ defined in plot D is re-displayed in plot E) as CD127 vs CD25 to define the sub-population CD25^{Hi} CD127^{lo} of T-regs. Plot F) and G) show the enrichment of the rare Beta7-integrin^{hi}CCR9⁺CD25^{hi} CD127^{lo} population of CD4 cells sorted according to the gating strategy outlined in plots A) to E) and re-analyzed by flow cytometry for the degree of sort purity. Panels H) and I) shows a parallel analyses of B7^{hi}CCR9⁺ cells with CD38 vs CD62L dotplots of panel I) being the daugthers of populations gated in panel H).

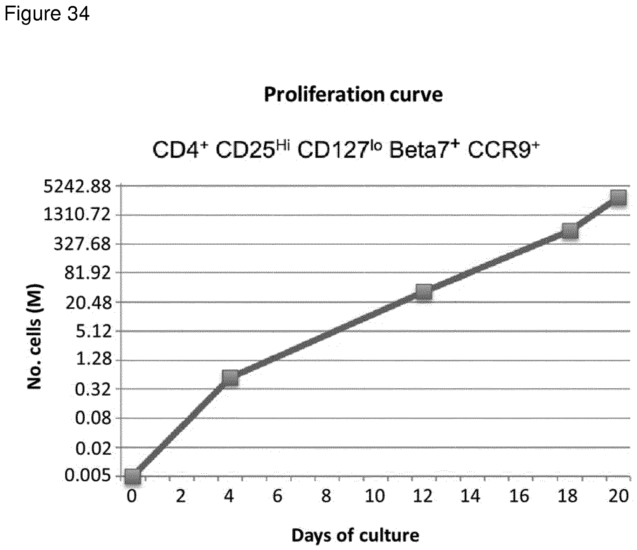

Figure 34. Expansion of sorted CD4⁺CD25^{hi}CD127^{lo}β7^{hi}CCR9⁺ Tregs in culture. The subset of CD4⁺CD25^{hi}CD127^{lo}β7^{hi}CCR9⁺ T-cell purified by FACS was cultured over several days. The proliferation curve displays the expansion of a starting pool of 5000 sorted CD4⁺CD25^{hi}CD127^{lo} CCR9⁺ Beta7^{Hi} T-cell reaching almost 3 billons cells over 20 days of culture.

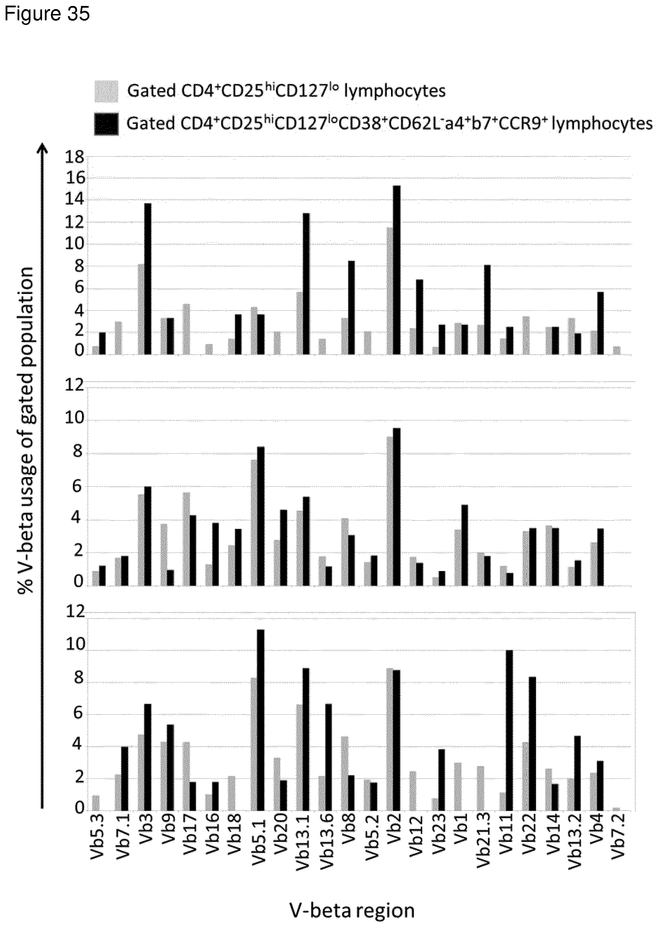

**Figure 35****. Peripheral CD4⁺CD25^{hi}CD127^{lo}CD62L⁻CD38⁺α4⁺β7⁺CCR9⁺ T-cells have skewed Vβ usage compared to CD4⁺CD25^{hi}CD127^{lo} cells.** PBMC recovered from blood of three healthy donors over ficoll were immediately labelled with CD4, CD25, CD127, CD38, CD62L, CD49d, β7 and CCR9 antibodies in addition to panels of Vβ-specific antibodies and analysed by flow cytometry. Coverage of donor C12 (top panel) was CD4⁺CD25^{hi}CD127^{lo} 75% and CD4⁺CD25^{hi}CD127^{lo}CD62L⁻CD38⁺a4⁺β7⁺CCR9⁺ 95%. Coverage of donor C6 (middle panel) was CD4⁺CD25^{hi}CD127^{lo} 72% and CD4⁺CD25^{hi}CD127^{lo}CD62L⁻CD38⁺α4⁺β7⁺CCR9⁺ 77%. Coverage of donor C26 (bottom panel) was CD4⁺CD25^{hi}CD127^{lo} 77% and CD4⁺CD25^{hi}CD127^{lo}CD62L⁻CD38⁺α4⁺β7⁺CCR9⁺ 93%.
**Figure 36****. Investigational model for identification of diseased tissue-specific Treg.** Model of investigational elements for identification of Treg cells originating from tissue Target (A), migrating through lymphatics (B', B and B"), and subsequently migrating through peripheral blood (C) to recirculate to tissue of origin (A). Alternate route of recirculation is to a secondary tissue of interest (X). Tissues A, B, B', B", C and X are considered major sampling points for investigation. Major migratory processes involving recirculation of Tregs back to tissue of origin are defined for target tissue emigration (α), and immigration (β). Emigration from secondary tissue of interest (X) is defined as αX, and immigration βX. Migration between main target tissue A, and communicating tissue X, is defined as migration process ε. Migration processes α, β, αX, βX and ε are considered as definable by Treg migratory marker expression, as applied to Tregs recovered from sampled tissues A, B, B', B", C and X. Major sources of analytical noise are those cells collected in all compartments that bare migratory marker expression for migratory processes γ and δ. γ and δ migratory process markers are considered negative selection criteria for investigation of Treg cells of A or X origin, with α, β, αX, βX or ε migratory behaviour.
**Figure 37****. CD103⁺ and/or CCR9⁺ CD4⁺ T-cells are present in large bowel LP at low frequency compared to small bowel LP.** Single cell suspensions prepared from indicated resected tissues of a representative CD patient with ileocaecal disease were immediately stained with the indicated antibodies. Plots display CD103 vs CCR9 of CD4⁺ gated cells.

Based on experiments, the inventors have made the following observations:
CD4 Tregs in the human peripheral blood may be identified and analytically and/or physically enriched through small-bowel tropic cell surface marker sets, and these CD4 Treg cells are strongly diminished in numbers within the inflamed tissues of CD patients. In additional the mechanistic basis of this immunological defect in CD patients is proposed to embody a numerical deficiency in CD103⁺ DC in inflamed tissues of CD patients. Mucosal emigrant and mucosal tropic Tregs as defined by the presented marker sets are considered as therapeutic candidates for the management of CD and other IBDs. Cells of the various identified compositions can be non-invasively recovered from peripheral blood preparations an expanded in vitro. Preparing targeted Treg subpopulations in this manner is proposed to restrict TCR clonal diversity to clonotypes specific for tissue-associated antigens. This supported by a skewed Vβ usage within the mucosal CD4+ Treg populations observed in peripheral circulation, when compared to non-mucosal Treg populations. These findings yield a practical method for prospecting any diseased target tissue, though particularly mucosal and skin tissues, to identify cells that are emigrant from said diseased tissue. Preparing targeted Treg subpopulations in this manner is proposed to restrict TCR clonal diversity to clonotypes specific for tissue-associated antigens, making these Treg fractions most suitable for starting materials for manufacture of cellular immunotherapies to treat inflammatory and autoimmune disorders of any tissue under investigation. This method also allows consideration of primary diseased tissue and the establishment of secondary inflammatory and autoimmune conditions in tissue with migratory lymphocyte communication, and offers a framework that would allow treatment of a disease in such a communicating tissue through treatment of the primary diseased tissue.

Firstly the inventors addressed whether CD4⁺FOXP3⁺T_{regs} in the human gut displayed higher CCR9 expression than CD4⁺FOXP3⁻ counterparts. **Figure 1A** shows flow cytometry analyses of single cell suspensions prepared from relatively proximal healthy tissue of the small intestine of a representative CD patient. CD4⁺FOXP3⁺ cells show greater CCR9 expression relative to CD4⁺FOXP3⁻ Teffs in both small and large bowel LP (mucosal lamina propria). CCR9 marking of CD4⁺FOXP3⁺ cells is greater overall in the small bowel LP, consistent with the regional differences in CCR9 marking reported previously (Papadakis et al. Gastroenterology 2001, 121, pp 246-254). The CD4⁺FOXP3⁺ population also carried a greater proportion of cells with integrin β7^{hi} phenotype, particularly in the small bowel LP. Cells with a CD4⁺FOXP3⁺β7^{hi} phenotype almost exclusively expressed CCR9 (**Figure 1B**). The preferential marking of CD4⁺FOXP3⁺ cells was also reflected in the mesenteric lymph nodes (MLNs) draining the small bowel (**figure 2**). Tregs more stringently gated on CD4⁺CD127^{lo}CD25^{hi}FOXP3⁺ phenotype(9), similarly have higher CCR9 expression than Teffs with CD4⁺CD127^{hi}CD25^{lo}FOXP3⁻ phenotype in small bowel MLN (**figure 3**); and in peripheral blood after further analytical enrichment by gating on β7⁺ (**figure 4**). These results are consistent with a stronger CCR9-dependent small-bowel tropism of Tregs compared to Teff counterparts from human gut. Moreover, direct parallels can be drawn with recent results regarding CCR9 expression on mouse T-cells, and the CCR9-dependency of oral tolerance (Wermers et al. Gastroenterology 2011, 140 (5), pp. 526-1535, and Cassani et al. Gastroenterology 2011,141, pp 2109-2118).

These observations reveal some interesting features of the co-expression of markers in the small and large bowel mucosal lamina propria (LP), and by association the MLN draining these tissues. Specifically, the presence of a β7-high population dominantly in small bowel is suggestive of the presence of and additional integrin pairing within this small bowel population. At the time it was speculated that this could be the αEβ7 pairing, which is addressed and confirmed herein.

Strikingly, the β7-high population in LP has the almost exclusive CCR9 marking in the healthy tissue analysed here. Thus β7-high and its integrin co-markers represent a proxy CCR9 marker at least in these tissues. This has implications for the understanding of fundamental migration mechanisms of T-cells in humans, but also provides analytical and therapeutic possibilities as mentioned herein.

### Mucosal-educated FOXP3+ Tregs in peripheral circulation carry higher CCR9 marking than FOXP3⁻ Teffs and CD patients have diminished overall CCR9 marking on mucosal-educated T-cells

The inventors excluded that preferential CCR9 marking was only occurring on resident Tregs in the LP and MLN, by investigating CCR9 expression of both mucosal-educated and mucosal-tropic Tregs in peripheral blood. A CD38⁺CD62L⁻ phenotype was used to enrich T-cells educated within the intestinal mucosa in flow cytometric analysis of peripheral blood mononuclear cells (PBMCs) from healthy controls (HC) and CD patients with small bowel disease. Observations confirmed the enrichment of β7 and CCR9 marking on CD38⁺CD62L⁻ T-cells, and showed that the CD38⁺CD62L⁻β7⁺ subpopulation is further enriched for CCR9 positivity (**figure 5A**). Furthermore, T-cells of CD38⁺CD62L⁻ phenotype predominate in both the human small and large bowel LP **(****figure 6**). CD4⁺FOXP3⁺ Treg numbers in circulation are not significantly diminished in CD patients compared to healthy controls as previously reported, although trend in this direction (**Figure 5B**). There is no significant difference in the total number of mucosal-educated CD4⁺CD38⁺CD62L⁻ Tregs between CD patients and healthy controls, while overall CCR9 positivity in this sub-population is strikingly reduced in CD patients (**Figure 5C**)**.** Preferential CCR9 marking of CD4⁺FOXP3⁺ T_{regs} over CD4⁺FOXP3⁻ effectors is observed in CD38⁺CD62L⁻β7⁺ mucosal emigrants present in peripheral circulation of healthy controls (HC). This preferential CCR9 marking of Tregs is maintained in CD patients, however the overall numbers of CCR9⁺ Tregs and non-Tregs are diminished in the mucosal-educated sub-population relative to healthy controls (**figure 5D**)**.**

There are two intriguing aspects to this directed analysis of peripheral recent mucosal emigrants. The overall numbers of Tregs in peripheral circulation is trending towards a deficiency in CD compared to healthy controls. However, when we look only at the recent emigrants that are also likely destined for recirculation to mucosa (CD38⁺CD62L⁻β7⁺), then we actually observe a trend towards increased numbers of Tregs. In addition these recent emigrant and recirculating T-cells have a marked reduction in CCR9 expression. Taken together, these data suggest that CD is not a disease defined by a deficiency of Tregs per se, but a deficiency in their ability to recirculate to the small bowel. This could also be construed as a strong retention of CCR9-carrying T-cells in the inflamed bowel of CD patients.

It is possible that the Treg population under the strong inflammatory conditions in the inflamed lamina propria is undergoing atrophy. The general inflammatory state would also draw more naive Tregs from peripheral circulation, decreasing the apparent incidence of total Tregs in the periphery. It appears that T-cells can still be exported from the inflamed mucosa-associated lymphoid tissues (i.e. MLN), though lack the important chemokine receptor trigger (CCR9) to facilitate re-import to the inflamed mucosa.

### CD4⁺α4⁺β7⁺ mucosal-tropic FOXP3⁺ Tregs in peripheral circulation carry higher CCR9 marking than FOXP3⁻ T_{effs} and CD patients have diminished CCR9 marking on mucosal-tropic T-cells

In addition to investigating CCR9 expression on mucosal-educated T-cells having emigrated to peripheral circulation, the total pool of mucosal-tropic T-cells in peripheral blood was examined through analytical enrichment of α4⁺β7⁺ expressing CD4⁺ T-cells. We show that T-cells with this α4⁺β7⁺ phenotype in peripheral circulation are highly enriched for CCR9 positivity (**Figure 7A-B**); indeed on average greater than 75% of all CCR9⁺ cells in peripheral circulation carry α4⁺β7⁺ expression (**figure 8**). Surprisingly, even though the total number of CD4⁺α4⁺β7⁺T-cells is similar in healthy controls and CD patients (**Figure 9**), CD4⁺FOXP3⁺ Treg numbers are relatively increased in this mucosal-tropic subpopulation in the peripheral blood of CD patients (**figure 7C**). The CD4⁺FOXP3⁺ Tregs in the peripheral mucosal-tropic population carry significantly higher CCR9 marking when compared to CD4⁺FOXP3⁻ Teffs in both healthy controls and CD patients, while overall CCR9 positivity is diminished in CD patients (**figure 7D**). These results strongly suggest that there are relatively more Tregs in the peripheral circulation of CD patients with the capacity to traffic to mucosal tissues, but these mucosal-tropic cells lack small-bowel specificity due to reduced levels of CCR9 expression. Explanations may be either a defect in CCR9 imprinting in the LP/MLN of CD patients, or a strong migration of CCR9-expressing T-cell subclasses to the inflamed small bowel mucosa in CD patients.

This data for the total pool of mucosal-tropic T-cells is in agreement with the data above regarding recent mucosal emigrants, revealing a striking defect in CCR9 expression on Tregs from CD patients. More interestingly, while the total numbers of mucosal-tropic T-cells are similar, the percentage of Tregs is actually higher. This is in contrast with the proportion of Tregs among all CD4 cells in circulation, and to the concept that CD is a disease defined by a deficiency in Treg populations. This data further supports the notion that CD is defined by Tregs being unable to recirculate to the small bowel mucosa - and indeed there is an apparent excess of mucosal-targeted Tregs available.

### Treg Purification

Two different purification strategies were used to purify Treg populations from mesenteric lymph nodes of patients, a full MACS enrichment, or a MACS pre-enrichment followed by FACS purification. The recovered populations are highly enriched in cells with the desired characteristics. That is, of character CD4⁺CD25^{hi}CD127^{lo}CD62L⁻CD38⁺α4⁺β7⁺CCR9⁺, simply owing to their MLN origin (see figures 1, 2, 3 and 6). Both of these enrichment and purification types were used to study Treg cell growth, homing receptor pattern stability and dynamics, and functionality, ex vivo. The enrichment/purification methods used in the present context are for illustrative purposes due to limiting yields for larger scale preparative experiments from peripheral blood. Purification of target populations from peripheral blood is presented below. Other suitable methods enrichment and purification may also be applied.

### MACS 2-step enrichment of Tregs from MLN

For research-graded preparations of Tregs from MLN and other tissues Miltenyi MACS approaches have primarily been used. **Figure 10** presents purification data using a Regulatory T Cell Isolation Kit II and an autoMACSpro instrument. This procedure uses a negative affinity selection on non-CD4-expressing and CD127-expressing cells. A subsequent positive affinity selection purifies the final CD4⁺CD127^{lo}CD25^{hi} population based on CD25 labelling.

### MACS enrichment and FACS purification of Tregs from MLN

A second means of Treg purification from MLN material is a 2-step approach using Miltenyi MACS enrichment of total CD4+ cells, then FACS purification on various parameters including CD25 and CD127 (**Figure 11**). The pre-enrichment was routinely conducted due to limitations in both FACS instrumentation and biological reagent resources at the time this work was conducted.

### Treg Expansion

The expansion of Tcells ex vivo utilises standardised methods that rely on stimulation of three core T-cell activating inputs. The minimal signals that a Tcell requires to activate and proliferate are stimulation of the TCR (aka CD3), co-stimulation via CD28 receptor, and a secondary mitogenic stimulation via Interleukin-2 receptor (IL2R). Standardized approaches use antibodies to stimulate both CD3 and CD28. Recombinant IL2 is used for the mitogenic input. Various modifications of the general method are used with regard to the vehicle, dosage and duration of CD3 and CD28 stimulation, in addition to the subsequent IL2 input. A general method is described in detail in the methods section, but it is believed that the T-cells can be expanded by generic methods. **Figure 12** shows typical ex vivo expansion curve of MACS-purified Tregs from patient MLN, where approximately 120- to 150- fold population expansion is achieved over 18 days of culture. Rapamycin was included in selected cultures to assess impact on Treg growth and culture stability. Rapamycin is routinely used to limit the growth of Teff subclasses in human Treg cultures, and thus polarise to a Treg phenotype over time. Three different Rapamycin dosing strategies were used here, where it was present for the entire 18 days, or just the first 8 or 15 days of expansion. In our hands Rapamycin strongly inhibited Treg growth (**Figure 12**), and did not promote Treg characteristic stability (**Figure 13**). The reason for this may be the MLN source of the cells, where we expect a bulk to be induced/experience Tregs, and not naïve Treg precursors isolated from peripheral circulation as investigated by others. In addition to the basic CD25-FOXP3 parameters presented in **Figure 13**, we monitored CD127 levels, which mirrored the CD25-FOXP3 population. Moreover, we expanded and manipulated Teff populations in parallel to ensure accurate comparison of what were likely to be stable Treg cells (not shown). These complimentary analyses suggest that the CD25-FOXP3 expression was indeed indicative of a true Treg phenotype, and not a sole result of strong activating stimuli.

Despite a natural enrichment for cells of mucosal emigrant and mucosal-tropic characteristics in our MACS-enriched Treg cultures (**refer** **figures 2 to 8**), we found that after 18 days of expansion the resultant T-regs were almost devoid of small-bowel tropic homing receptor expression (**Figure 14**). In vivo, these receptors are imprinted by tolerogenic stimuli provided directly by DCs. In the absence of such inputs to maintain receptor expression, it is clear why we observe little or no receptor expression after 18 days of culture

This absence of gut-tropic homing receptors presents a caveat for our method of therapy. Namely, we select the Treg populations based on their inherent homing characteristics, however, once expanded in vitro by generic methods, they lack the qualities required to traffic back to the diseased bowel on re-infusion to the patient. It has been established, mainly using mouse tissues, that T-cells can be forced to express small-bowel homing receptors including α4-and β7- intergrins, and CCR9.

In the following a simple set of observations outlines the considerations and method behind homing receptor 'repatterning' after expansion.

### Treg Receptor Re-patterning

To determine the culture parameters required for receptor repatterning on ex vivo expanded Tregs, we first used short term (4 days) growth of Treg cells in the presence of absence of the tolerogenic stimuli ATRA (all-trans retinoic acid, also referred to as RA in some figures), and transforming growth factor-beta (TGF or TGF-beta). In addition, we assessed the impact of Rapamycin on the expression of homing receptors of the short-term, and on the tolerogenic induction of homing recptors.

**Figure 15a** shows 4-day expansions of MACS-enriched and FACS-purified Tregs in the presence of the indicated stimuli. The top left panel shows cells activated and grown in a generic manner without further stimulation. These cells retain significant levels of β7- intergrin and CCR9 co-expression. Strikingly, the addition of Rapamycin (RAPA) strongly attenuates the expression of β7- intergrin and CCR9 (bottom left panel). Addition of TGF/ATRA to these cultures strongly induces β7- intergrin, but not CCR9 (top right). Rapamycin attenuates CCR9 expression, but not β7- intergrin (bottom right).

**Figure 15b** demonstrates that increasing concentrations of TGF on a background of ATRA stimulation can almost completely polarise Tregs towards a β7⁺CCR9⁺ phenotype. Rapamycin addition almost completely abrogates CCR9 positivity in this system.

These experiments establish the basic parameters of receptor repatterning, in as much that we now appreciate that any such approach should be done in the absence or Rapamycin, and with minimal ATRA and TGF stimuli. **Figure 16** shows day-18 expanded MACS-enriched Tregs that have been stimulated for a further 6 days with combinations of three tolerogenic stimuli. These stimuli were part of a much larger titration, and are presented in this focused context for clarity. **Figure 16a** shows that the lack of α4β7 expression on Tregs after 18-days of expansion (**refer** **Figure 14**) persists after 6 further days of culture without additional stimuli. ATRA alone is sufficient to induce α4β7 expression in a dose-dependent manner (**figure 16c****,d**). IL10 is sufficient to weakly induce β7- but not α4- integrin expression (**figure 16e**), an effect that appears to be anergised by TGF (**figure 16g**). The most effective stimuli for α4β7 induction were found to be low doses ATRA in combination with IL10, or IL10 and TGF (**figure 16h****, i**).

The expression of CCR9 on these stimulated cells was further assessed in parallel, which is displayed against β7 expression in **figure 17****.** Several important interactions of the stimuli can be observed when considered in context with the above α4β7 data. First, while TGF alone is insufficient to induce α4β7 expression (**figure 16b**), it is a moderately effective inducer of CCR9 expression at the dosage presented (**figure 17b****).** While IL10 is a strong inducer of CCR9 expression ((**figure 17e**), TGF and IL10 appear to have an anergistic effect when provided as co-stimuli (**figure 17g**). ATRA alone is a moderate inducer of CCR9 expression (**figure 17c****,d**), an effect that may be marginally enhanced in combination with IL10 (**figure 17h**)**.** However, all three tolerogenic stimuli in combination have a strong polarising effect on CCR9 expression, while maintaining a correct α4β7 pattern (**figure 17i**) and (**figure 16i**).

Overall, conditions were established with which to 'repattern' the correct α4⁺β7⁺CCR9⁺ homing characteristics on ex vivo expanded Tregs. A combination of low-dose ATRA, TGF and IL10 is sufficient to achieve correct homing receptor patterns with acceptable efficiency.

### Treg Functionality

The standard manner in which to test the immunosuppressive functionality of Tregs in vitro is a mixed culture assay. Part of Treg immunosuppressive function is to suppress Teff cell division, we thus measure the degree of Teff cell division in the presence of Treg cells. In our setup we used partially purified Teffs from MLN to test the functionality of ex vivo expanded Tregs from the same patient. Teffs are labeled with carboxyfluorescein succinimidyl ester (CFSE), which is a strongly fluorescent compound that is taken up by the Teff cells. Rapid crossing of the plasma membrane is facilitated by the succinimidyl group, which is subsequently cleaved by intrinsic cellular esterase activity, ensuring retention of the fluorescent carboxyfluorescein in the cell. On cell division, the diffuse carboxyfluorescein is partitioned approximately equally between the two resulting cells. Therefore, one can monitor the cell division of Teff cells in vitro in the presence of (unlabeled) Tregs by monitoring step-wise decrease in Teff fluorescence intensity.

**Figure 18** shows the suppression of freshly purified Teff cells by 18-day expanded autologous Tregs. This demonstrates the suppressive capacity of expanded Treg cells in this system.

### Mucosal emigrant and homing markers on small bowel Tregs in inflamed CD patient tissues

The experimental data presented above regarding purification and expansion of Tregs utilised disease-draining SLN as Treg source material. It is reasoned that SLN will be highly enriched in Tregs with relevant TCR clonotypes, considering their physical disease and/or tissue association. Moreover, SLN and MLN in general will be highly enriched for the tropic and emigrant populations that are of general interest for therapeutic purposes, with their observed deficiencies in peripheral blood. While the SLN are indeed highly enriched for these populations when compared to the peripheral blood compartment, close analysis of patient material provides further insights into the observed peripheral deficiency of CCR9-expressing Tregs.

**Figure 19** shows the contour plots of CD38 vs CD62L of total CD4+FOXP3-CD25lo Teffs (left panels) and CD4+FOXP3+CD25hi Tregs (right panels) recovered from resected tissues of a representative CD patient with ileocaecal disease. Normal MLN in the surgical field, two SLN (1 and 2), inflamed lamina propria (LP) and normal LP is presented from top to bottom. When observing both Tregs and Teffs from normal MLN one can see the expected CD38 positivity and CD62L negativity that correlates with mucosal emigrant populations. This population is more strongly represented in the Treg cells. In both SLNs analysed, the proportion of CD62L+ cells, that is likely to represent direct MLN immigrants from peripheral circulation, is notably increased in both Treg and Teff populations. This may either be due to increased direct immigration to the SLNs compared to healthy MLN, or relatively diminished trafficking of cells from the LP to the SLN. In the four bottommost panels we can observe significant CD62L+ cell numbers in the inflamed LP, but not adjacent healthy tissue. Again, this could reflect a strong and aberrant immigration of cells from circulation, and/or a poor patterning of correct receptors of the T-cells within the LP.

To test whether correct patterning was being achieved on T-cells in inflamed and adjacent healthy CD tissue, CD103 and CCR9 expression was assessed. **Figure 20** shows CD103 vs CCR9 contour plots of total CD4+FOXP3-CD25lo Teffs (left panels) and CD4+FOXP3+CD25hi Tregs (right panels) recovered from resected tissues of a representative CD patient with ileocaecal disease from tissues as above. In normal MLN one can immediately observe the fundamental difference between Teffs (left) and Tregs (right), in that Tregs in the MLN carry very high levels of CD103 and CCR9 and Teffs do not. This could indicate that Tregs are more attuned to trafficking from LP to MLN, and/or they are more likely to be patterned in this way by MLN DCs. The latter is consistent with the tolerogenic function of CD103+ DCs that pattern both CCR9 and CD103 expression. The second striking contrast that may be observed here is the complete loss of both CD103 and CCR9 expression on cells in disease-draining SLN. Moreover, a similar though less dramatic decrease in CD103 and CCR9 expression can be observed in the inflamed LP compared to the healthy tissue. Interestingly, it is the Teff cells that are most strongly expressing CD103 and CCR9 in the normal LP, not the Tregs as in the normal MLN. This may indicate that Teffs are more likely to be retained in the LP than the Tregs, and thus express high levels of the retention integrin CD103. This is consistent overall with the balance of Tregs and Teffs in these two compartments.

To ensure that the observed loss of CD103 and CCR9 expression was not simply due to the dilution of these cells by immigrants from circulation (see **figure 19**), CD38⁺CD62L⁻ Tregs were analysed in the same compartments as **Figure 20****.** **Figure 21** shows a similar analysis of Tregs as in **Figure 20**, though a further analytical enrichment of CD38⁺CD62L⁻ was used. These data demonstrate that there appears to be a deficiency in CD103 and CCR9 expression on Tregs in the inflamed tissues, despite expression of a mucosal phenotype (CD38⁺CD62L⁻). One can also note the relative enrichment in the expression of CD103 and CCR9 (**Figure 21**) when compared to the right hand panels of **figure 20****.**

The data presented above collectively suggest a defect in the CD103 and CCR9 patterning of T-cells within inflamed tissues of CD patients. The DC subset that is responsible for patterning this receptor expression on T-cells are known to be a CD103+ DC subset. The possibility of a numerical deficiency in this CD103+ DC population was tested as a possible cause of CCR9 and CD103 deficiency. **Figure 22** shows analysis of CD45⁺CD11c^{hi}CD80⁺HLA-DR^{hi}CD103⁺ DC in the healthy MLN and disease-draining SLN of a CD patient. Strikingly, there is huge numerical deficiency in the CD103+ subset of HLA-DR^{hi} DC cells in the SLN. Sufficient cell numbers could not be recovered for a reliable analysis of inflamed and normal LP from this patient. However, limited analyses show a similar trend (not shown).

### Identification of mucosal emigrant and small bowel tropic Tregs in peripheral blood

Aside from the obvious practical limitations of harvesting Tcell material from SLN for therapeutic applications, and the strong CCR9 expression defect observed in some patients even within inflamed tissue, the recovery of mucosal emigrant and small bowel tropic Tcells (Tregs?) directly from peripheral blood is attractive. In figures 5 and 7, these populations were treated somewhat separately. While mucosal emigrants were defined as CD38⁺CD62L⁻ β7⁺, this does not fully embody a fully pure candidate for sorting of this target population from peripheral blood. **Figure 23a** shows the full gating strategy used for analytical identification of mucosal emigrant and small bowel tropic Tcells in peripheral blood. Full enrichment is achieved by the addition of alpha4-integrin to the staining panel, allowing for exclusion of β7⁺ cells that are α4 negative, a major contaminant using just β7+ criteria. An antibody for staining of a shared α4β7 epitope was not available for this study, though the two dimensional plot of these parameters reveals novel relationships as described in subsequent sections. **Figure 23b** and **23c** show the staining of total PBMCs for the enriched parameters, demonstrating the strong enrichment of desired cell populations.

**Figure 24** displays the use of the gating strategy to analytically enrich Teffs and Tregs for comparison. Here FOXP3 vs CD25 is used to identify fixed cells. Exchanging FOXP3 for CD127 can be used to target viable cells. The very high enrichment of the target α4⁺β7⁺CCR9⁺ small bowel tropic population can be seen within the total CD4 T-cell population (refer **figure 23**), and compared to the Teff population.

### Addition of CD103 mucosal retention marker identifies peripheral T-cell subsets

In the above data was presented that indicated two distinct populations of T-cells with regard to their positive expression of β7-integrin, a β7⁺ and a β7^{hi} population. It was shown that the β7^{hi} population in both the small and large bowel LP displayed almost 100% CCR9 positivity, in healthy tissue. The quantitative difference in β7 expression is clearly due to the co-expression of a second integrin pair, in this case αE (CD103). The majority of β7⁺ cells were presumed to be cells expressing solely the α4β7 pair, while β7^{hi} cells express higher levels of β7 owing to the fact that they require additional β7 to pair with αE, suggesting β7^{hi} cells express both the α4β7 and αEβ7 integrin pairs. The significance of this is that α4β7 is thought to be required for migration into mucosal tissues, while αEβ7 is required for retention.

Given that αEβ7 is regarded as a retention marker for mucosal T-cells, it is obvious why we observe high levels β7^{hi} cells in mucosal tissues. What was not clear is why we observed a significantly greater proportion of β7^{hi} cells in the small bowel, when compared to the large bowel (**Figure 1**). An explanation comes from co-expression of CCR9 on β7^{hi} cells. It is known that both αE and CCR9 are strongly induced by a similar set of stimuli, including ATRA and TGFbeta, which are likely to be provided by CD103⁺ DCs in the LP and MLN environments. Cells recovered from the bowel LP thus represent cells that are being environmentally imprinted with α4β7 and αEβ7 integrin pairs and CCR9. Interestingly, CD103 is often cited as being of higher expression on CD4 Tregs, and has been proposed to be a defining marker of a subset of CD8 Tregs. This relationship was investigated in peripheral blood. Although CD103/αE is considered a mucosal retention marker, one does indeed observe CD103 positive cells in the peripheral blood. This is because the retention of αEβ7-expressing cells within mucosal tissue is likely to be directed by short-range homing. That is, cells that emigrate from the local mucosal tissues into blood stream will dominantly and selectively re-enter mucosa when expressing αEβ7 due to binding of cognate receptors (E-cadherin) on local HEVs.

**Figure 25a** presents an analysis of blood from a healthy donor where CD4 cells are gated and displayed as β7 vs α4 dotplots, as in **Figures 7** **and** **8**. Our expectation is that CD4 cells with a α4⁺β7^{hi} phenotype will be highly enriched for CD103, and naturally CCR9 as a strongly co-expressed marker (**note, figures designate β7^{hi} as B7++**). Cells within gates presented in **Figure 25a** are displayed as CD103 vs CCR9 contour plots in **figure 25b to figure 25e****.** As anticipated, the α4⁺β7^{hi} population is highly enriched for CD103, with some 80% of all cells expressing CD103. This population is also highly enriched for CCR9 expression (**figure 25b**).

To confirm and expand the relationship between CD103 expression and the expression of α4 and β7- integrins, one can treat the same data in a differing manner. **Figure 26a** simply shows gated CD4 cells as a CD4 vs CD103 dotplot. From here, total gated CD103- and CD103+ cells are displayed a β7 vs α4 dotplots in **figure 26b and 26c** respectively. The negative population appears as a standard pool of CD4 T-cells with regard to β7 and α4 expression, although strikingly lack the expression of a α4⁺β7^{hi} population (**figure 26b**). In contrast the CD103+ population is highly enriched for the α4⁺β7^{hi} (**figure 26bc**, **and compare** **figure 25a**). We can also observe the enrichment of cells of another rare population, those that carry β7 expression, but lack α4. The tissue origin of these cells that likely express the αEβ7 pair in the absence of α4β7, is unclear.

Finally, this data can be used to visualise the quite clear expression of CD103 on the β7^{hi} population (**figure 27a**). This CD4⁺β7^{hi}CD103⁺ population is highly enriched for α4⁺CCR9⁺ cells (**Figure 27b and 27c**).

Overall, these simple analyses demonstrate an analytical enrichment strategy for identifying CD4⁺α4⁺β7^{hi}αE⁺CCR9⁺ cells in the peripheral blood. These cells are likely to represent mucosal emigrants with a very strong propensity to recirculate to the small bowel. Therefore one could predict the CD4⁺α4⁺β7^{hi}αE⁺CCR9⁺ population to be highly represented in the CD38⁺CD62L⁻ population. This is confirmed in the analyses presented in **figure 28****.** Panels B) and C) display the now familiar parameters of total CD4 T-cells in a β7 vs α4 dotplot and CD38 vs CD62L dotplot, respectively, while panel A) displays CD103 vs CCR9 dotplot of total CD4 T-cells. Single positive CD103 cells are re-displayed in panel D), CD103 CCR9 double positives in panel E), and CCR9 single positives in panel F). These populations reveal the expected α4 and β7 staining consistent with above analyses. Both of the CD103 CCR9 double positive and CCR9 single positives show an enrichment of the CD38⁺CD62L⁻ mucosal emigrant population. CCR9 single positives are also enriched for CD38⁺CD62L⁺, and are very likely to represent recent thymic emigrants.

In summary, the preliminary identification of CD4⁺α4⁺β7^{hi}αE⁺CCR9⁺ population in peripheral blood, which is likely to represent mucosal emigrants with a strong propensity to recirculate to the small bowel, presents a further means to identify Treg cells based on homing receptor patterns for adoptive immunotherapy. Coupled to Treg markers and the CD38CD62L marker sets, we are able to identify the signatures described with therapeutic potential in Crohn's disease, in particular the following two overlapping subsets of Tregs.
1) CD4⁺CD25^{hi}CD127^{lo}α4⁺β7^{high}αE⁺CCR9⁺
2) CD4⁺CD25^{hi}CD127^{lo}CD62L⁻CD38⁺α4+β7^{high}αE⁺CCR9⁺

The significance of the CD103+ CD4 Treg population is underscored by the recent work defining the role of CCR9 in establishment of oral tolerance. A new theory suggests that dominant recirculation of iTregs from the LP back to the LP is required for establishment of oral tolerance in a CCR9-dependent manner. Thus, CD4⁺CD25^{hi}CD127^{lo}α4⁺β7^{high}αE⁺CCR9⁺ Tregs could represent a Treg population that makes a major contribution to intestinal homeostasis, despite their low numbers in the periphery.

To further confirm both thymic emigrant nature of CD4⁺CD38⁺CD62L⁺ cells, and indeed the expected antigen-experienced nature of CD4⁺CD38⁺CD62L⁻ cells, the expression of CCR7 and CD45RA was analysed on these subpopulations. Firstly, FOXP3⁺ Tregs gated for CD38⁺CD62L⁺ were most highly enriched fro CD45RA expression (**Figure 29e**), supporting their enrichment ofr naive cells. In contrast, CD38⁺CD62L⁻ cells expressed little CD45RA, and were enriched fro CCR9 expression (**Figure 29d**). The recent thymic emigrant nature of CD4⁺CD25^{hi}CD127^{lo}CD38⁺CD62L⁺CCR9⁺cells was further confirmed by the high enrichment of CCR7 expression within this population (**Figure 30e**).

In order to more define the recent mucosal emigrant population of Treg cells of the small bowel, a high throughput screen was conducted using CD4⁺CD62L⁻CCR9⁺ as the test population (small bowel emigrant and tropic) and CD4⁺CD62L⁻CCR9⁻β7⁺ as the generally mucosal-tropic reference population. **Figure 31** shows an example of different adhesion molecule expression in the CD4⁺CD62L⁻CCR9⁺ population in comparison to the CD4⁺CD62L⁻CCR9⁻β7⁺ population that is targeted to mucosal tissues in general (**Figure 31** **A to D**). In this example, CD195 (CCR5) is almost absent in the CD4⁺CD62L⁻CCR9⁺ population. It is thus anticipated that CD195 may be used as a marker of preferred condition X-, with which to select for mucosal emigrant, immigrant and educated CD4+ Treg cells with small bowel tropism. The table presented in **Figure 31** **E** summarises other migratory-type markers associated with the CD4⁺CD62L⁻CCR9⁺ population. The markers positively correlated are of condition X+ and the markers negatively correlated are of condition X-. In the preferred aspect markers denoted X+ are used as a positive selection marker and markers denoted X- are used as a negative selection marker for the purification of mucosal emigrant, immigrant and educated CD4+ Treg cells with small bowel tropism. Each marker in this table is also assigned a class, where class 1 represents a strong association with the CD4⁺CD62L⁻CCR9⁺ population and high functional significance. Class 2 represents a strong association with the CD4⁺CD62L⁻CCR9⁺ population or high functional significance. Class 3 represents weak association and/or uncertain functional significance.

The aforementioned markers relate to tissue localisation, emigration, immigration and retention. In a similar experiment a high throughput screen was conducted to identify functional markers that are enriched within mucosal-tropic Treg populations when contrasted against mucosal-tropic cells that are non-Treg in nature. Analyses of cells with CD4⁺CD25^{hi}CD127^{lo}α4⁺β7⁺ character revealed strong enrichment of surface markers that denote regulatory function, and a restriction of markers that generally denote pro-inflammatory functions.

**Figure 32** shows an example of a functional marker, CD39 (ENTPD1), which is a putative immunosuppressive element on the surface of T-cells, and which is enriched in the CD4⁺CD25^{hi}CD127^{lo}α4⁺β7⁺ population. It is thus anticipated that CD39 may be used as a marker of preferred condition Y+, with which to select for Treg cells within mucosal emigrant, immigrant and educated CD4+ T-cell populations. The table presented in **Figure 32** **D** summarises other functional-type markers associated with the CD4⁺CD25^{hi}CD127^{lo}α4⁺β7⁺ population. The markers positively correlated are of condition Y+, and largely represent entities with putative immunosuppressive activities, where in the preferred aspect they are used as a positive selection marker for the purification of Treg cells from mucosal emigrant, immigrant and educated CD4⁺ T-cell populations. The markers negatively correlated are of condition Y-, and largely represent entities with putative pro-inflammatory activities, where in the preferred aspect they are used as a negative selection marker for the purification of Treg cells from mucosal emigrant, immigrant and educated CD4⁺ T-cell populations. Each marker in this table is also assigned a class, where class 1 represents a strong association with the CD4⁺CD25^{hi}CD127^{lo}α4⁺β7⁺ population and high functional significance. Class 2 represents a strong association with the CD4⁺CD25^{hi}CD127^{lo}α4⁺β7⁺ population or high functional significance. Class 3 represents weak association and/or uncertain functional significance.

In order to assess the feasibility of recovering the rare mucosal emigrant and tropic CD4⁺ Tregs from peripheral circulation, CD4⁺CD25^{hi}CD127^{lo}β7^{hi}CCR9⁺ Tregs were sorted from peripheral blood at high purity; PBMCs from healthy donors were labelled and sorted on the basis of these defined markers (**Figure 33**). **Figure 33** **a to e** show the basic gating strategy of FACS-based purification of these cells, and **Figure 33** **f and g** displays achieved purity of greater than 95%. **Figure 33h and i** show that CD4⁺CD25^{hi}CD127^{lo}β7^{hi}CCR9⁺target cells are largely of antigen experienced and recent activation character.

As proof of concept that, CD4⁺CD25^{hi}CD127^{lo}β7^{hi}CCR9⁺ purified from peripheral blood of could be expanded as a therapeutic population, cells purified by FACS as described in **Figure 33** were expanded with recombinant stimuli in vitro. **Figure 34** displays a representative growth curve of such an expansion.

Finally, to test the hypothesis that mucosal emigrant CD4⁺ Tregs in peripheral circulation are in some way clonally restricted due to their activated, emigrant and recirculating nature, an assessment of Vβ usage among CD4⁺CD25^{hi}CD127^{lo}α4⁺β7⁺CD38⁺CD62L⁻CCR9⁺in peripheral circulation was conducted with total peripheral Tregs (CD4⁺CD25^{hi}CD127^{lo}) as reference. (**Figure 35**). Across three healthy donors, the usage of Vβ segments was markedly different between CD4⁺CD25^{hi}CD127^{lo}α4⁺β7⁺CD38⁺CD62L⁻CCR9⁺and the total pool of CD4⁺CD25 ^{hi}CD127^{lo} lymphocytes. This indirectly supports the proposal that CD4⁺CD25^{hi}CD127^{lo}α4⁺β7⁺CD38⁺CD62L⁻CCR9⁺ mucosal emigrant Tregs are activated against a restricted set of antigens in the mucosa, and exported for recirculation in order to support regional and/or systemic tolerance.
From the case study above, a method for identification of Treg cells originating from any diseased tissue of interest is elucidated. The major elements of this model are defined in **Figure 36****.** Cells from the tissue of interest, Target tissue (A), are exported through lymphatics (B', B and B"), and subsequently migrate through peripheral blood (C) to recirculate to tissue of origin (A). Alternate route of recirculation is to a secondary tissue of interest (X). Tissues A, B, B', B", C and X are considered major sampling points for investigation. Major migratory processes involving recirculation of Tregs back to tissue of origin are defined for target tissue emigration (α), and immigration (β). Emigration from secondary tissue of interest (X) is defined as αX, and immigration βX. Migration between main target tissue A, and communicating tissue X, is defined as migration process ε. Migration processes α, β, αX, βX and ε are considered as definable by Treg migratory marker expression, as applied to Tregs recovered from sampled tissues A, B, B', B", C and X. Major sources of analytical noise are those cells collected in all compartments that bare migratory marker expression for migratory processes γ and δ. γ and δ migratory process markers are considered negative selection criteria for investigation of Treg cells of A or X origin, with α, β, αX, βX or ε migratory behaviour.

**Figure 37** shows that CD4⁺ cells with CCR9⁺CD103⁺ characteristics, denoting small bowel tropism, are present in the LP of the large bowel. This is also apparent in lymph nodes draining these distinct tissues, which suggests that colonic Tregs may be locally imprinted, in small numbers, to recirculate to the small bowel. This is suggestive that a process of α emigration, but βX immigration (as defined above) could be an active process, considering tissue A as colon, and tissue X as small bowel in this example.

### Experimental Material and Methods

### Material

Fluorochrome-conjugated antibodies were obtained from BD Biosciences or BioLegend; CD4-FITC, CD4-PE/Cy7 (OKT4), CD25-APC (2A3, M-A251), CD25-PE/Cy7 (BC960, M-A251), CD38-BV421 (HIRT2), CD38-PE (HIT2), CD45RO-PerCP/Cy5.5 (UCHL1) CD49d-PE/Cy7 (9F10), CD62L-PE/Cy7 (DREG-56), CD127-PerCP/Cy5.5, CD127-PE (A019D5, HIL-7R-M21), FOXP3-PE (259D/C7), FOXP3-AlexaFluor647 (206D), integrinβ7-PerCP/Cy5.5, integrinβ7-FITC (FIB27) CD62L BV421 (DREG-55), CD4 BV510 (SK3), CD25 BV605 (2A3), CD1c PE (L161), CD3 FITC (HIT3a), CD3 PE-CF594 (UCHT1), CD3 APC-H7 (SK7), CD4 BV605 (RPA-T4), CD4 PerCP (SK3), CD4 APC (RPA-T4), CD4 APC-H7 (RPA-T4), CD8 BV510 (RPA-T8), CD8 BV605 (SK1), CD8 BV786 (RPA-T8), CD8 Alexa 488 (RPA-T8), CD8 PerCP-Cy5.5 (RPA-T8), CD8 PE (RPA-T8), CD8 PE-Cy7 (RPA-T8), CD8 APC-H7 (SK1), CD11a PE (HI111), CD11b BV510 (ICRF44), CD11b PE-Cy7 (ICRF44), CD11c BV421 (B-ly6), CD11c BV605 (B-ly6), CD11c PE (B-ly6), CD14 BV510 (MϕP9), CD14 BV711 (MϕP9), CD14APC (M5E2), CD16 PerCP-Cy5.5 (3G8), CD16 PE (B73.1), CD18 BV421 (6,7), CD19 BV510 (SJ25C1), CD19 BV711 (SJ25C1), CD19 PE-Cy7 (SJ25C1), CD25 BV510 (MA251), CD25 BV786 (M-A251), CD25 PerCP-Cy5.5 (M-A251), CD25 PE-Cy7 (MA251), CD28 BV421 (CD28.2), CD28 BV605 (CD28.2), CD28 BV711 (CD28.2), CD28 FITC (CD28.2), CD28 PerCP-Cy5.5 (CD28.2), CD28 APC-H7 (CD28.2), CD29 BV510 (MAR4), CD29 PE (MAR4), CD29 APC (MAR4), CD31 BV605 (WM59), CD38 FITC (HIT2CD38), PE-CF594 (HIT2CD38), PE-Cy7 (HIT2CD38), Alexa700 (HIT2), CD38 APC-H7 (HB7), CD39 BV711 (Tü66), CD39 FITC (Tü66), CD45 BV605 (HI30), CD45 BV786 (HI30), CD45 FITC (HI30), CD45 PE (HI30), CD45 PE-Cy7 (HI30), CD45RA BV421 (HI100), CD45RA BV605 (HI100), CD45RA BV711 (HI100), CD45RA PerCP-Cy5.5 (HI100), CD45RA PE (HI100), CD45RO BV605 (UCHL1), CD45RO BV711 (UCHL1), CD45RO APC (UCHL1), CD49a PE (SR84), CD49b PE (12F1), CD49c PE (C3 II.1), CD49d BV510 (9F10), CD49d BV711 (9F10), CD49d PerCP-Cy5.5 (9F10), CD49d PE (9F10), CD49d PE-CF594 (9F10), CD49e PE (IIA1), CD49f PE (GoH3), CD56 BV510 (NCAM16.2), CD56 BV711 (NCAM16.2), CD62L BV510 (DREG-56), CD62L BV605 (DREG-56), CD69 BV605 (FN50), CD69 BV711 (FN50), CD69 PerCP-Cy5.5 (FN50), CD69 PE-Cy7 (FN50), CD73 BV605 (AD2), CD79a BV421 (HM47), CD79a PE (HM47), CD79a APC (HM47), CD79b PE (3A2-2E7), CD79b PE-Cy5 (CB3-1), CD80 BV605 (L307.4), CD80 PE (L307.4), CD80 PE-Cy7 (L307.4), CD80 APC (2D10), CD83 PerCP-Cy5.5 (HB15e), CD83 APC (HB15e), CD86 BV421 (2331), CD86 PerCP-Cy5.5 82331), CD86 APC (2331), CD103 BV711 (Ber-ACT8), CD103 FITC (Ber-ACT8), CD103 PE (Ber-ACT8), CD127 BV421 (HIL-7R-M21), CD127 BV605 (HIL-7R-M21), CD127 BV650 (HIL-7R-M21), CD127 BV711 (HIL-7R-M21), CD127 FITC (HIL-7R-M21), CD141 BV510 (1A4), CD141 PE (1A4), CD152 BV421 (BNI3), CD152 BV786 (BNI3), CD163 PerCP-Cy5.5 (GHI/61), CD192 BV421 (K036C2), CD196 BV421 (11A9), CD197 FITC (3D12), CD197 PerCP-Cy5.5 (150503), CD199 Alexa 488 (112509), CD199 FITC (112509), CD199 PE (112509), CD199 PE (L053E8), CD199 PE (248621), CD199 PE-Cy7 (L053E8), CD199 Alexa 647 (112509), CD199 Alexa 647 (L053E8), CD199 Alexa 647 (BL/CCR9), CD199 APC (112509), CD303 BV421 (201A), CD357 APC (621), Annexin V APC, β7 integrin BV421 (FIB504), β7 integrin BV605 (FIB504), β7 integrin PE (FIB504), β7 integrin APC (FIB504), CX3CR1 PerCP-Cy5.5 (2A9-1), FoxP3 Alexa 488 (259D/C7), Granzyme B BV421 (GB11), Granzyme B FITC (GB11), Granzyme B PE-CF594 (GB11), Helios PE (22F6), HLA-A2 PE-Cy7 (BB7.2), HLA-A,B,C PE-Cy5 (G46-2.6), HLA-E PE (3D12), HLA-G PE (87G), HLA-DM PE (MaP.DM1), HLA-DR PerCP-Cy5.5 (G46-6), HLA-DR PE-Cy7 (G46-6), HLA-DR APC (G46-6), HLA-DRB1, HLA-DR, DP, DQ FITC (Tü39), HLA-DR, DP, DQ Alexa 647 (Tü39), HLA-DQ FITC (Tu169), IFN-g Alexa 647 (4S.B3), IL-1b PE (AS10), IL-2 FITC (MQ1-17H12), IL-2 FITC (MQ1-17H12), IL-4 FITC (MP4-25D2), IL-10 APC (JES3-19F1), IL-12 FITC (C11.5), IL-17A PE (SCPL1362), IL-35 PE (B032F6), Ig κ light chain PE (G20-193), Light chain λ PE (JDC-12), IgM BV605 (G20-127), IgM FITC (G20-127), IgM FITC IgM PE-Cy5 (G20-127), Lineage cocktail FITC, Perforin BV421 (δG9), Perforin Alexa 488 (δG9), Syk FITC (4D10), Syk PY352 PE (17A/P-ZAP70), Syk PY352 PE-Cy7 (17A/P-ZAP70), Syk PY352 Alexa 647 (17A/P-ZAP70), TCR αβ BV510 (T10B9.1A-31), TCR αβ BV786 (T10B9.1A-31), TCR γδ FITC (B1), TCR γδ-1 FITC (11F2), TCR γδ PE-CF594 (B1), TGF-b1 BV421 (TW4-9E7), TNF-a APC (MAb11), and unlabelled antibodies were obtained from BD Biosciecnes; CD1a (HI149), CD28 (L293), CD51/61 (23C6), CD1b (M-T101), CD29 (HUTS-21), CD53 (HI29), CD1d (CD1d42), CD30 (BerH8), CD54 (LB-2), CD2 (RPA-2.10), CD31 (WM59), CD55 (IA10), CD3 (HIT 3a), CD32 (FL18.26), CD56 (B159), CD4 (RPA-T4), CD33 (HIM3- 4), CD57 (NK-1), CD4v4 (L120), CD34 (581), CD58 (1C3), CD5 (L17F12), CD35 (E11), CD59 (p282, H19), CD6 (M-T605), CD36 (CB38, NL07), CD61 (VI-PL2), CD7 (M-T701), CD37 (M- B371), CD62E (68- 5H11), CD8a (SK1), CD38 (HIT 2), CD62L (Dreg 56), CD8b (2ST 8.5H7), CD39 (TU66), CD62P (AK-4), CD9 (M-L13), CD40 (5C3), CD63 (H5C6), CD10 (HI10a), CD41a (HIP8), CD64 (10.1), CD11a (G43-25B), CD41b (HIP2), CD66 (a,c,d,e) (B1.1/CD66), CD11b (D12), CD42a (ALMA.16), CD66b (G10F5), CD11c (B- ly 6), CD42b (HIP1), CD66f (IID10), CD13 (WM15), CD43 (1G10), CD69 (FN50), CD14 (M5E2), CD44 (G44- 26), CD70 (Ki- 24), CD15 (HI98), CD45 (HI30), CD71 (M-A712), CD15s (CSLEX1), CD45RA (HI100), CD72 (J4- 117), CD16 (3G8), CD45RB (MT4), CD73 (AD2), CD18 (6.7), CD45RO (UCHL1), CD74 (M- B741), CD19 (HIB19), CD46 (E4.3), CD75 (LN1), CD20 (2H7), CD47 (B6H12), CD77 (5B5), CD21 (B-ly 4), CD48 (T U145), CD79b (CB3- 1), CD22 (HIB22 CD49a SR84 CD80 L307.4 CD23 EBVCS-5 CD49b AK-7 CD81 JS-81), CD24 (ML5), CD49c (C3 II.1), CD83 (HB15e), CD25 (M-A251), CD49d (9F10), CD84 (2G7), CD26 (M-A261), CD49e (VC5), CD85 (GHI/75), CD27 (M-T271), CD50 (TU41), CD86 (2331, FUN-1), CD123 (9F5), CD172b (B4B6), CD87 (VIM5), CD124 (hlL4R- M57), CD177 (MEM- 166), CD88 (D53-1473), CD126 (M5), CD178 (NOK- 1), CD89 (A59), CD127 (hIL-7R- M21), CD180 (G28- 8), CD90 (5E10), CD128b (6C6), CD181 (5A12), CD91 (A2MR-alpha 2), CD130 (AM64), CD183 (1C6/CXCR3), CDw93 (R139), CD134 (ACT35), CD184 (12G5), CD94 (HP- 3D9), CD135 (4G8), CD193 (5E8), CD95 (DX2), CD137 (4B4-1), CD195 (2D7/CCR5), CD97 (VIM3b), CD137 (Ligand C65- 485), CD196 (11A9), CD98 (UM7F8), CD138 (Mi15), CD197 (2H4), CD99 (TU12), CD140a (alpha R1), CD200 (MRC OX-104), CD99R (HIT 4), CD140b (28D4), CD205 (MG38), CD100 (A8), CD141 (1A4), CD206 (19.2), CD102 (CBR-1C2/2.1), CD142 (HTF-1), CD209 (DCN46), CD103 (Ber-ACT8), CD144 (55-7H1), CD220 (3B6/IR), CD105 (266), CD146 (P1H12), CD221 (3B7), CD106 (51- 10C9), CD147 (HIM6), CD226 (DX11), CD107a (H4A3), CD150 (A12), CD227 (HMPV), CD107b (H4B4), CD151 (14A2.H1), CD229 (HLy9.1.25), CD108 (KS-2), CD152 (BNI3), CD231 (M3-3D9, SN1a), CD109 (TEA 2/16), CD153 (D2-1173), CD235a (GA-R2, HIR2), CD112 (R2.525), CD154 (TRAP1), CD243 (17F9), CD114 (LMM741), CD158a (HP- 3E4), CD244 (2- 69), CD116 (M5D12), CD158b (CH-L), CD255 (CARL-1), CD117 (Y B5.B8), CD161 (DX12), CD268 (11C1), CD118 (12D3), CD162 (KPL-1), CD271 (C40-1457), CD119 (GIR-208), CD163 (GHI/61), CD273 (MIH18), CD120a (MABTNFR1-A1), CD164 (N6B6), CD274 (MIH1), CD121a (HIL1R-M1), CD165 (SN2), CD275 (2D3/B7- H2), CD121b (MNC2), CD166 (3A6), CD278 (DX29), CD122 (Mik-beta 3), CD171 (5G3), CD279 (MIH4), fMLP receptor (5F1), Ms IgG2a IC (G155-178), CD282 (11G7), γδTCR (B1), Ms IgG2b IC (27- 35), CD305 (DX26), HPC (BB9), Ms IgG3 IC (J606), CD309 (89106), HLA-A,B,C (G46-2.6), CD49f (GoH3), CD314 (1D11), HLA-A2 (BB7.2), CD104 (439- 9B), CD321 (M.AB.F11), HLA- DQ (TU169), CD120b (hTNFR-M1), CDw327 (E20-1232), HLA- DR (G46-6, L243), CD132 (TUGh4), CDw328 (F023-420), HLA-DR, DP, DQ (TU39), CD201 (RCR-252), CDw329 (E10-286), Invariant NK T (6B11), CD210 (3F9), CD335 (9E2/NKp46), Disialoganglioside GD2 (14.G2a), CD212 (2B6/12beta 2), CD336 (P44-8.1), MIC A/B (6D4), CD267 (1A1-K21-M22), CD337 (P30-15), NKB1 (DX9), CD294 (BM16), CD338 (5D3), SSEA-1 (MC480), SSEA-3 (MC631), CD304 (Neu24.7), SSEA-4 (MC813-70), CLA (HECA- 452), αβT CR (T10B9.1A-31), TRA-1-60 (TRA-1-60), Integrin β7 (FIB504), β2- mic roglobulin (TU99), TRA-1-81 (TRA-1-81), Rt IgM IC (R4-22), BLTR-1 (203/14F11), Vβ 23 (AHUT 7), Rt IgG1 IC (R3- 34), CLIP (CerCLIP), Vβ 8 (JR2), Rt IgG2a IC (R35- 95), CMRF-44 (CMRF44), CD326 (EBA-1), Rt IgG2b IC (A95-1), CMRF-56 (CMRF56), Ms IgM IC (G155-228), EGF Receptor (EGFR1), Ms IgG1 IC (MOPC- 21) and Zombie NIR™ Fixable Viability Kit or BD Biosciences; CD4-PacificBlue (RPA-T4); collagenaselV, DNasel, DTT, EDTA and sodium azide from *SigmaAldrich*; *FicollPaquePlus* from *GEHealthcare*, RPMI media, BSA and FCS from *Life Technologies*; IOTest Beta Mark TCR V Kit from Beckman Coulter.

### Patients and Tissue Preparation

All subjects gave their written informed consent under the Helsinki guidelines and local ethics committee. CD patients undergoing ileoceacal resection were recruited to the study. We collected small bowel (ileum) and large bowel (ceacum/ascending colon), including MLN draining these regions. Control samples were from colorectal cancer patients undergoing right-sided hemicolectomy. Intestinal lamina propria from the small and large bowel was separated via microdissection. The dissected lamina propria was minced into 1-2 mm pieces and single cell suspensions were prepared in RPMI 1640 containing 5% FBS, 50 µg/ml gentamycin and 50 µg/ml Penicillin/Streptomycin using the Medimachine with a 50 µm Medicon (BD Biosciences). The cell suspension was filtered through a 70-µm nylon mesh (BD Biosciences), centrifuged and the pellet resuspended in FACS buffer (PBS containing 2% FBS) for subsequent antibody staining. Lymphocytes from MLN were isolated by mechanical disruption of lymph nodes after surrounding fat tissue was removed by dissection. The cell suspension was filtered through a 40-µm nylon mesh (BD Biosciences), centrifuged and the pellet resuspended in FACS buffer for subsequent antibody staining.

### Patients and Blood Preparation

All subjects gave their written informed consent under the Helsinki guidelines and local ethics committee. Healthy donors were recruited to the blood cohorts. Blood drawn into EDTA tubes was diluted 1:2 in PBS with 2 mM EDTA and PBMCs collected over a *FicollPaquePlus* density gradient by centrifugation. PBMCs were washed 3 times in wash buffer (PBS, 0.2% BSA, 5 mM EDTA) before immediate flow cytometry.

### Direct Cell Purification by FACS

Extracellular antigens were stained in FACS buffer (PBS, 2% BSA) using appropriate combinations of fluorophore-conjugated antibodies (BioLegend and BD Biosciences). Specific cell populations were purified by fluorescence-activated cell sorting (FACS) using a BD Influx cell sorter with BD FACS Sortware (BD Biosciences) to acquire data. Final analyses utilized *FlowJo* software (*Tree Star Inc*.).

### Expansion of sorted cell populations

The sorted cell populations were expanded in OpTmizer media with 2mM Glutamax (both Life Technologies) and either autologous or commercial human serum (Sigma) using MACS GMP ExpAct Treg Kit (Miltenyi Biotec) and in the presence of recombinant human IL-2 (Miltenyi Biotec).

### Flow Cytometry

Zombie NIR Fixable Viability Kit (Biolegend) was used as a dead cell marker. Surface antigens were stained in FACS buffer (PBS containing 2% FBS) and intracellular FoxP3 was stained after fixation and permeabilization using the human FoxP3 buffer set (BD Biosciences). Cells were acquired using a LSRFortessa flow cytometer with Diva 8 software (BD Biosciences). Final analysis was performed using FlowJo 10 software (Tree Star Inc.).

### Statistics

All data was expressed as mean ± SEM. Pair wise comparisons were two-tailed Mann-Whitney U-tests. Significance testing of multiple parameters was calculated with Krus-kal-Wallis one-way ANOVA and Dunn's post-test of selected columns. A p value < 0.05 was considered significant.

## Claims

1. A method for identifying CD4+ Treg cells suitable for use as starting material in cellular immunotherapy, the method comprising
i) analysing samples from target tissue A to identify CD4+ Treg cells with migratory character between the diseased tissue, collecting lymphatics, peripheral blood, distinct tissue adjacent to the diseased target tissue A and/or distinct tissue that is not vicinal though has migratory Treg communication with target tissue A,
ii) optionally analysing samples from target tissue A to identify CD4⁺ Treg cells with immunosuppressive function in tissue A,
iii) optionally analysing samples from lymphatic tissue B to identify CD4⁺ Treg cells with migratory character between disease draining lymphatics and non-disease draining lymphatics of diseased or non-diseased target tissue A,
iv) optionally analysing samples from lymphatic tissue B to identify CD4⁺ Treg cells with immunosuppressive function in tissue B where the Treg cells are also emigrant from target tissue A,
v) analysing samples from peripheral blood, tissue C, to identify CD4+ Treg cells with migratory character and/or immunosuppressive function where the Treg cells are also emigrant from target tissue A,
vi) analysing sample(s) from tissue compartments A and/or B and C, that are analytically or physically depleted of emigrants from thymus and/or immigrants from peripheral blood to a lymph node, to restrict analyses to CD4⁺ Treg cells of target tissue A origin and/or tropism,
to identify emigrant CD4⁺ Treg cell populations of target tissue A,
to identify emigrant CD4⁺ Treg cell populations with propensity to immigrate to target tissue A,
to identify a migratory and/or functional defect in the CD4+ Treg cell population identified as expressing migratory and/or functional elements specific for target tissue A in any of tissue A, B or C, and
whereby a combination of surface or intracellular markers on CD4+ Treg cells is identified, which combination identifies which surface or intracellular markers should be present and which surface markers should not be present in CD4+ Treg cell populations suitable for use as starting material in cellular immunotherapy.

2. A method according to claim 1, wherein the sample from tissue A is selected from solid tissues, interstitial fluids of solid tissue, oedemic or inflammatory fluids of diseased tissue regions, or tissues represented in a fluid phase.

3. A method according to claim 1 or 2, wherein the sample from tissue A is selected from one or more of the following:
i) Epithelial mucosal surfaces for investigation of intra-epithelial cell populations as collected by mucosal scrapings or lavage sampling, or fractionation of biopsy/resection specimens,
ii) Sub-epithelial surfaces (e.g. lamina propria) as collected by biopsy or resection,
iii) Stroma of solid tissues as collected by biopsy or resection,
iv) Parenchyma of solid tissues as collected by biopsy or resection,
v) Endothelial and endothelial-vicinal tissues as collected by resection,
vi) Dermal layers as collected by cutaneous punch sampling, biopsy by incision or samples of tissues collected for grafting,
vii) Interstitial fluids of solid tissues collected by passive fluid collection methods,
viii) Synovial fluids of joint capsules or bursae as collected by active sampling methods,
iix) Cerebrospinal fluids as collected by active sampling methods,
ix) Oedemic or lymphedema fluids of solid tissues of bodily cavities collected by passive or active sampling methods (e.g. ascites of peritoneal cavity, or oedemic and lymphedemic fluids accumulating in solid tissues),
x) Nervous tissues as collected by biopsy or recovery from resected tissues or limb amputation,
xi) Skeletal muscle tissues as collected by biopsy or recovery from resected tissues or limb amputation.

4. A method according to any of the preceding claims, wherein option iii) of the method claimed in claim 1 is mandatory and the comparison is made either with
a) samples from a single subject suffering from an inflammatory or autoimmune disease or
b) samples from a subject suffering from an inflammatory or autoimmune disease and a healthy volunteer.

5. A method according to any of the preceding claims, wherein tissue B represents lymph nodes directly draining target tissue A via collecting lymphatic vessels.

6. A method according to any of the preceding claims, wherein tissue B is selected from one or more of the following:
i) disease draining (sentinel) lymph nodes as sampled by resection and processing, or active sampling by puncture and fluid draw.
ii) lymph fluids of the diseased tissue as collected by microsurgical access of collecting lymph vessel and installation of a cannula for passive fluid collection.
iii) Distal lymph fluids communicating from disease draining lymph nodes as collected by surgical installation of a cannula for passive fluid collection of minor distal lymphatic vessels, or active sampling of major distal lymphatic vessels where appropriate.

7. A method according to any of the preceding claims, wherein one or more of the analyses of the samples is a method for single-cell or highly restricted cell population analyses.

8. A method according to claim 7, wherein said method for single-cell or highly restricted cell population analyses may be one or more of the following:
i) Flow cytometric methods detecting surface antigen expression,
ii) Flow cytometric methods detecting intracellular antigen expression,
iii) Flow cytometric methods detecting transcript or genomic parameters by in situ probe hybridisation techniques,
iv) Flow cytometric analyses of enzyme function or metabolite abundance.

9. A method according to any of the preceding claims further comprising a purification or enrichment step wherein the purification or enrichment involves one or more of:
v) Flow cytometric purification of single cells for submission to downstream analytical workflows,
vi) Flow cytometric purification of highly restricted cell populations and subpopulations for submission to downstream analytical workflows,
vii) Substrate immunoaffinity enrichment of highly restricted cell populations and subpopulations for submission to downstream analytical workflows, and/or
viii) Substrate immunoaffinity enrichment of highly restricted cell populations and subpopulations, coupled with flow cytometric purification of single cells and/or highly restricted cell populations, for submission to downstream analytical workflows.

10. A method for obtaining a CD4⁺ Treg cell population for use as starting material in cellular immunotherapy, the method comprising
i) subjecting peripheral blood from a patient suffering from an inflammatory or an autoimmune disease to single-cell analysis, whereby CD4+ Treg cells having the signatures identified in the method defined in any of claims 1-13 are separated from the blood.

11. A method according to claim 10, wherein analytical filters are applied to
i) exclude cells that gain access to lymph nodes via HEV, and
ii) exclude cells that are recent thymic emigrants.

12. A method according to claim 11, wherein the cells that gain access to lymph nodes via HEV are CD62L+ cells.

13. A method according to claim 10 or 11, wherein the cells that are recent thymic emigrants are CCR9+CD45RA+, CCR9+CCR7+, CCD9+CD62L+, or CCR9+CD45RO- cells.

14. A method according to claims 10-13, wherein the cells to be excluded are CCR9+CCR7+CD62L+CD45RA+CD45RO- cells.

15. A method according to any of claims 10-14, wherein analyses include conditions for identifying CD4+ Treg cells that are emigrant and immigrant cells such as integrin-type or other adhesion molecules associated with Target-A tissue adhesion and transmigration through tissue-integral vasculature.

## Patentansprüche

1. Ein Verfahren zur Identifizierung von CD4⁺ Treg Zellen, die zur Verwendung als Startmaterial in der zellulären Immuntherapie geeignet sind, das Verfahren umfassend
i) das Analysieren von Proben aus Zielgewebe A, um CD4⁺ Treg Zellen mit migratorischer Eigenschaft zwischen dem erkrankten Gewebe, den sammelnden Lymphgefäßen, dem peripheren Blut, den abgegrenzten Geweben neben dem erkrankten Zielgewebe A und/ oder abgegrenztes Gewebe, das nicht daneben ist, dennoch aber migratorische Treg Kommunikation mit dem Zielgewebe A hat, zu identifizieren,
ii) optional das Analysieren von Proben aus Zielgewebe A, um CD4⁺ Treg Zellen mit immun-supprimierender Funktion in Gewebe A zu identifizieren,
iii) optional das Analysieren von Proben aus dem lymphatischen Gewebe B, um CD4⁺ Treg Zellen mit migratorischer Eigenschaft zwischen erkrankten drainierenden Lymphgefäßen und nicht-erkrankten drainierenden Lymphgefäßen von erkranktem oder nicht-erkranktem Zielgewebe A zu identifizieren,
iv) optional das Analysieren von Proben aus lymphatischem Gewebe B, um CD4⁺ Treg Zellen mit immun-supprimierender Funktion in Gewebe B zu identifizieren, wobei die Treg Zellen auch aus Zielgewebe A ausgewandert sind,
v) das Analysieren von Proben aus dem peripheren Blut, Gewebe C, um CD4⁺ Treg Zellen mit migratorischer Eigenschaft und/ oder immun-supprimierender Funktion zu identifizieren, wobei die Treg Zellen auch aus Zielgewebe A ausgewandert sind,
vi) das Analysieren von Probe(n) aus Gewebe-Kompartimenten A und/ oder B und C, die analytisch oder physikalisch frei von aus dem Thymus ausgewanderten Zellen und/ oder immigrierten Zellen aus dem peripheren Blut in einen Lymphknoten sind, um die Analysen auf CD4⁺ Treg Zellen, die ihren Ursprung im Zielgewebe A haben und/ oder Tropismus zu beschränken,
um ausgewanderte CD4⁺ Treg Zellpopulationen von Zielgewebe A zu identifizieren,
um ausgewanderte CD4⁺ Treg Zellpopulationen mit Tendenz zur Immigration in Zielgewebe A zu identifizieren,
um einen migratorischen oder funktionellen Defekt in der CD4⁺ Treg Zellpopulation zu identifizieren, die in einem der Gewebe A, B oder C identifiziert ist als migratorische und/ oder funtionelle Elemente exprimierend, die spezifisch für Zielgewebe A ist, und wobei eine Kombination von Oberflächen- oder intrazellulären Markern auf CD4⁺ Treg Zellen identifiziert ist, deren Kombination identifiziert, welche Oberflächen oder intrazellulären Marker vorliegen sollten und welche Oberflächenmarker nicht vorliegen sollten auf CD4⁺ Treg Zellpopulationen, die zur Verwendung als Startmaterial in der zellulären Immuntherapie geeignet sind.

2. Ein Verfahren nach Anspruch 1, wobei die Probe von Gewebe A ausgewählt ist aus festem Gewebe, interstitiellen Flüssigkeiten von festem Gewebe, ödemischen oder entzündlichen Flüssigkeiten aus erkrankten Gewebebereichen oder Gewebe, das in flüssiger Phase repräsentiert ist.

3. Ein Verfahren nach Anspruch 1 oder 2, wobei die Probe aus Gewebe A ausgewählt ist von einem oder mehreren der folgenden:
i) epitheliale mukosale Oberflächen für die Erforschung von intra-epithelialen Zellpopulationen wie sie bei mukosalen Ausschabungen oder Spülungsprobenentnahme gesammelt werden, oder Fraktionierungs- oder Biopsie/Resektionsproben,
ii) sub-epitheliale Oberflächen (z.B. lamina propria) wie sie durch Biopsie oder Resektion gesammelt werden,
iii) Stroma von festen Geweben wie sie durch Biopsie oder Resektion gesammelt werden,
iv) Parenchym von festen Geweben wie sie durch Biopsie oder Resektion gesammelt werden,
v) endotheliale oder endothel-nahe Gewebe wie sie durch Resektion gesammelt werden,
vi) dermale Schichten wie sie durch Stanz-Probenentnahme, Biopsien wie sie durch Schnitte gesammelt werden oder Gewebeproben, die für die Transplantation gesammelt werden,
vii) interstitielle Flüssigkeiten von festen Geweben gesammelt durch passive Fluss-Sammelmethoden,
viii) Synovialflüssigkeiten von Gelenkskapseln oder Schleimbeuteln wie sie durch aktive Sammelmethoden gesammelt werden,
iix) Cerebrospinalflüssigkeiten wie sie durch aktive Sammelmethoden gesammelt werden
ix) ödemische oder lymphödemische Flüssigkeiten von festen Geweben von Körperhöhlen gesammelt durch passive oder aktive Sammelmethoden (z.B. Aszites aus der Bauchhöhle oder ödemische und lymphödemische Flüssigkeiten, die in festen Geweben akkumulieren),
x) Nervengewebe wie sie durch Biopsie oder Gewinnung von reseziertem Gewebe oder Extremitätsamputation gesammelt werden,
xi) skeletale Muskelgewebe wie sie durch Biopsie oder Gewinnung von reseziertem Gewebe oder Extremitätsamputation gesammelt werden.

4. Ein Verfahren nach einem der vorhergehenden Ansprüche, wobei Option iii) des Verfahrens, das in Anspruch 1 beansprucht wird, obligatorisch ist und der Vergleich entweder mit
a) Proben eines einzelnen Subjekts, das an einer entzündlichen oder Autoimmun-Erkrankung leidet oder
b) Proben eines Subjekt, das an einer entzündlichen oder Autoimmun-Erkrankung leidet, und eines gesunden Freiwilligen
angestellt wird.

5. Ein Verfahren nach einem der vorhergehenden Ansprüche, wobei Gewebe B Lymphknoten darstellt, die direkt Zielgewebe A über Sammellymphgefäße drainieren.

6. Ein Verfahren nach einem der vorhergehenden Ansprüche, wobei Gewebe B ausgewählt ist aus einem oder mehreren der folgenden:
i) erkrankte drainierende (Wächter) Lymphknoten wie sie bei Resektion und Weiterbearbeitung, oder dem aktiven Sammeln mittels Punktur und Flüssigkeitsabnahme gesammelt werden.
ii) Lymphflüssigkeiten des erkrankten Gewebes wie sie mittels mikrochirurgischem Eingriffs zum Sammeln der Lymphgefäße und dem Anbringen einer Kanüle für passive Flüssigkeitsabnahme gesammelt werden.
iii) distale Lymphflüssigkeiten, die aus den erkrankten drainierenden Lymphknoten kommunizieren, wie sie durch das chirurgische Anbringen einer Kanüle für die passive Flüssigkeitssammlung der kleinen distalen lymphatischen Gefäße oder - wo angemessen - das aktive Sammeln großer distaler lymphatischer Gefäße gesammelt werden.

7. Ein Verfahren nach einem der vorhergehenden Ansprüche, wobei eine oder mehrere der Analysen der Proben ein Verfahren zu Einzelzell- oder stark limitierten Zellpopulationsanalysen ist.

8. Ein Verfahren nach Anspruch 7, wobei jenes Verfahren zu Einzelzell- oder stark limitierten Zellpopulationsanalysen eines oder mehrere der folgenden sein kann:
i) durchflusszytometrische Verfahren, die die Oberflächenantigenexpression detektieren,
ii) durchflusszytometrische Verfahren, die die intrazelluläre Antigenexpression detektieren,
iii) durchflusszytometrische Verfahren, die transkriptionelle oder genomische Parameter durch in situ Sonden-Hybridisierungstechniken detektieren,
iv) durchflusszytometrische Analysen der Enzymfunktion oder Metabolitenabundanz.

9. Ein Verfahren nach einem der vorhergehenden Ansprüche, ferner umfassend einen Aufreinigungs- oder Anreicherungsschritt, wobei die Aufreinigung oder Anreicherung eines oder mehrere der folgenden beinhaltet:
v) durchflusszytometrische Aufreinigung der Einzelzellen für die Weitergabe an nachgelagerte analytische Arbeitsabläufe,
vi) durchflusszytometrische Aufreinigung der stark limitierten Zellpopulationen und Subpopulationen zur Weitergabe an nachgelagerte analytische Arbeitsabläufe,
vii) Substrat-Immunoaffinitätsanreicherung der stark limitierten Zellpopulationen und Subpopulationen zur Weitergabe an nachgelagerte analytische Arbeitsabläufe und/ oder
viii) Substrat-Immunoaffinitätsanreicherung der stark limitierten Zellpopulationen und Subpopulationen, verbunden mit durchflusszytometrischer Aufreinigung der Einzelzellen und/ oder stark limitierten Zellpopulationen zur Weitergabe an nachgelagerte analytische Arbeitsabläufe.

10. Ein Verfahren zur Gewinnung einer CD4⁺ Treg Zellpopulation zur Verwendung als Startmaterial in der zellulären Immuntherapie, das Verfahren umfassend
i) das Unterziehen des peripheren Blutes eines Patienten, der an einer entzündlichen oder Autoimmun-Erkrankung leidet der Einzelzell-Analyse, wobei CD4⁺ Treg Zellen, die die Signaturen haben, die in dem Verfahren, das in einem der Ansprüche 1-13 definiert ist, identifiziert wurden, vom Blut abgetrennt werden.

11. Ein Verfahren nach Anspruch 10, wobei analytische Filter angewendet werden, um
i) Zellen auszuschließen, die Zugang zu den Lymphknoten via HEV erlangen und
ii) Zellen auszuschließen, die kürzliche thymische Auswanderer sind.

12. Ein Verfahren nach Anspruch 11, wobei die Zellen, die Zugang zu den Lymphknoten via HEV erlangen CD62L⁺ Zellen sind.

13. Ein Verfahren nach Anspruch 10 oder 11, wobei die Zellen, die kürzliche thymische Auswanderer sind, CCR9⁺CD45RA⁺, CCR9⁺CCR7⁺, CCD9⁺CD62L⁺ oder CCR9⁺CD45RO⁻ Zellen sind.

14. Ein Verfahren nach den Ansprüchen 10-13, wobei die Zellen, die ausgeschlossen werden sollen, CCR9⁺CCR7⁺CD62L⁺CD45RA⁺CD45RO⁻Zellen sind.

15. Ein Verfahren nach einem der Ansprüche 10-14, wobei die Analysen Bedingungen zur Identifizierung von CD4⁺ Treg Zellen einschließen, die ausgewanderte und immigrierte Zellen sind, wie Integrin-Typ oder andere Adhäsionsmoleküle, die mit Zielgewebe A Adhäsion und Transmigration durch gewebe-integrale Gefäße assoziiert sind.

## Revendications

1. Procédé pour identifier des cellules Treg CD4⁺ appropriées pour une utilisation en tant que matériel de départ dans une immunothérapie cellulaire, le procédé comprenant
i) l'analyse d'échantillons d'un tissu cible A afin d'identifier des cellules Treg CD4⁺ présentant un caractère migratoire entre le tissu pathologique, les vaisseaux lymphatiques collecteurs, le sang périphérique, un tissu distinct adjacent au tissu cible A pathologique et/ou un tissu distinct non voisin qui présente cependant une communication de Treg migratoires avec le tissu cible A,
ii) facultativement l'analyse d'échantillons du tissu cible A afin d'identifier des cellules Treg CD4⁺ présentant une fonction immunosuppressive dans le tissu A,
iii) facultativement l'analyse d'échantillons d'un tissu lymphatique B afin d'identifier des cellules Treg CD4⁺ présentant un caractère migratoire entre des vaisseaux lymphatiques drainant la maladie et des vaisseaux lymphatiques ne drainant pas la maladie d'un tissu cible A pathologique ou non pathologique,
iv) facultativement l'analyse d'échantillons du tissu lymphatique B afin d'identifier des cellules Treg CD4⁺ présentant une fonction immunosuppressive dans le tissu B, où les cellules Treg sont également des émigrants provenant du tissu cible A,
v) l'analyse d'échantillons du sang périphérique, le tissu C, afin d'identifier des cellules Treg CD4⁺ présentant un caractère migratoire et/ou une fonction immunosuppressive, où les cellules Treg sont également des émigrants provenant du tissu cible A,
vi) l'analyse d'échantillon(s) des compartiments tissulaires A et/ou B et C qui, d'un point de vue analytique ou physique, présentent une déplétion en émigrants du thymus et/ou en immigrants du sang périphérique vers un ganglion lymphatique, afin de restreindre les analyses aux cellules Treg CD4⁺ ayant pour origine et/ou présentant un tropisme pour le tissu cible A,
afin d'identifier les populations de cellules Treg CD4⁺ émigrantes du tissu cible A,
afin d'identifier les populations de cellules Treg CD4⁺ émigrantes ayant une propension à immigrer vers le tissu cible A,
afin d'identifier une anomalie migratoire et/ou fonctionnelle dans la population de cellules Treg CD4⁺ identifiée comme exprimant des éléments migratoires et/ou fonctionnels spécifiques pour le tissu cible A dans l'un quelconque du tissu A, B ou C, et
moyennant quoi une combinaison de marqueurs de surface ou intracellulaires sur les cellules Treg CD4⁺ est identifiée, laquelle combinaison identifie les marqueurs de surface ou intracellulaires qui doivent être présents et les marqueurs de surface qui ne doivent pas être présents dans des populations de cellules Treg CD4⁺ appropriées pour une utilisation en tant que matériel de départ dans une immunothérapie cellulaire.

2. Procédé selon la revendication 1, dans lequel l'échantillon du tissu A est sélectionné parmi des tissus solides, des liquides interstitiels d'un tissu solide, des liquides d'oedème ou inflammatoires de régions tissulaires pathologiques, ou des tissus représentés dans une phase liquide.

3. Procédé selon la revendication 1 ou 2, dans lequel l'échantillon du tissu A est sélectionné parmi un ou plusieurs des éléments suivants :
i) des surfaces muqueuses épithéliales pour l'étude de populations cellulaires intra-épithéliales, telles que collectées par grattages des muqueuses ou prélèvement d'échantillons de lavage, ou fractionnement d'échantillons de biopsie/résection,
ii) des surfaces sous-épithéliales (par exemple, lamina propria), telles que collectées par biopsie ou résection,
iii) un stroma de tissus solides, tel que collecté par biopsie ou résection,
iv) un parenchyme de tissus solides, tel que collecté par biopsie ou résection,
v) des tissus endothéliaux et voisins de l'endothélium, tels que collectés par résection,
vi) des couches dermiques, telles que collectées par prélèvement cutané au poinçon, biopsie par incision ou des échantillons de tissus collectés à des fins de greffe,
vii) des liquides interstitiels de tissus solides collectés par des procédés de collecte de liquide passive,
viii) des liquides synoviaux de capsules articulaires ou de bourses séreuses, tels que collectés par des procédés de prélèvement actif,
ix) des liquides céphalorachidiens, tels que collectés par des procédés de prélèvement actif,
x) des liquides d'oedème ou de lymphoedème de tissus solides de cavités corporelles collectés par des procédés de prélèvement passif ou actif (par exemple, ascites de la cavité péritonéale, ou liquides d'oedème et de lymphoedème s'accumulant dans des tissus solides),
xi) des tissus nerveux, tels que collectés par biopsie ou récupération à partir de tissus réséqués ou suite à l'amputation d'un membre,
xii) des tissus de muscles squelettiques, tels que collectés par biopsie ou récupération à partir de tissus réséqués ou suite à l'amputation d'un membre.

4. Procédé selon l'une quelconque des revendications précédentes, dans lequel l'option iii) du procédé revendiqué dans la revendication 1 est obligatoire et la comparaison est effectuée avec
a) des échantillons d'un sujet individuel souffrant d'une maladie inflammatoire ou auto-immune ou
b) des échantillons d'un sujet souffrant d'une maladie inflammatoire ou auto-immune et d'un volontaire sain.

5. Procédé selon l'une quelconque des revendications précédentes, dans lequel le tissu B représente des ganglions lymphatiques drainant directement le tissu cible A via les vaisseaux lymphatiques collecteurs.

6. Procédé selon l'une quelconque des revendications précédentes, dans lequel le tissu B est sélectionné parmi un ou plusieurs des éléments suivants :
i) des ganglions lymphatiques drainant la maladie (sentinelles), tels que prélevés par résection et traitement, ou prélèvement actif par ponction et aspiration de liquide,
ii) des liquides lymphatiques du tissu pathologique, tels que collectés par accès microchirurgical d'un vaisseau lymphatique collecteur et l'installation d'une canule pour une collecte de liquide passive,
iii) des liquides lymphatiques distaux communiquant à partir de ganglions lymphatiques drainant la maladie, tels que collectés par l'installation chirurgicale d'une canule pour une collecte de liquide passive de vaisseaux lymphatiques distaux mineurs, ou prélèvement actif de vaisseaux lymphatiques distaux majeurs tel qu'approprié.

7. Procédé selon l'une quelconque des revendications précédentes, dans lequel une ou plusieurs des analyses des échantillons est un procédé pour des analyses d'une cellule individuelle ou d'une population cellulaire fortement restreinte.

8. Procédé selon la revendication 7, où ledit procédé pour des analyses d'une cellule individuelle ou d'une population cellulaire fortement restreinte peut être un ou plusieurs des éléments suivants :
i) des procédés de cytométrie en flux détectant l'expression d'un antigène de surface,
ii) des procédés de cytométrie en flux détectant l'expression d'un antigène intracellulaire,
iii) des procédés de cytométrie en flux détectant un transcrit ou des paramètres génomiques par des techniques d'hybridation de sonde *in situ*,
iv) des analyses de cytométrie en flux d'une fonction enzymatique ou de l'abondance d'un métabolite.

9. Procédé selon l'une quelconque des revendications précédentes, comprenant en outre une étape de purification ou d'enrichissement dans laquelle la purification ou l'enrichissement implique un ou plusieurs éléments parmi :
v) une purification par cytométrie en flux de cellules individuelles afin de les soumettre à des flux de travail analytiques en aval,
vi) une purification par cytométrie en flux de populations et sous-populations cellulaires fortement restreintes afin de les soumettre à des flux de travail analytiques en aval,
vii) un enrichissement par immunoaffinité pour un substrat de populations et sous-populations cellulaires fortement restreintes afin de les soumettre à des flux de travail analytiques en aval, et/ou
viii) un enrichissement par immunoaffinité pour un substrat de populations et sous-populations cellulaires fortement restreintes, couplé à une purification par cytométrie en flux de cellules individuelles et/ou de populations cellulaires fortement restreintes, afin de les soumettre à des flux de travail analytiques en aval.

10. Procédé pour obtenir une population de cellules Treg CD4⁺ destinée à être utilisée en tant que matériel de départ dans une immunothérapie cellulaire, le procédé comprenant
i) le fait de soumettre le sang périphérique d'un patient souffrant d'une maladie inflammatoire ou auto-immune à une analyse de cellules individuelles, moyennant quoi des cellules Treg CD4⁺ présentant les signatures identifiées dans le procédé défini dans l'une quelconque des revendications 1-13 sont séparées du sang.

11. Procédé selon la revendication 10, dans lequel des filtres analytiques sont appliqués pour
i) exclure les cellules qui accèdent aux ganglions lymphatiques via le VHE, et
ii) exclure les cellules qui sont des émigrants thymiques récents.

12. Procédé selon la revendication 11, dans lequel les cellules qui accèdent aux ganglions lymphatiques via le VHE sont des cellules CD62L+.

13. Procédé selon la revendication 10 ou 11, dans lequel les cellules qui sont des émigrants thymiques récents sont des cellules CCR9+CD45RA+, CCR9+CCR7+, CCD9+CD62L+, ou CCR9+CD45RO-.

14. Procédé selon les revendications 10 à 13, dans lequel les cellules devant être exclues sont des cellules CCR9+CCR7+CD62L+CD45RA+CD45RO-.

15. Procédé selon l'une quelconque des revendications 10 à 14, dans lequel les analyses comprennent des conditions pour identifier des cellules Treg CD4⁺ qui sont des cellules émigrantes et immigrantes telles que des molécules de type intégrine ou d'autres molécules d'adhésion associées à une adhésion au tissu cible A et à une transmigration dans le tissu-système vasculaire entier.
